# EUROPEAN PATENT APPLICATION

(11) **EP 4 050 008 A1**
(43) Date of publication of application: **31.08.2022**
(21) Application number: 20879564.1
(22) Date of filing: 23.10.2020
(51) Int. Cl.: C07D 403/12, C07D 405/14, C07F 9/6558, A61K 31/53, A61K 31/685, A61K 31/675, A61P 1/16, A61P 9/10, A61P 3/04, A61P 3/06, A61P 3/10, A61P 5/14, A61P 29/00, A61P 35/00, A61P 9/00, A61P 37/02, A61P 31/00, A61P 3/00, A61P 31/20, A61P 35/02

(54) **PYRIDAZINONE OR PYRIDAZINE COMPOUND AND DERIVATIVE AND PHARMACEUTICAL COMPOSITION THEREOF**

(30) Priority: 24.10.2019 CN 201911019555
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); CUI, Dawei, Shanghai 201203 (CN); PANG, Xudong, Shanghai 201203 (CN); WANG, Zhubing, Shanghai 201203 (CN)
(74) Representative: dompatent von Kreisler Selting Werner - Partnerschaft von Patent- und Rechtsanwälten mbB
(86) International application number: PCT/CN2020/123330
(87) International publication number: WO 2021/078274

(57) **Abstract**

The present invention relates to a pyridazinone or pyridazine compound and a derivative and a pharmaceutical composition thereof. Specifically, the present invention provides a pyridazinone or pyridazine compound as shown in formula (I) or (Ia), or a stereoisomer, tautomer, enantiomer, diastereoisomer, resonant body, pharmaceutically acceptable salt, hydrate, solvate, or crystal form thereof. The compound as shown in formula (I) or (Ia) has an excellent agonistic effect and pharmacodynamic property on a thyroid hormone β receptor.

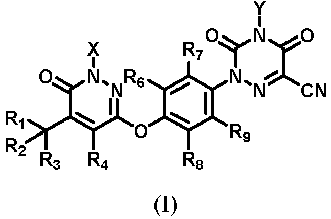

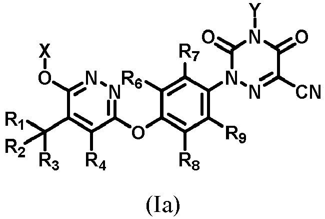

## Description

### FIELD OF THE INVENTION

The present invention relates to the field of medicine, in particular, it relates to pyridazinone or pyridazine compound and derivative and pharmaceutical composition thereof.

### BACKGROUND TECHNIQUE

Thyroid hormones are mainly used to maintain homeostasis in the human body and are therefore essential for human growth and development. Hypothyroidism can cause abnormalities in heart function, weight, metabolism, cholesterol, muscle, and behavior. Thyroid hormones can regulate body weight and cholesterol levels, but are prone to various side effects, especially on the heart and skeletal muscles. In the body, thyroid hormones achieve biological activity mainly through thyroid hormone receptors. Thyroid hormone receptors are divided into α and β subtypes, of which thyroid hormone α receptor is mainly related to heart rate control, while thyroid hormone β receptor is mainly related to lowering cholesterol and promoting metabolism.

Synthetic thyroid hormone β receptor agonist can selectively act on thyroid hormone β receptor. In preclinical animal experiments, it has been found that they can play a significant role in weight loss, lipid regulation and insulin sensitization, and at the same time, they have less side effects such as less tachycardia and skeletal muscle reduction compared with natural thyroid hormones, so they are expected to become new a generation of drugs for treating inflammatory and metabolic disease (such as non-alcoholic steatohepatitis, non-alcoholic fatty liver disease, liver fibrosis and cirrhosis, etc.). Companies such as Madrigal and Viking are currently developing specific agonists for thyroid hormone β receptors, such as MGL-3196 (Martha J. Kelly, Sherrie Pietranico-Cole, J. Douglas Larigan et al., J. Med. Chem. 2014, 57: 3912-3923) at early clinical stage. The structural formula of MGL-3196 is as follows:

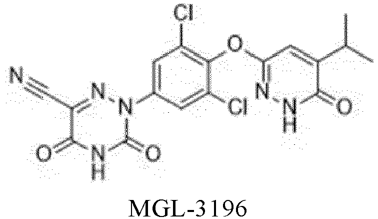

However, the existing thyroid hormone β receptor agonists also have many shortcomings, such as short action time *in vivo,* low bioavailability, and the need for multiple administrations, which limit the efficacy of thyroid hormone β receptor agonists.

Therefore, there is still a need in the art to develop thyroid hormone β receptor agonists with better pharmacodynamic properties, thereby improving the therapeutic effect on diseases.

### SUMMARY OF THE INVENTION

The object of the present invention is to provide a pyridazinone or pyridazine compound represented by formula (I) or (Ia) or a stereoisomer, tautomer, nantiomer, diastereomer, resonator, and pharmaceutically acceptable salt thereof. The compound represented by the formula (I) or (Ia) has excellent agonistic effect and pharmacodynamic properties on the thyroid hormone β receptor.

The first aspect of the present invention provides a pyridazinone or pyridazine compound represented by formula (I) or (Ia) or stereoisomer, tautomer, enantiomer, diastereomer, resonator, pharmaceutically acceptable salt, hydrate, solvate, or crystal form thereof, 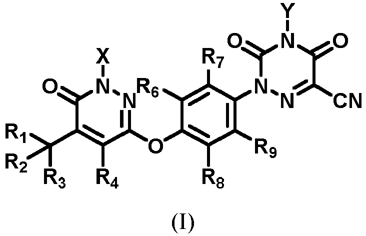 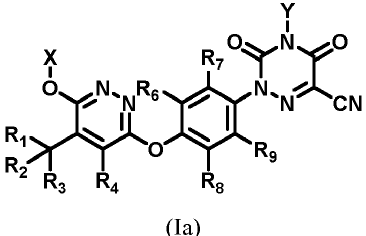 wherein,
X is selected from H or -C(R₁₇R₁₈)OZ,
Y is selected from H or -C(R₁₉R₂₀)OW, provided that both X and Y are not hydrogen at a time;
Z and W are each independently selected from -C(O)OR₅, -C(O)NHR₅, -C(O)NR₅R₁₀, -C(O)R₅, -P(=O)(X₁R₁₁)(X₂R₁₂), -P(=O)(X₁R₁₁)(X₃R₁₄R₁₅), -P(=O)(X₃R₁₄R₁₅)(X₃R₁₄R₁₅), -CH₂P(=O)(X₁R₁₁)(X₂R₁₂), -CH₂P(=O)(X₁R₁₁)(X₃R₁₄R₁₅), -CH₂P(=O)(X₃R₁₄R₁₅)(X₃R₁₄R₁₅), -P(=S)(X₁R₁₁)(X₂R₁₂), -P(=S)(X₁R₁₁)(X₃R₁₄R₁₅), -P(=S)(X₃R₁₄R₁₅)(X₃R₁₄R₁₅), -CH₂P(=S)(X₁R₁₁)(X₂R₁₂), -CH₂P(=S)(X₁R₁₁)(X₃R₁₄R₁₅), -CH₂P(=S)(X₃R₁₄R₁₅)(X₃R₁₄R₁₅), -P(=NR₁₃)(X₁R₁₁)(X₂R₁₂), -P(=NR₁₃)(X₁R₁₁)(X₃R₁₄R₁₅), -P(=NR₁₃)(X₃R₁₄R₁₅)(X₃R₁₄R₁₅), -CH₂P(=NR₁₃)(X₁R₁₁)(X₂R₁₂), -CH₂P(=NR₁₃)(X₁R₁₁)(X₃R₁₄R₁₅), -CH₂P(=NR₁₃)(X₃R₁₄R₁₅)(X₃R₁₄R₁₅), -OP(=O)(X₁R₁₁)(X₂R₁₂), -OP(=O)(X₁R₁₁)(X₃R₁₄R₁₅), -OP(=O)(X₃R₁₄R₁₅)(X₃R₁₄R₁₅), -OCH₂P(=O)(X₁R₁₁)(X₂R₁₂), -OCH₂P(=O)(X₁R₁₁)(X₃R₁₄R₁₅), -OCH₂P(=O)(X₃R₁₄R₁₅)(X₃R₁₄R₁₅), -OP(=S)(X₁R₁₁)(X₂R₁₂), -OP(=S)(X₁R₁₁)(X₃R₁₄R₁₅), -OP(=S)(X₃R₁₄R₁₅)(X₃R₁₄R₁₅), -OCH₂P(=S)(X₁R₁₁)(X₂R₁₂), -OCH₂P(=S)(X₁R₁₁)(X₃R₁₄R₁₅), -OCH₂P(=S)(X₃R₁₄R₁₅)(X₃R₁₄R₁₅), -OP(=NR₁₃)(X₁R₁₁)(X₂R₁₂), -OP(=NR₁₃)(X₁R₁₁)(X₃R₁₄R₁₅), -OP(=NR₁₃)(X₃R₁₄R₁₅)(X₃R₁₄R₁₅), -OCH₂P(=NR₁₃)(X₁R₁₁)(X₂R₁₂), -OCH₂P(=NR₁₃)(X₁Rₙ)(X₃R₁₄R₁₅), and -OCH₂P(=NR₁₃)(X₃R₁₄R₁₅)(X₃R₁₄R₁₅);
R₁, R₂ and R₃ are each independently selected from the group consisting of hydrogen, deuterium, undeuterated or one or more (preferably 1-4) deuterated or perdeuterated C1-C4 alkyl, hydroxyl, or two of R₁, R₂ and R₃ together with adjacent C form substituted or unsubstituted C3-C8 cycloalkyl; the "substituted" refers to being substituted by one or more substituents selected from the group consisting of C1-C4 alkyl, C3-C8 cycloalkyl, hydroxyl, amino, carbonyl, C2-C8 ester group, cyano, ether group, thioether group, C2-C8 amido, and sulfonamido;
R₄, R₆, R₇, R₈ and R₉ are each independently selected from the group consisting of hydrogen, deuterium, and halogen;
R₅ is substituted or unsubstituted C1-C20 alkyl, substituted or unsubstituted C3-C20 cycloalkyl, substituted or unsubstituted 4-20 membered heterocycloalkyl, substituted or unsubstituted C6-C20 aryl, substituted or unsubstituted C6-C20 aryl-C1-C8 alkyl-, substituted or unsubstituted 5-12 membered heteroaryl, substituted or unsubstituted 5-12 membered heteroaryl-C1-C8 alkyl-, or substituted or unsubstituted C1-C4 alkyl-(substituted or unsubstituted C₁-C₄ alkyl-O)ₘ-substituted or unsubstituted C1-C4 alkyl-, wherein, m is positive integer from 1 to 8; the "substituted" refers to being substituted by one or more substituents selected from the group consisting of C1-C4 alkyl, C1-C4 alkoxy, C3-C8 cycloalkyl, halogen, hydroxyl, amino, amino, C2-C8 carbonyl, C2-C8 ester group, cyano, ether group, thioether group, C2-C8 amido, and sulfonamido;
R₁₀ is selected from substituted or unsubstituted C1-C6 alkyl, substituted or unsubstituted C3-C8 cycloalkyl, or substituted or unsubstituted 4-10 membered heterocycloalkyl; the "substituted" refers to being substituted by one or more substituents selected from the group consisting of C1-C4 alkyl, C1-C4 alkoxy, C3-C8 cycloalkyl, halogen, hydroxyl, amino, amino, C2-C8 carbonyl, C2-C8 ester group, cyano, ether group, thioether group, C2-C8 amido, or sulfonamido;
X₁ and X₂ are each independently selected from the group consisting of oxygen, and sulfur;
X₃ is nitrogen;
R₁₁, R₁₂, R₁₃, R₁₄ and R₁₅ are each independently selected from the group consisting of hydrogen, substituted or unsubstituted C1-C20 alkyl, substituted or unsubstituted deuterated C1-C20 alkyl, substituted or unsubstituted C3-C10 cycloalkyl, substituted or unsubstituted C4-C10 heterocycloalkyl, substituted or unsubstituted C6-C10 aryl, substituted or unsubstituted 5-10 membered heteroaryl, or R₁₁ and R₁₂ together with adjacent X₁, X₂ and P form substituted or unsubstituted 5-7 membered heterocycloalkyl; the "substituted" refers to being substituted by one or more substituents selected from the group consisting of deuterium, C1-C20 alkyl, halogenated C1-C20 alkyl, C1-C6 alkoxy, C3-C10 cycloalkyl, 4-10 membered heterocycloalkyl, C6-C10 aryl, halogenated C6-C10 aryl, C5-C10 heteroaryl, halogen, amino, nitro, -COR₁₆, -COOR₁₆, -OCOOR₁₆, cyano, hydroxyl, amido, and sulfonamido;
R₁₆ is selected from the group consisting of hydrogen, substituted or unsubstituted C1-C18 alkyl, substituted or unsubstituted deuterated C1-C20 alkyl, substituted or unsubstituted C3-C10 cycloalkyl, substituted or unsubstituted C3-C10 cycloalkenyl, substituted or unsubstituted C6-C10 aryl, amino, substituted or unsubstituted 4-10 membered heterocycloalkyl, wherein the "substituted" refers to being substituted by one or more C6-C10 aryl;
R₁₇, R₁₈, R₁₉ and R₂₀ are each independently selected from the group consisting of hydrogen, deuterium, substituted or unsubstituted C1-C3 alkyl, provided that when X is attached to N and Y is hydrogen, both R₁₇ and R₁₈ are not hydrogen at a time.

In another preferred embodiment, the compound is the compound of formula Ib:

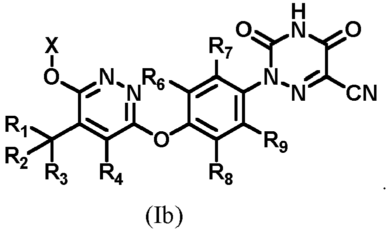

In another preferred embodiment, X is H, and Y is -C(R₁₉R₂₀)OW.

In another preferred embodiment, X is -C(R₁₇R₁₈)OZ, and Y is H or -C(R₁₉R₂₀)OW.

In another preferred embodiment, in formula (I), X is -C(R₁₇R₁₈)OZ, and Y is H.

In another preferred example, in formula (I), X is -C(R₁₇R₁₈)OZ, and Y is H.

In another preferred embodiment, in formula (Ia), X is -C(R₁₇R₁₈)OZ, and Y is H.

In another preferred embodiment, each heterocycle of the heterocycloalkyl and heteroaryl independently has 1-3 (preferably 1, 2 or 3) heteroatoms selected from N, O and S.

In another preferred embodiment, at least one of R₁, R₂, R₃, and R₄ is deuterated or deuterium.

In another preferred embodiment, at least two of R₁, R₂, R₃, and R₄ are deuterated or deuterium.

In another preferred embodiment, at least three of R₁, R₂, R₃, and R₄ are deuterated or deuterium.

In another preferred embodiment, all four of R₁, R₂, R₃, and R₄ are deuterated or deuterium.

In another preferred embodiment, all four of R₁, R₂, R₃, and R₄ are perdeuterated or deuterium.

In another preferred embodiment, at least one of R₁, R₂, R₃, and R₄ is selected from the group consisting of deuterium, one or more deuterated or perdeuterated C₁-C₄ alkyl.

In another preferred embodiment, at least two of R₁, R₂, R₃, and R₄ are selected from the group consisting of deuterium, one or more deuterated or perdeuterated C₁-C₄ alkyl.

In another preferred embodiment, at least three of R₁, R₂, R₃, and R₄ are selected from the group consisting of deuterium, one or more deuterated or perdeuterated C1-C4 alkyl.

In another preferred embodiment, R₁, R₂, R₃, and R₄ are independently selected from the group consisting of deuterium, one or more deuterated or perdeuterated C1-C4 alkyl.

In another preferred embodiment, R₁ and R₃ are independently selected from the group consisting of one or more deuterated or perdeuterated C1-C4 alkyl.

In another preferred embodiment, R₂ and R₄ are independently selected from the group consisting of hydrogen and deuterium.

In another preferred embodiment, R₁ and R₃ are CD₃.

In another preferred embodiment, R₂ is deuterium.

In another preferred embodiment, R₄ is deuterium.

In another preferred embodiment, R₇ and R₉ are deuterium.

In another preferred embodiment, at least one of R₁, R₂, and R₃ is selected from the group consisting of deuterium, one or more deuterated or perdeuterated C1-C4 alkyl.

In another preferred embodiment, at least two of R₁, R₂, and R₃ are selected from the group consisting of deuterium, one or more deuterated or perdeuterated C1-C4 alkyl.

In another preferred embodiment, R₁, R₂, and R₃ are independently selected from the group consisting of deuterium, one or more deuterated or perdeuterated C1-C4 alkyl.

In another preferred embodiment, in the compound, the deuterium isotope content of deuterium at the deuterated position is greater than the natural deuterium isotope content.

In another preferred embodiment, in the compound, the deuterium isotope content of deuterium at the deuterated position is at least 30%, preferably 50%, more preferably 75%, more preferably 95%, most preferably 99%, such as 100% greater than the natural deuterium isotope content (0.015%).

In another preferred embodiment, only one of X and Y is hydrogen.

In another preferred embodiment, X is hydrogen, and Y is not hydrogen.

In another preferred embodiment, Y is hydrogen and X is not hydrogen.

In another preferred embodiment, neither X nor Y is hydrogen.

In another preferred embodiment, R₁, R₂ and R₃ are each independently selected from the group consisting of hydrogen, deuterium, undeuterated or one or more (preferably 1-4) deuterated or perdeuterated C1-C4 alkyl.

In another preferred embodiment, R₁, R₂ and R₃ are each independently hydrogen or undeuterated C1-C4 alkyl.

In another preferred embodiment, R₁, R₂ and R₃ are each independently hydrogen or methyl. In another preferred embodiment, R₄ is hydrogen or deuterium.

In another preferred embodiment, R₅ is substituted or unsubstituted C1-C20 alkyl, substituted or unsubstituted C3-C10 cycloalkyl, substituted or unsubstituted 4-10 membered heterocycloalkyl, substituted or unsubstituted C6-C12 aryl, substituted or unsubstituted C6-C12 aryl-C1-C4 alkyl-, substituted or unsubstituted C6-C12 heteroaryl, substituted or unsubstituted C6-C12 heteroaryl-C1-C4 alkyl-, or substituted or unsubstituted C1-C4 alkyl-(substituted or unsubstituted C1-C4 alkyl-O)ₘ-substituted or unsubstituted C1-C4 alkyl-.

In another preferred embodiment, Z and W are each independently -C(O)OR₅.

In another preferred embodiment, R₅ is substituted or unsubstituted C1-C8 alkyl.

In another preferred embodiment, R₇ is hydrogen or deuterium.

In another preferred embodiment, R₉ is hydrogen or deuterium.

In another preferred embodiment, R₆ is halogen.

In another preferred embodiment, R₈ is halogen.

In another preferred embodiment, R₆, R₇, R₈ and R₉ are each independently selected from the group consisting of hydrogen, deuterium, and halogen.

In another preferred embodiment, R₁₇, R₁₈, R₁₉ and R₂₀ are each independently selected from the group consisting of hydrogen, substituted or unsubstituted C1-C3 alkyl, provided that when X is attached to N and Y is hydrogen, both R₁₇ and R₁₈ are not hydrogen at a time.

In another preferred embodiment, R₁₇, R₁₈, R₁₉ and R₂₀ are each independently selected from the group consisting of hydrogen and methyl, provided that when X is attached to N and Y is hydrogen, both R₁₇ and R₁₈ are not hydrogen at a time.

In another preferred embodiment, R₁₇ is hydrogen, and R₁₈ is hydrogen, or substituted or unsubstituted C1-C3 alkyl.

In another preferred embodiment, when X is attached to N and Y is hydrogen, both R₁₇ and R₁₈ are not hydrogen at a time.

In another preferred embodiment, R₁₉ is hydrogen, and R₂₀ is hydrogen, or substituted or unsubstituted C1-C3 alkyl.

In another preferred embodiment, R₁₇ is hydrogen, and R₁₈ is hydrogen or methyl.

In another preferred embodiment, R₁₉ is hydrogen, and R₂₀ is hydrogen or methyl.

In another preferred embodiment, the compound is the compound represented by formula II-A or formula (II-B): 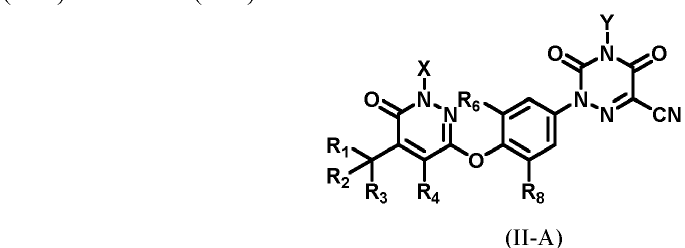 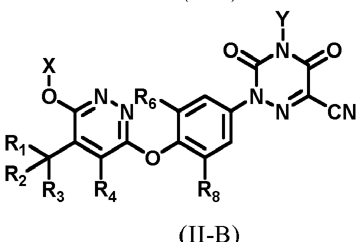 wherein, X, Y, R₁, R₂, R₃, R₄, R₆, and R₈ are defined as above.

In another preferred embodiment, the compound is the compound represented by formula (III-A) or formula (III-B): 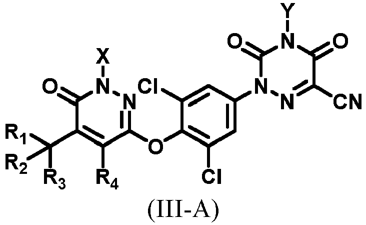 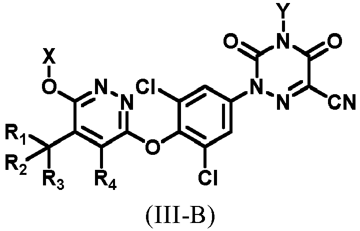 wherein, X, Y, R₁, R₂, R₃, and R₄ are defined as above.

In another preferred embodiment, the compound is a pyridazinone or pyridazine compound as shown in formula (IV-A), (IV-B), (IV-C), (IV-D) or a stereoisomer, tautomer, enantiomer, diastereomer, resonator, pharmaceutically acceptable salt, hydrate, solvate, or crystal form thereof, 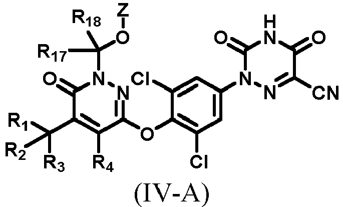 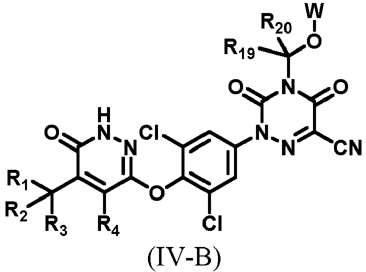 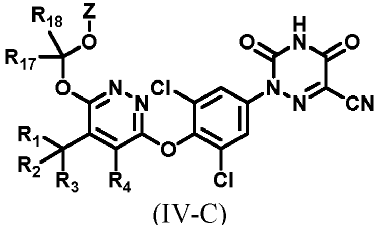 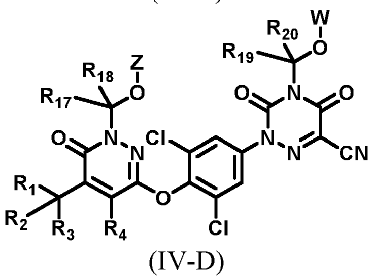 wherein, R₁, R₂, R₃, R₄, R₁₇, R₁₈, R₁₉, R₂₀, Z, and W are defined as above.

In another preferred embodiment, the compound is a pyridazinone or pyridazine compound as shown in formula (V-A), (V-B), (V-C), and (V-D), or a stereoisomer, tautomer, enantiomer, diastereomer, resonator, pharmaceutically acceptable salt, hydrate, solvate, or crystal form thereof, 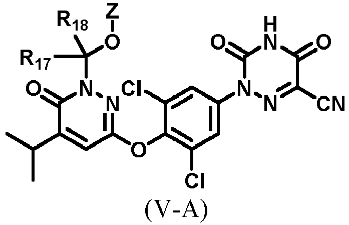 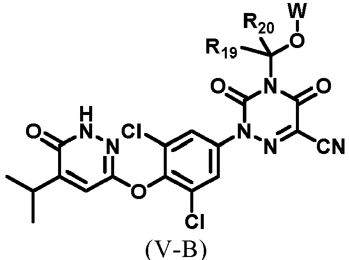 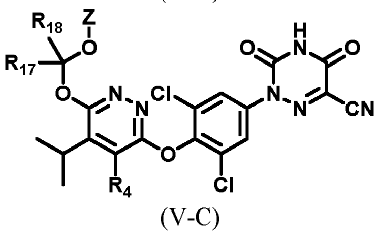 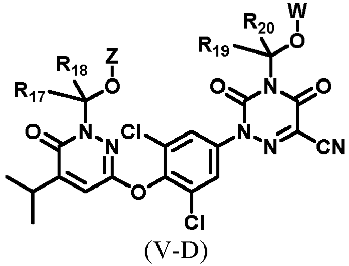 wherein, R₄, R₁₇, R₁₈, R₁₉, R₂₀, Z and W are defined as above.

In another preferred embodiment, the compound is selected from the group consisting of:

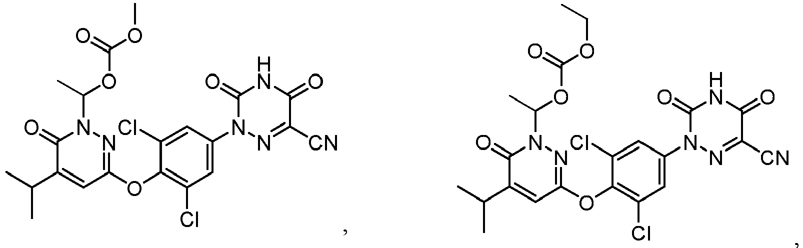

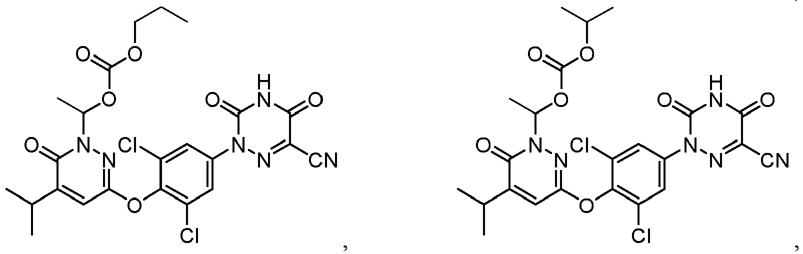

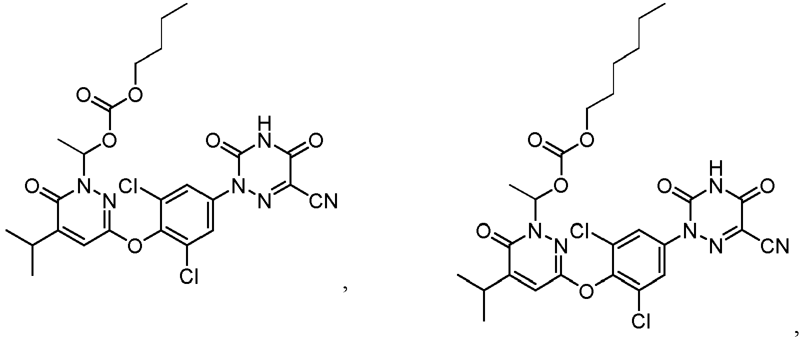

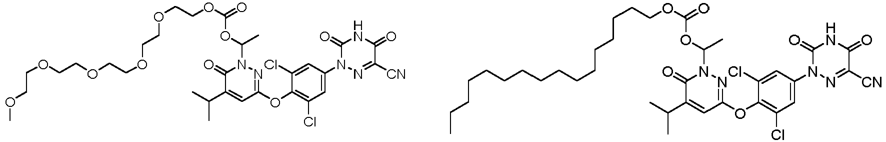

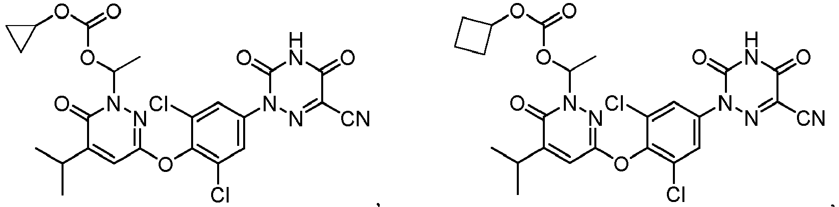

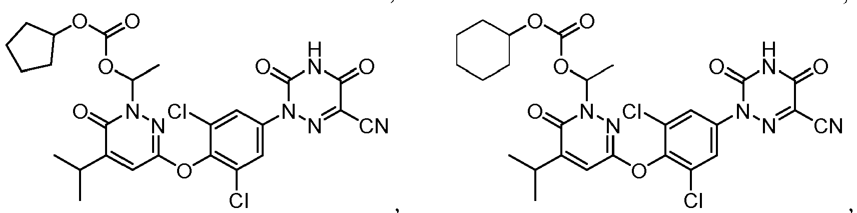

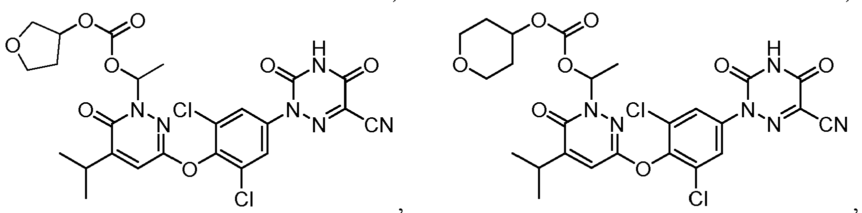

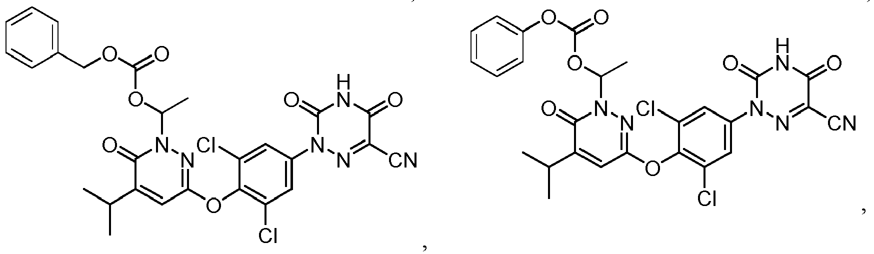

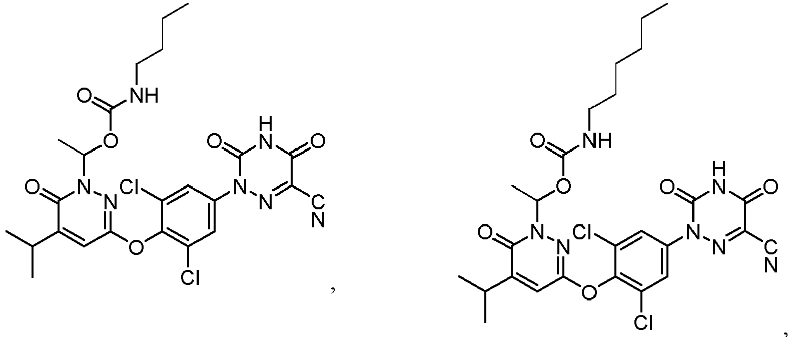

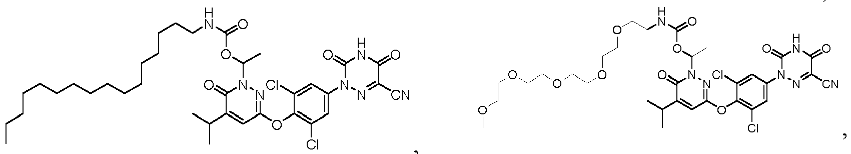

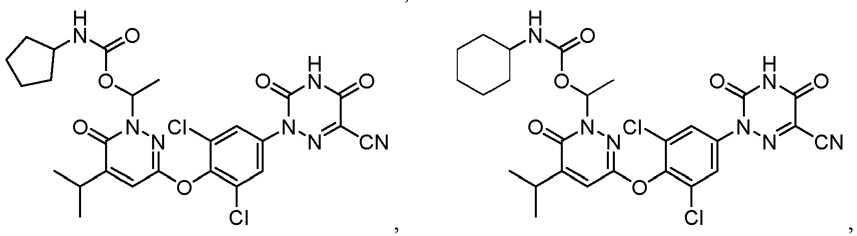

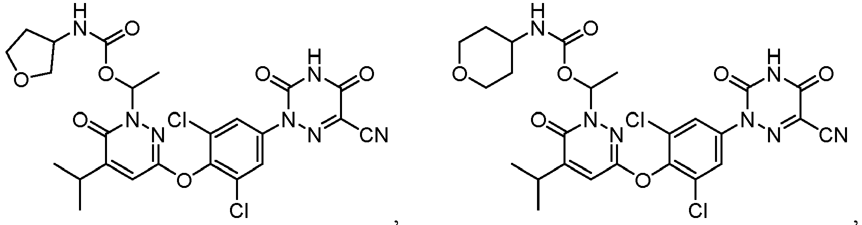

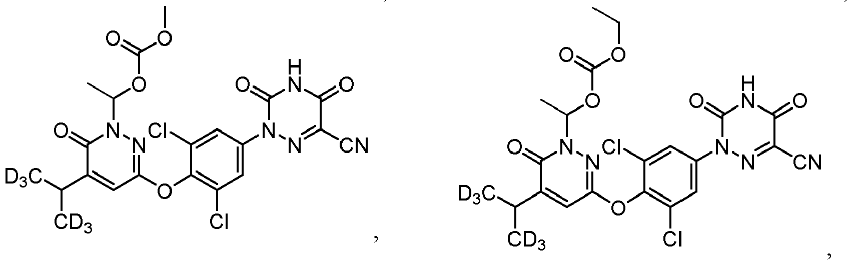

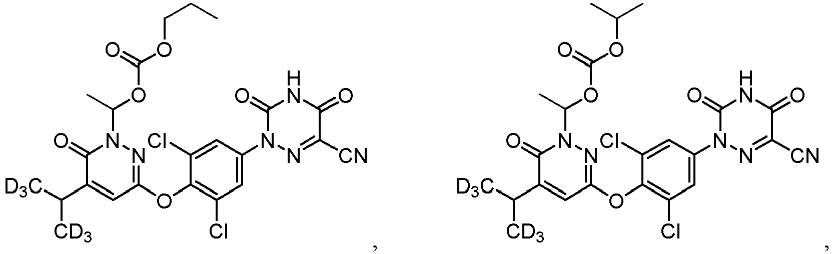

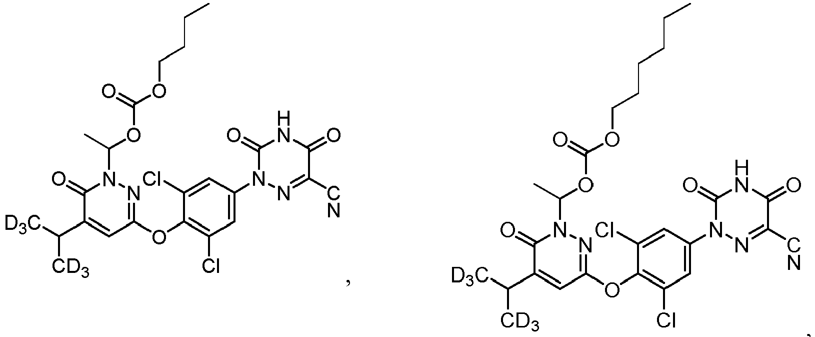

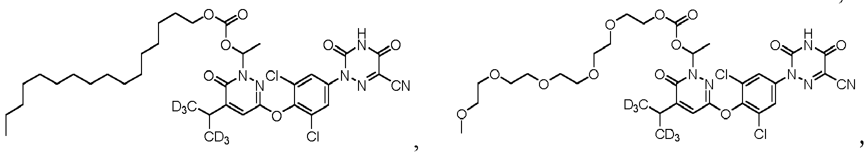

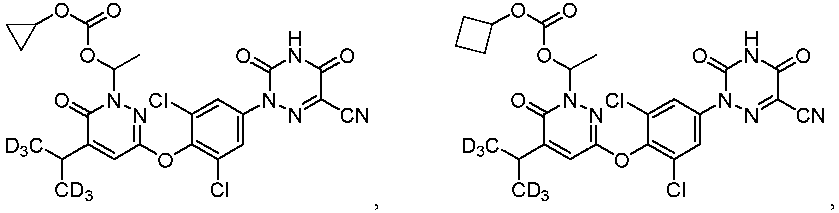

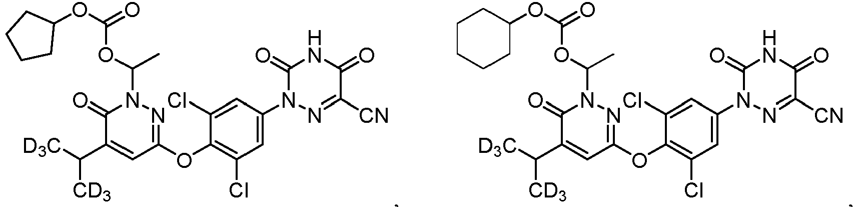

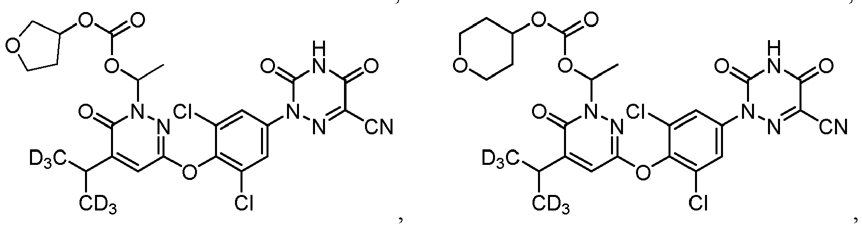

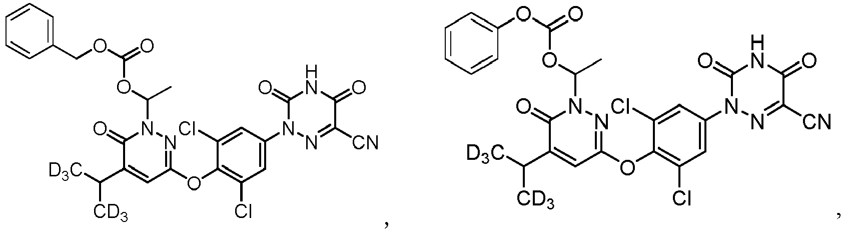

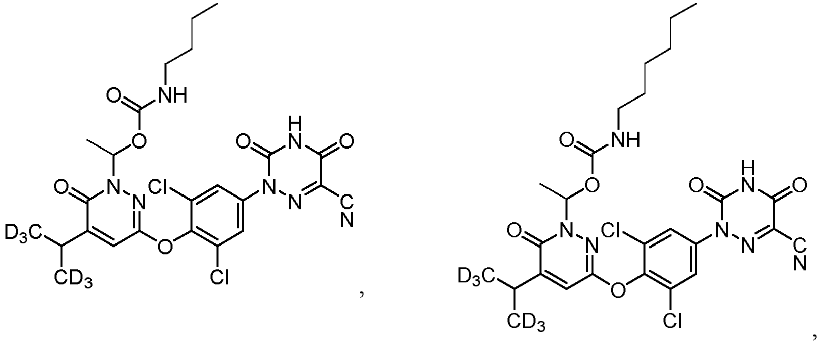

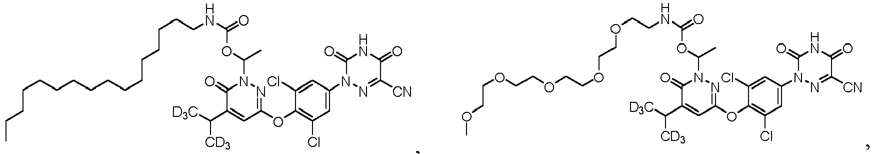

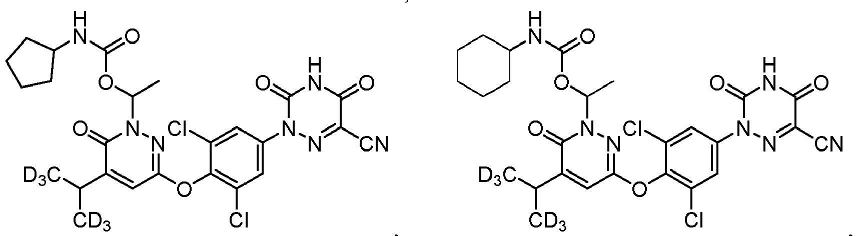

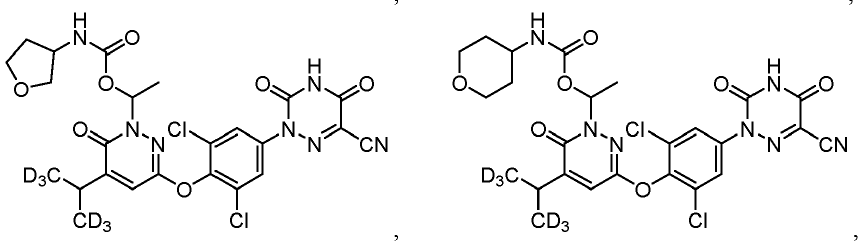

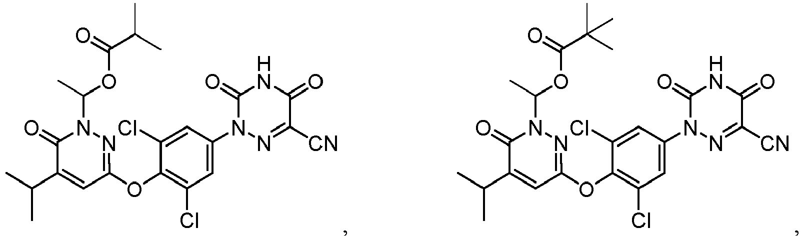

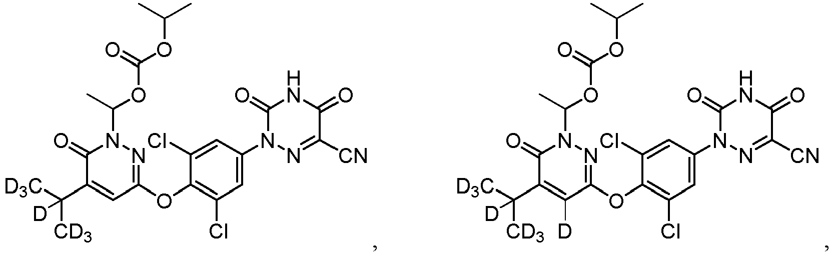

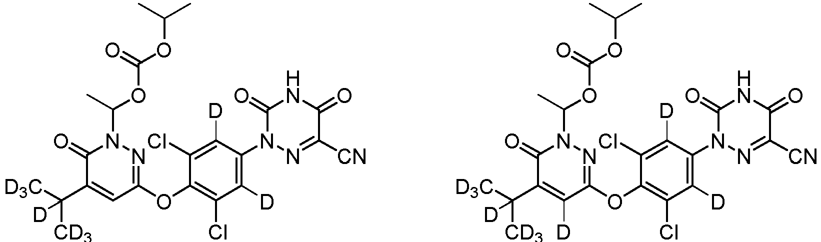

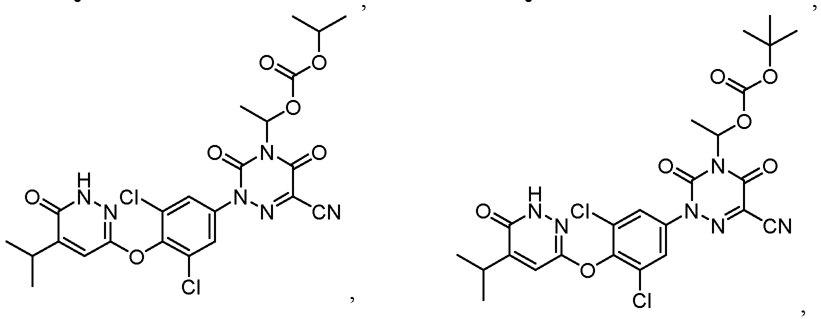

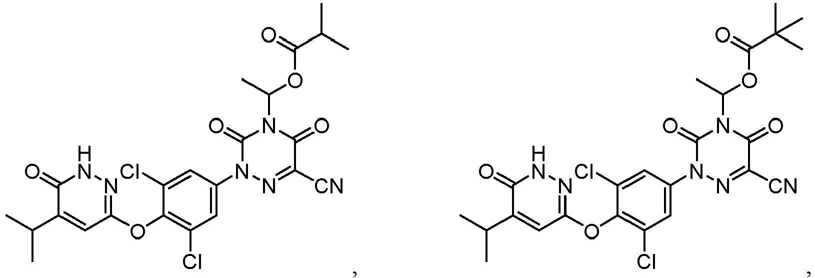

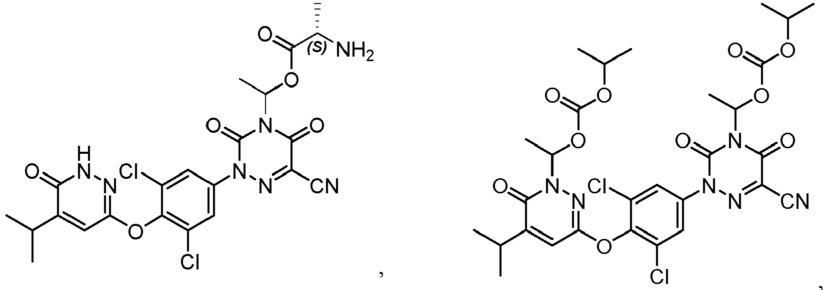

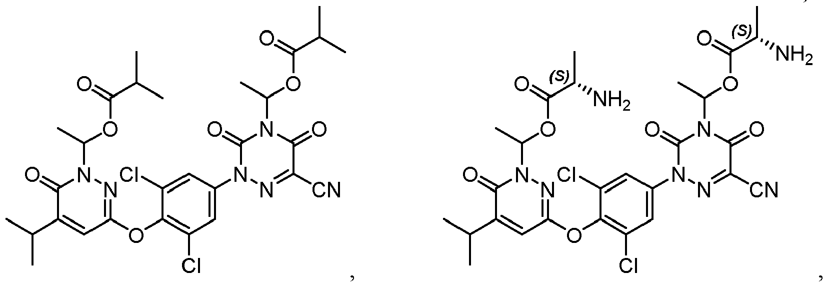

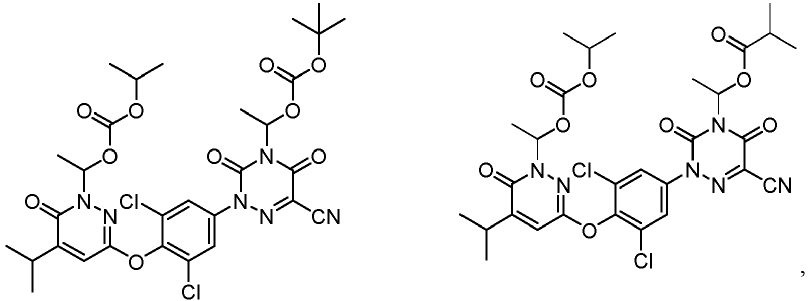

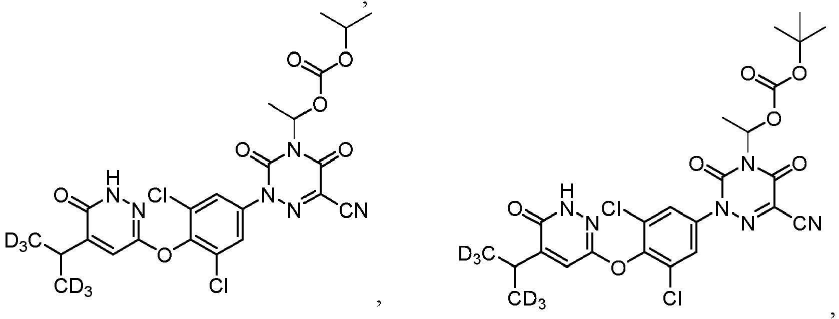

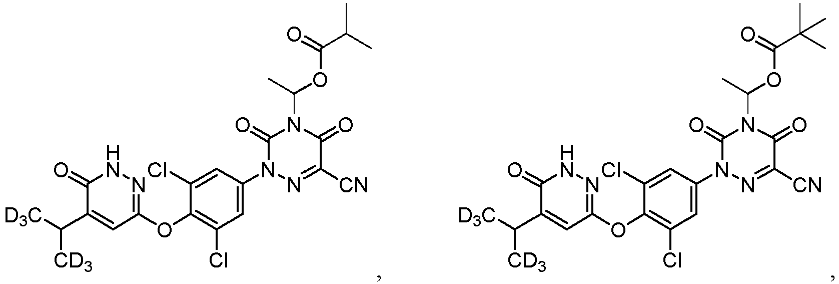

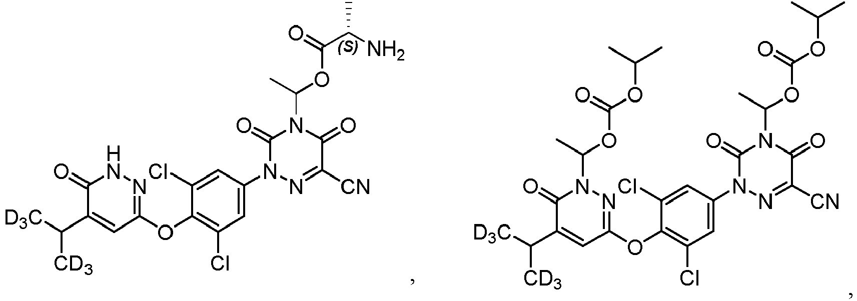

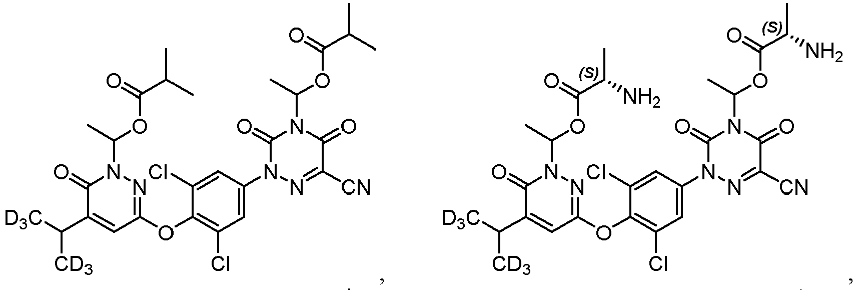

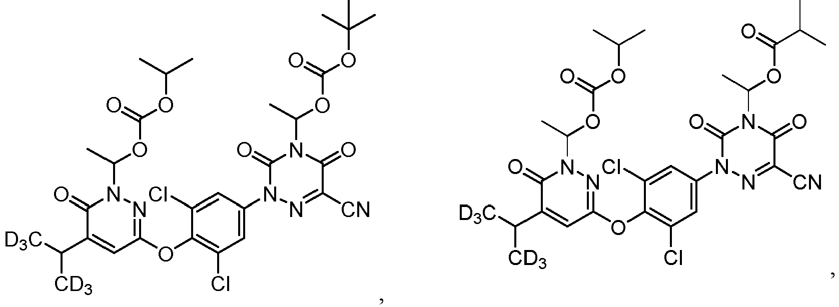

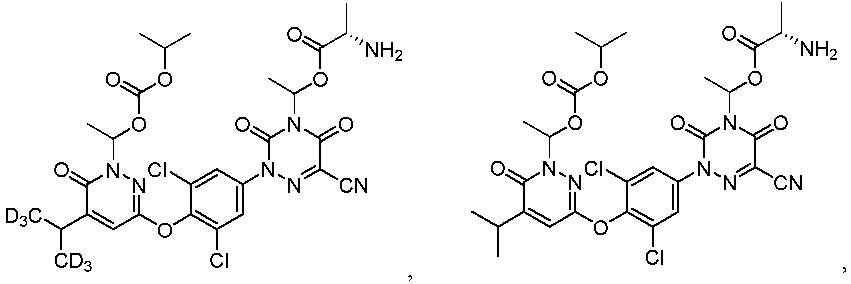

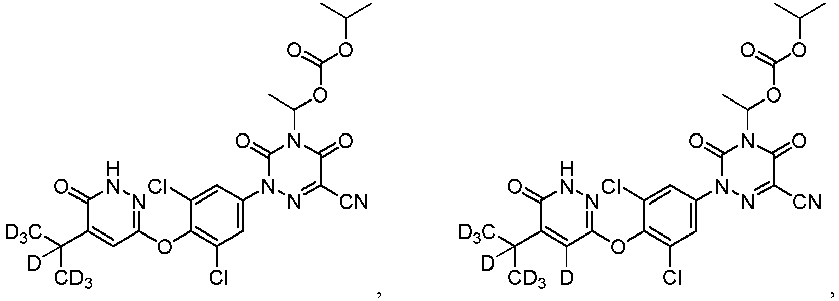

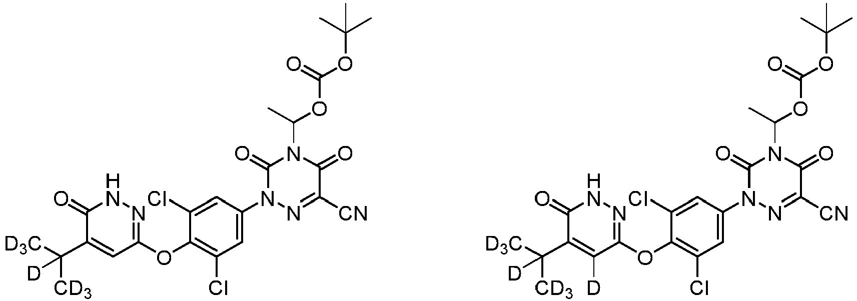

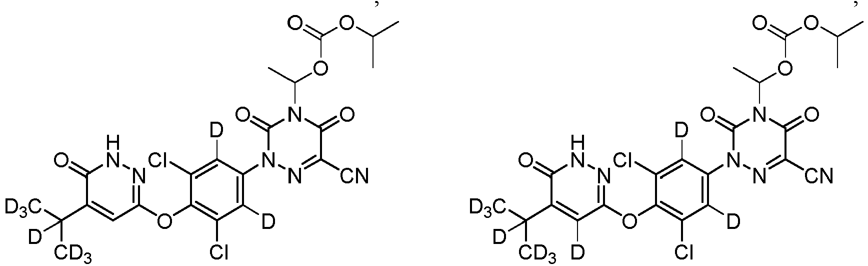

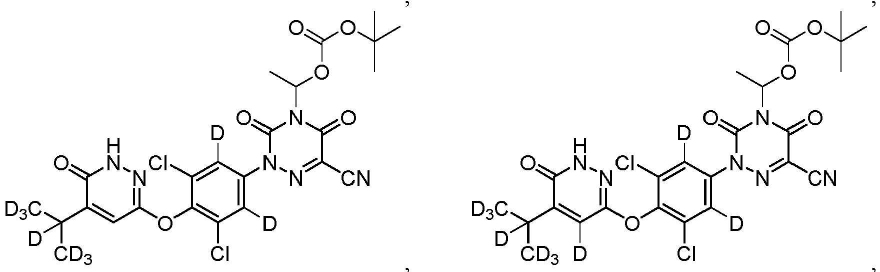

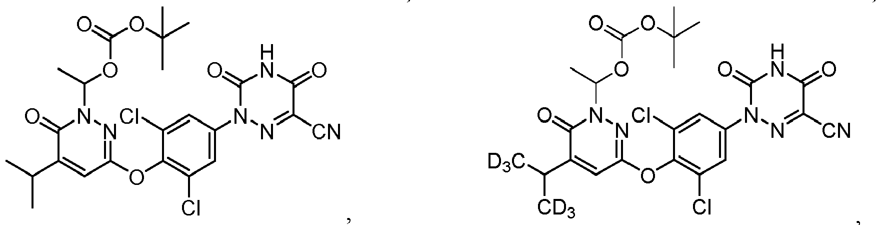

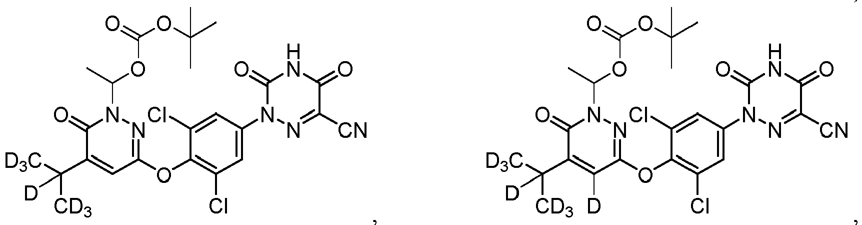

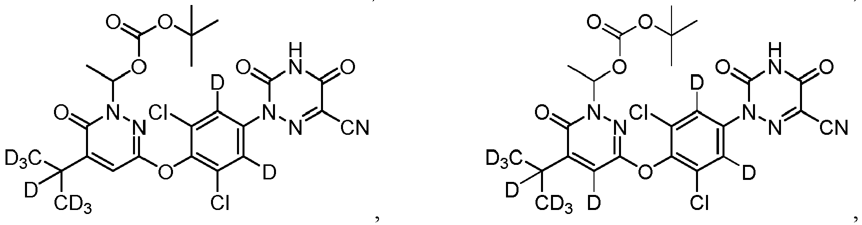

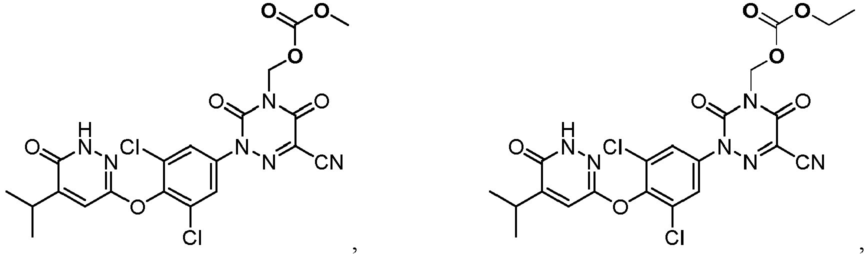

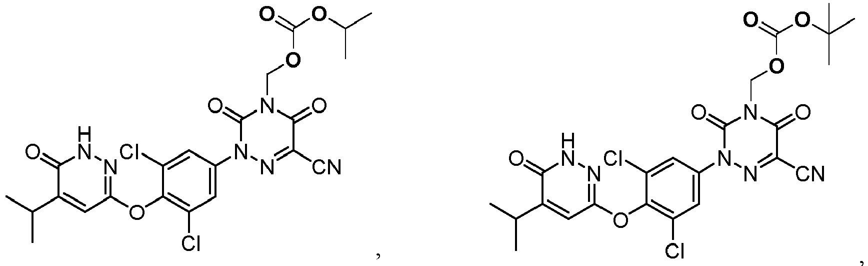

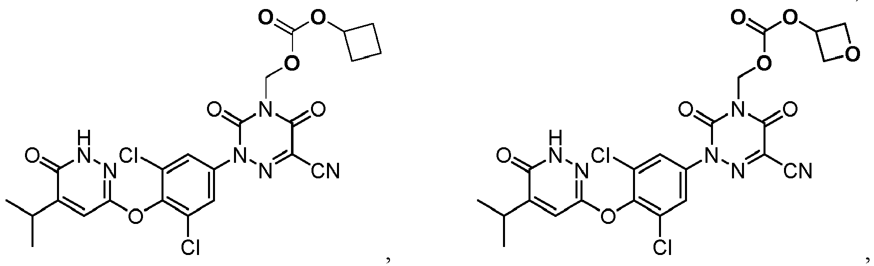

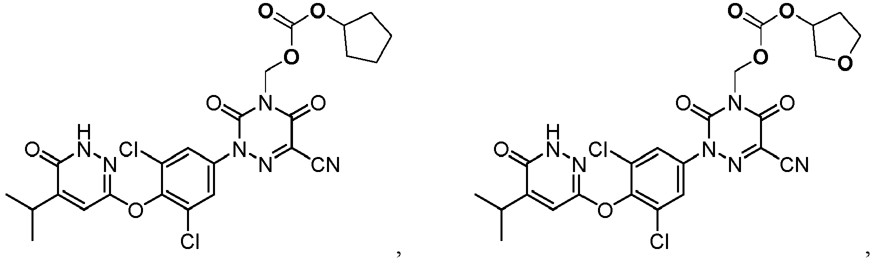

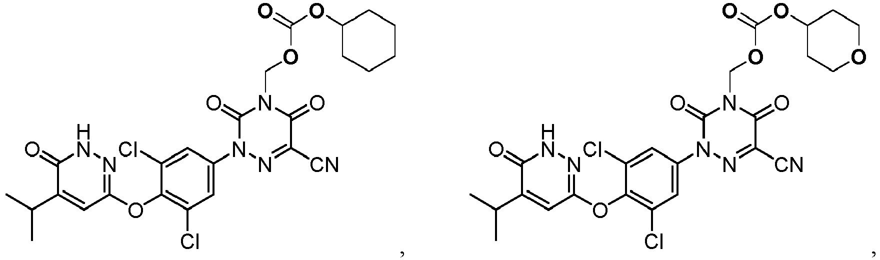

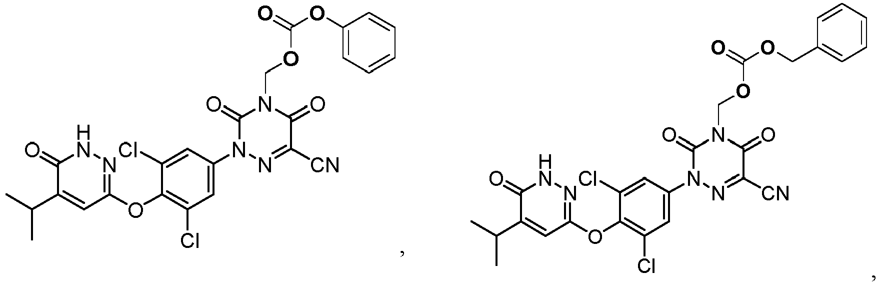

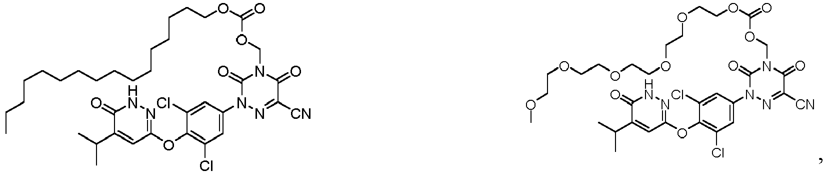

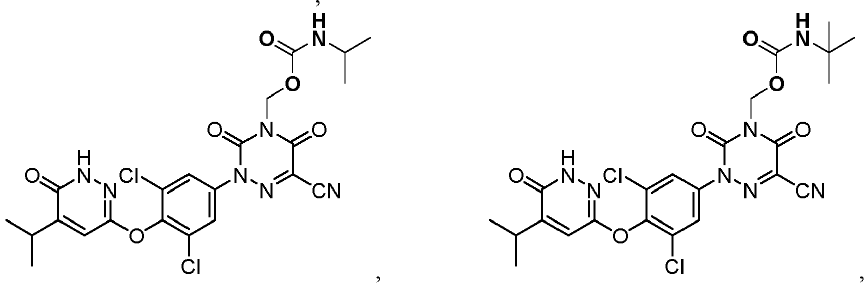

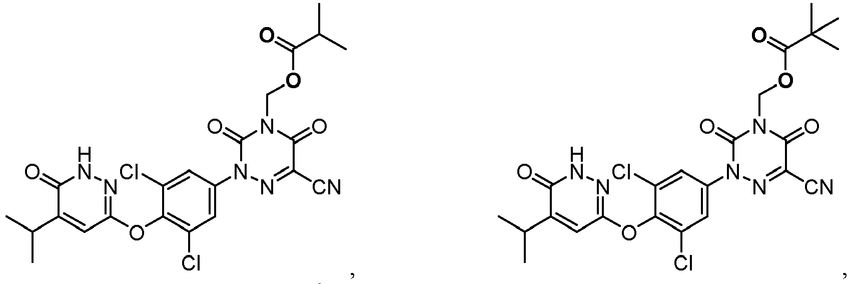

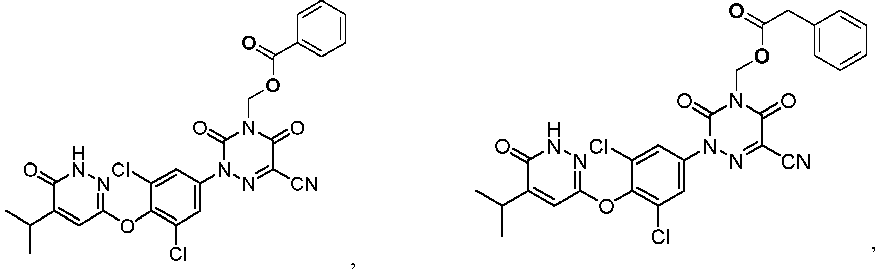

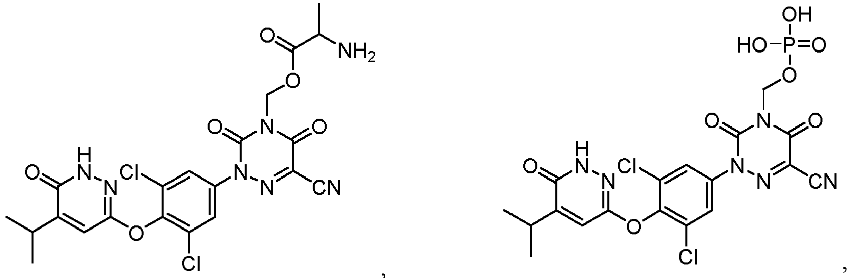

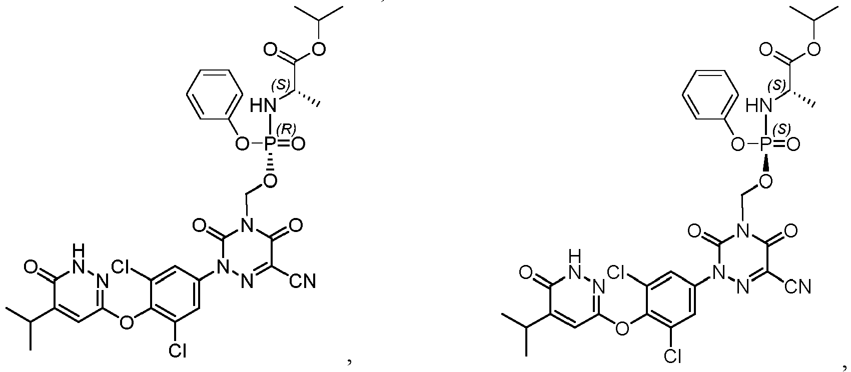

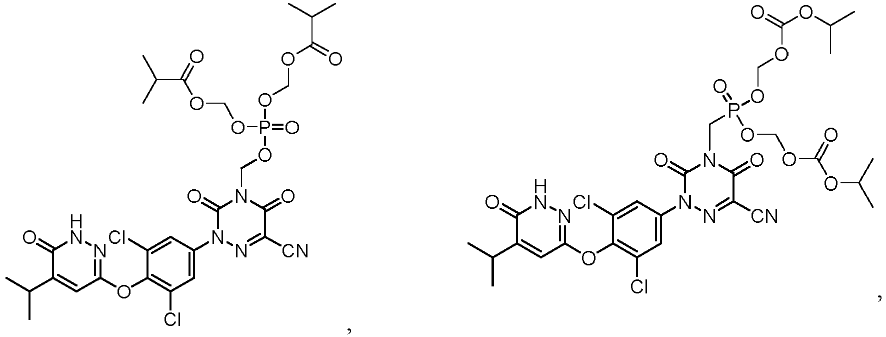

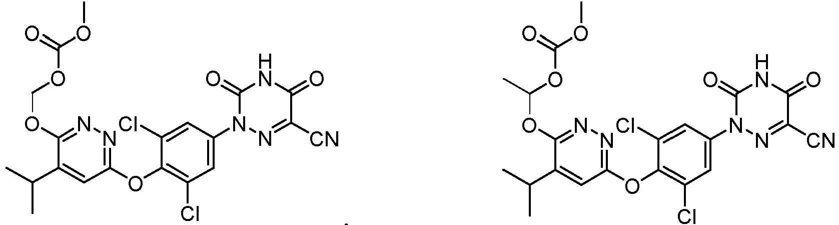

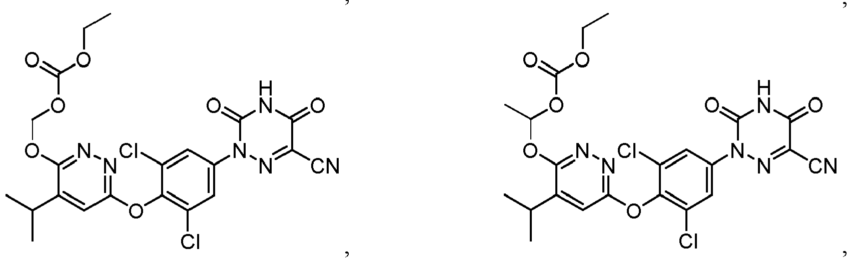

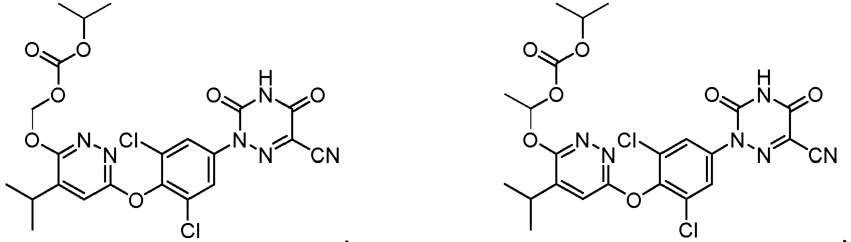

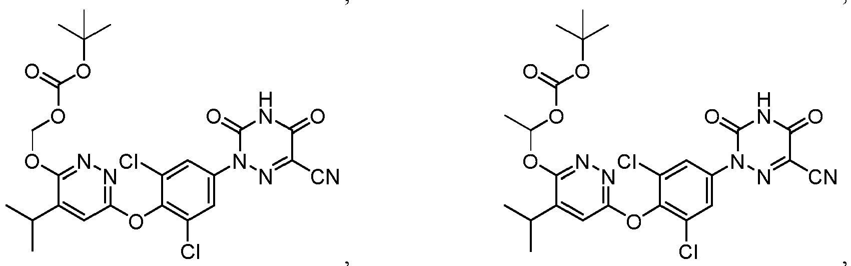

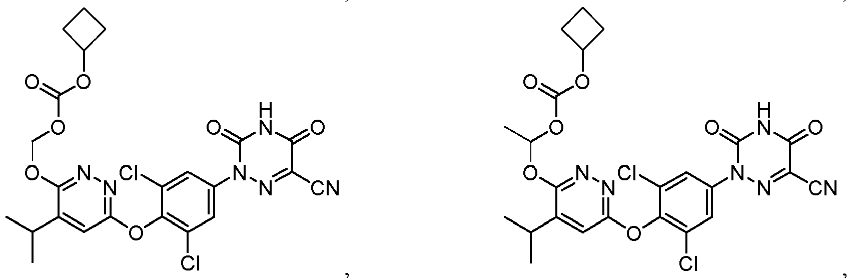

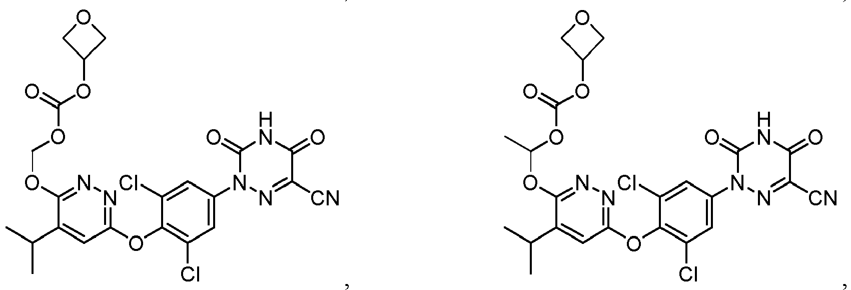

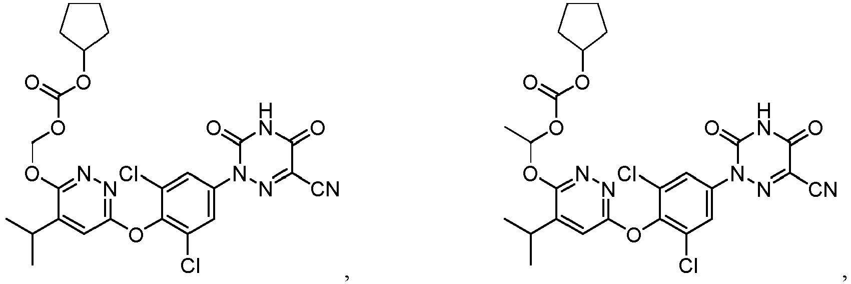

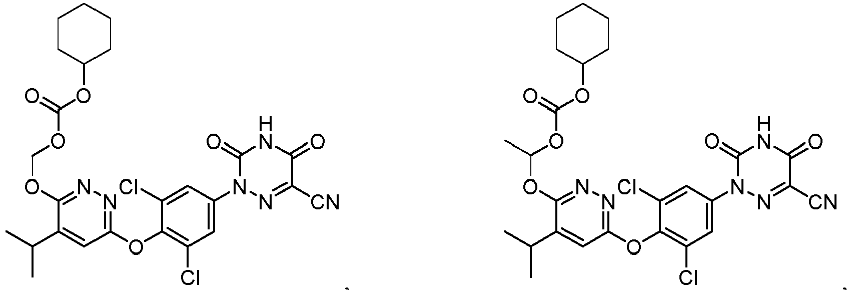

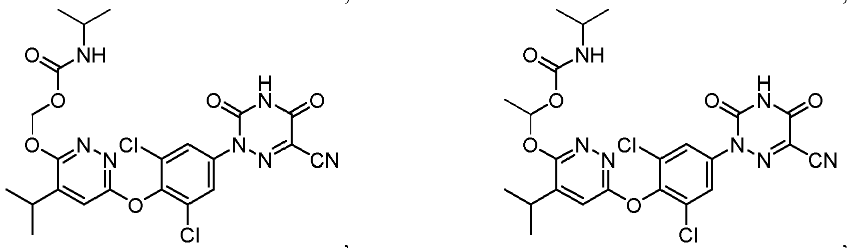

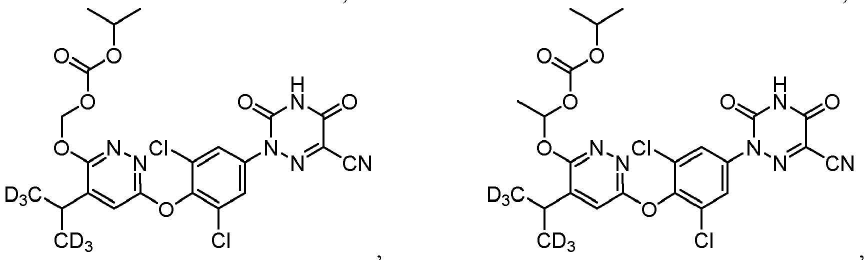

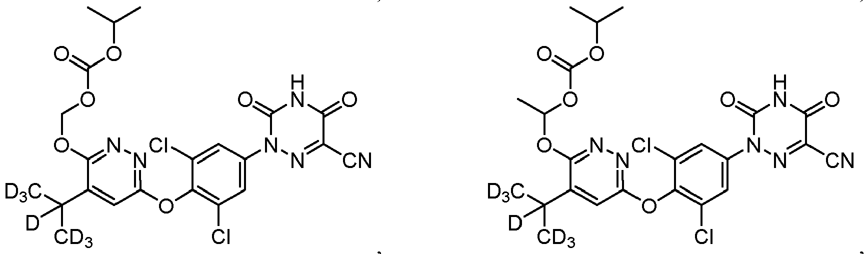

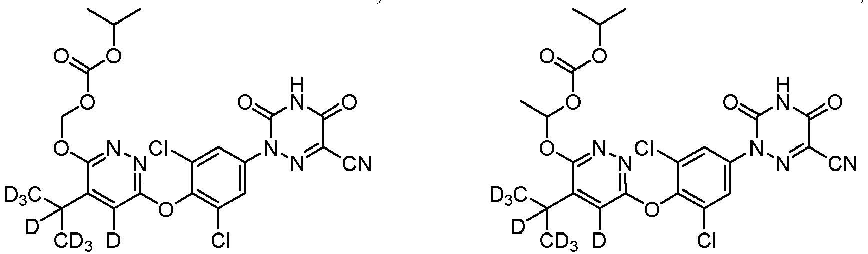

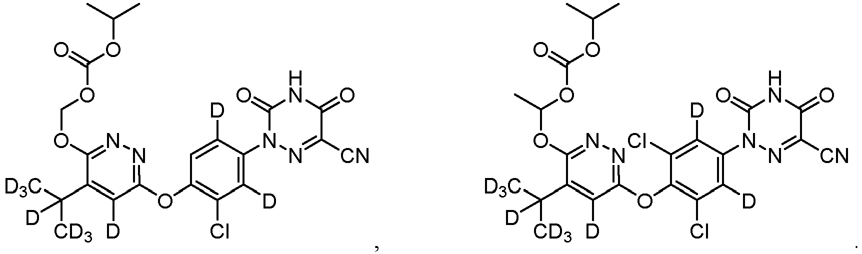

The second aspect of the present invention provides a method for preparing the pyridazinone or pyridazine compound as shown in formula (I) or (Ia) according to the first aspect of the present invention, or the stereoisomer, tautomer, enantiomer, diastereomer, resonate, pharmaceutically acceptable salt, hydrate, solvate, or crystal form thereof, and the method comprises the steps of: 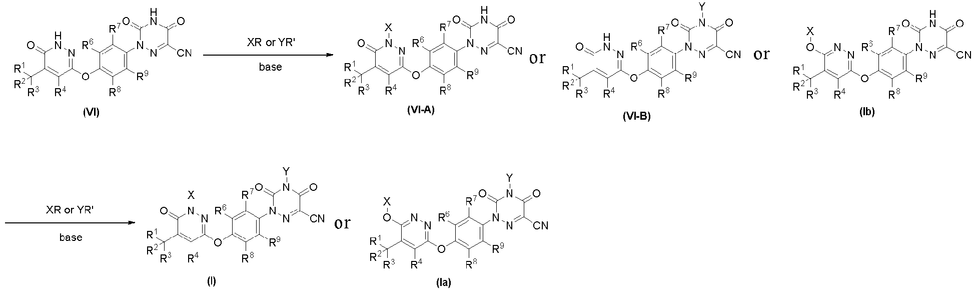
(1) reacting a compound of formula (VI) with XR or YR' to obtain an intermediate of formula (VI-A), (VI-B) or (IB) under alkaline condition
(2) reacting the intermediate of formula (VI-A), (VI-B) or (IB) with YR' or XR to obtain the compound of formula (I) or (Ia) under alkaline condition;
wherein, R, R' are leaving groups.

In another preferred embodiment, the alkaline condition is an alkaline agent selected from the group consisting of NaH, cesium carbonate, triethylamine, DIPEA, and a combination thereof.

In another preferred embodiment, the leaving group is halogen, preferably Cl, Br or I. Alternatively, the method comprises the steps of:

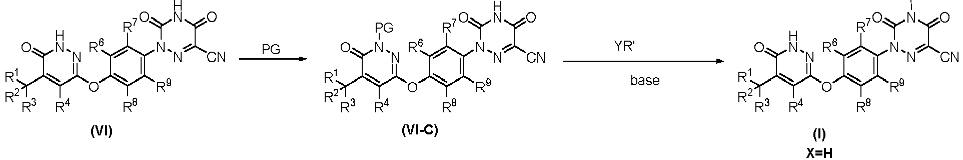

(1) protecting a compound of formula (VI) by protecting group (PG is a protecting group, such as SEM or THP) to obtain an intermediate of (VI-C), and then reacting the intermediate of (VI-C) with YR' under alkaline condition to obtain the compound of formula (I) (X is hydrogen).

The third aspect of the present invention provides a pharmaceutical composition comprising:
1) a therapeutically effective amount of the pyridazinone or pyridazine compound represented by formula (I) or (Ia) according to the first aspect of the present invention, or the stereoisomer, tautomer, enantiomer, diastereomer, resonator, pharmaceutically acceptable salt, hydrate, solvate, or crystal form thereof; and
2) a pharmaceutically acceptable carrier.

In another preferred embodiment, the content of the pyridazinone or pyridazine compound represented by formula (I) or (Ia), or the stereoisomer, tautomer, enantiomer, diastereomer, resonator, pharmaceutically acceptable salt, hydrate, solvate, or crystal form thereof is 0.01-99.99 wt.%, preferably 0.1-99.9 wt.%, more preferably 1-99wt.%, more preferably 5-95 wt.%, more preferably 10-90 wt.%, more preferably 20-80 wt.%, most preferably 30-70 wt.%, based on the weight of the composition.

In another preferred embodiment, the pharmaceutical composition further comprises other active ingredient.

In another preferred embodiment, the other active ingredient is active ingredient for preventing and/or treating a disease selected from the group consisting of inflammation, cancer, cardiovascular disease, infection, immunological disease, metabolic disease, and a combination thereof.

In another preferred embodiment, the pharmaceutical composition further comprises other drug for preventing and/or treating a disease selected from the group consisting of inflammation, cancer, cardiovascular disease, infection, immunological disease, metabolic disease, and a combination thereof.

In another preferred embodiment, the pharmaceutical composition further comprises drug selected from the group consisting of interferon α (standard INFα and polyethanolated INFα), nucleoside drugs (such as Telbivudine, Lamivudine, Clevudine, Adefovir, Tenofovir Besifovir, Tenofovir Disoproxil Fumarate (TDF), Tenofovir Alafenamide Fumarate (TAF) and HDP-PMPA (CMX157), etc.), shell protein allosteric modulators (such as BAY41-4109, RG-7907, NVR 3-778, ABI-H0731, ABI-H2158, JNJ-56136379, GLS 4JHS, etc.), cccDNA inhibitors, TLR3/7/8/9 agonists (such as RG-7854, GS9620, etc.), hepatitis B virus entry inhibitors (such as Myrcludex B, etc.), interference nucleotides (such as ARB 1467, ARB 1740, etc.), HBV surface antigen inhibitors (such as RG7834, REP2139, REP2165, etc.), CRISPER/Cas9, and a combination thereof, bile acid receptor (FXR) agonists (such as Obeticholic acid, Tropifexor, GS-9674, ZG5266), peroxisome proliferative activation receptor (PPAR) agonists (such as Elafibranor, Saroglitazar, Remogliflozin Etabonate), thyroid hormone receptor β (THPβ) agonists (such as MGL-3196), diacylglycerol-O-acyltransferase (DGAT) inhibitors (such as Pradigast, PF-06865571), acetyl-CoA carboxylase (ACC) inhibitors (such as GS-0976, PF-05221304), caspase inhibitors (such as Emricasan), smoothened receptor (SMO) inhibitors (such as Vismodegib), galectin inhibitors (such as GR-MD-02), C-C chemokine receptor 2 and 5 (such as CCR2 and CCR5) double antagonists (such as Cenicriviroc), ketohexokinase (KHK) inhibitors (PF-06835919), glucagon-like peptide-1 (GLP-1) receptor agonists (such as Liraglutide, semaglutide), lysyl oxidase like protein 2 (LOXL2) monoclonal antibody (such as simtuzumab), composite of bile acid and arachidonic acid (Aramchol), and a combination thereof.

In another preferred embodiment, the other active ingredient is active ingredient for preventing and/or treating a disease selected from the group consisting of non-alcoholic fatty hepatitis, non-alcoholic fatty liver disease, liver fibrosis, cirrhosis (eg. primary biliary cirrhosis), gallstone, atherosclerosis, obesity, diabetes, and a combination thereof.

In another preferred embodiment, the dosage form of the pharmaceutical composition is oral formulation or parenteral formulation (such as injection, infusion)

In another preferred embodiment, the dosage form of the pharmaceutical composition is selected from the group consisting of injections, blades, tablets, pills, powders, granules, aerosols, suppositories, membranes, droplets, and topical liniments.

In another preferred embodiment, the pharmaceutical composition is controlled-release formulation, sustained-release formulation or nano-formulation.

The fourth aspect of the present invention provides a use of the pyridazinone or pyridazine compound represented by formula (I) or (Ia) according to the first aspect of the present invention, or the stereoisomer, tautomer, enantiomer, diastereomer, resonate, pharmaceutically acceptable salt, hydrate, solvate, or crystal form thereof, or the pharmaceutical composition according to the third aspect of the present invention, for (i) preparing thyroid hormone receptor agonist pharmaceutical preparation or drug; (ii) preparing pharmaceutical preparation or drug for a disease related to decreased thyroid hormone receptor activity; and/or (iii) preparing pharmaceutical preparation or drug for preventing and/or treating a disease selected from the group consisting of inflammation, cancer, cardiovascular disease, infection, immunological disease, metabolic disease, and a combination thereof.

In another preferred embodiment, the disease related to decreased thyroid hormone receptor activity is selected from the group consisting of inflammation, cancer, cardiovascular disease, infection, immunological disease, metabolic disease, and a combination thereof.

In another preferred embodiment, the thyroid hormone receptor is thyroid hormone α receptor and/or thyroid hormone β receptor.

In another preferred embodiment, the thyroid hormone receptor is thyroid hormone β receptor.

In another preferred embodiment, the disease is selected from the group consisting of non-alcoholic fatty hepatitis, non-alcoholic fatty liver disease, liver fibrosis, cirrhosis (such as primary biliary cirrhosis), gallstone, atherosclerosis, obesity, hyperlipidemia, diabetes.

In another preferred embodiment, the hyperlipidemia is high triglyceride and/ high cholesterol.

In another preferred embodiment, the obesity is obesity caused by high fat diet.

In another preferred embodiment, the disease is selected from the group consisting of primary biliary cirrhosis (PBC), primary sclerosing cholangitis (PSC), cholestasis, autoimmune hepatitis, viral hepatitis (such as hepatitis B), alcoholic liver disease, non-alcoholic fatty liver disease (NAFLD), non-alcoholic steatohepatitis (NASH), liver fibrosis, arteriosclerosis, dyslipidemia, hypercholesterolemia, hypertriglyceridemia, type I diabetes, type II diabetes, obesity.

In another preferred embodiment, the cancer is selected from the group consisting of lung cancer, breast cancer, prostate cancer, esophageal cancer, colorectal cancer, blood cancer, bone cancer, kidney cancer, stomach cancer, liver cancer, bowel cancer, and a combination thereof.

The fifth aspect of the invention provides a thyroid hormone receptor agonist, the agonist comprises agonized effective amount of the pyridazinone or pyridazine compound as shown in formula (I) or (Ia) according to the first aspect of the present invention, or the stereoisomer, tautomer, enantiomer, diastereomer, resonator, pharmaceutically acceptable salt, hydrate, solvate, or crystal form thereof.

The sixth aspect of the invention provides an *in vitro* non-therapeutic and non-diagnostic method for enhancing or improving thyroid hormone receptor activity, wherein the method comprises the step of: in an *in vitro* culture system, thyroid hormone receptors or cells expressing thyroid hormone receptors are contacted with the pyridazinone or pyridazine compound represented by formula (I) or (Ia) according to the first aspect of the present invention, or the stereoisomer, tautomer, enantiomer, diastereomer, resonator, pharmaceutically acceptable salt, hydrate, solvate, or crystal form thereof, thereby enhancing or improving thyroid hormone receptor activity.

The seventh aspect of the invention provides a method of preventing and/or treating a disease selected from the group consisting of inflammation, cancer, cardiovascular disease, infection, immunological disease, metabolic disease, diabetes, comprising administering an effective amount of the pyridazinone or pyridazine compound represented by formula (I) or (Ia) according to the first aspect of the present invention, or the stereoisomer, tautomer, enantiomer, diastereomer, resonator, pharmaceutically acceptable salt, hydrate, solvate, or crystal form thereof to a patient in need thereof.

It should be understood that, within the scope of the present invention, the above technical features of the present invention and the technical features specifically described in the following descriptions (such as the examples) can be combined with each other to form a new or preferred technical solution. Due to space limitations, they will not be repeated herein.

### DESCRIPTION OF THE DRAWING

Figure 1 shows the plasma concentration-time curves of MGL-3196 (curve number 1) and the compound of Example 5 of WO2009037172A1 (curve number 2) in Test Example 1, wherein the plasma concentration is the concentration of MGL-3196.
Figure 2 shows the plasma concentration-time curves of MGL-3196 (curve number 1) and compound 6A (curve number 2) in Test Example 2, wherein the plasma concentration is the concentration of MGL-3196.
Figure 3 shows the plasma concentration-time curves of MGL-3196 (curve number 1) and compound 6B (curve number 2) in Test Example 3, wherein the plasma concentration is the concentration of MGL-3196.
Figure 4 shows the plasma concentration-time curves of MGL-3196 (curve number 1), compound 2A (curve number 3) and compound 2B (curve number 2) in Test Example 4, wherein the plasma concentration is MGL-3196 concentration.

### DETAILED DESCRIPTION OF THE INVENTION

After intensive and extensive research, the inventors unexpectedly developed a pyridazinone or pyridazine compound represented by formula (I) or (Ia) or a stereoisomer, tautomer, enantiomer, diastereomer, resonator, pharmaceutically acceptable salt, hydrate, solvate, or crystal form thereof. The pyridazinone or pyridazine compound represented by formula (I) of the present invention or its isomer formula (Ia) compound has excellent selective agonistic effect on thyroid hormone β receptor and better pharmacodynamic properties. The present invention has been completed on this basis.

### Terms

As used herein, the terms "include" "comprise" and "contain", which are used interchangeably, include not only closed definitions, but also semi-closed, and open definitions. In other words, the term includes "consist of" and "substantially consist of".

It should be understood that one of ordinary skill in the art can select the substituents and substitution patterns on the compounds of the present invention to obtain chemically stable compounds, which can be obtained by techniques known in the art and synthesis method as described below. If it is substituted by more than one substituent group, it should be understood that the multiple groups can be on the same carbon or on different carbons, as long as a stable structure is produced.

As use herein, "R1", "R₁" and "R¹" have the same meaning and can be replaced with each other, and other similar definitions have the same meaning.

In the present invention, unless otherwise indicated, the terms used have the same meaning with that of generally known to those skilled in the art. As used herein, the substitution refers to being each independently substituted by one or more (preferably 1, 2, 3 or 4) hydrogen and/or deuterium.

As used herein, the term "alkyl" refers to a straight (ie, unbranched) or branched saturated hydrocarbyl containing only carbon atoms, or a group of a combination of straight and branched chains. When the alkyl group is preceded by a carbon number limitation (e.g., C1-C20 alkyl), it means that the alkyl group has 1 to 20 carbon atoms. For example, C1-C4 alkyl refers to an alkyl group containing 1 to 4 carbon atoms, and representative examples include methyl, ethyl, propyl, isopropyl, butyl, isobutyl, sec-butyl, tert-butyl, or the like.

As used herein, the term "cycloalkyl" refers to a ring system group having a saturated or partially saturated monocyclic ring, bicyclic ring or polycyclic ring (fused, bridged or spiro ring). When a certain cycloalkyl group is preceded by a carbon number limitation (e.g., C3-C20), it means that the cycloalkyl group has 3 to 20 carbon atoms. In some preferred embodiments, the term "C3-C8 cycloalkyl" refers to a saturated or partially saturated monocyclic or bicyclic alkyl group having 3 to 8 carbon atoms, including cyclopropyl, cyclobutyl, cyclopentyl, cycloheptyl, or the like. "Spirocycloalkyl" refers to a bicyclic or polycyclic group that shares a carbon atom (called a spiro atom) between the monocyclic rings. These rings may contain one or more double bonds, but none of the rings have a fully conjugated π-electron system. "Fused cycloalkyl" refers to an all-carbon bicyclic or polycyclic group in which each ring share two neighboring carbon atoms with other ring(s), one or more rings of which may contain one or more double bonds, but none of the rings have a fully conjugated π-electron system. "Bridged cycloalkyl" refers to an all-carbon polycyclic group in which any two rings share two carbon atoms that are not directly bonded. These rings may contain one or more double bonds, but none of the rings have a fully conjugated π-electron system. Some representative examples of cycloalkyl groups are as follows, including but not limited to:

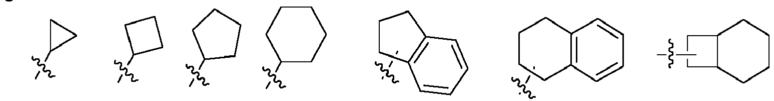

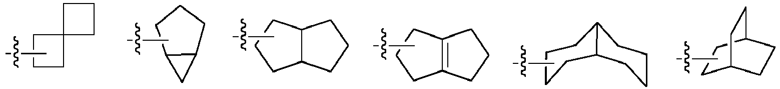

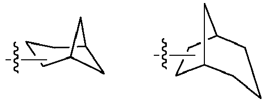

As used herein, the term "cycloalkenyl" refers to a monocyclic ring, bicyclic ring or polycyclic ring (fused, bridged or spiro ring) having at least one carbon-carbon double bond, but cannot be an aromatic structure. When a cycloalkenyl is preceded by a carbon number limitation (such as C3-C8), it means that the cycloalkenyl contains 3 to 8 ring carbon atoms.

As used herein, the term "carbonyl" is =O, and the double bond (=) is attached to carbon to form an organic functional group (C=O).

As used herein, the term "cyano" represents -CN.

The term "heterocycloalkyl" is also referred to as "heterocyclyl", which refers to a completely saturated or partially unsaturated cyclic group (including but not limited to, for example, 4-7-membered monocyclic, 6-11 membered bicyclic, or 8-16 membered tricyclic ring system) in which at least one heteroatom is present in a ring having at least one carbon atom. When a heterocycloalkyl group is preceded by a member limitation, which means the ring atom number of heterocycloalkyl group, for example, 4-20 membered heterocycloalkyl is a heterocycloalkyl group having 4 to 20 ring atoms. Each heteroatom-containing heterocyclic ring can contain one or more (such as 1, 2, 3, or 4) heteroatoms selected from the group consisting of nitrogen, oxygen, and sulfur atom, wherein the nitrogen or sulfur atom may be oxidized and the nitrogen atom may be quaternized. Heterocyclyl can be attached to the residue of any heteroatom or carbon atom of the ring or ring molecule. Typical monocyclic heterocyclic rings include, but are not limited to azetidinyl, pyrrolidyl, oxetanyl, pyrazolinyl, imidazolinyl, imidazolidinyl, oxazolidinyl, isoxazolidinyl, thiazolidinyl, isothiazolidinyl, tetrahydrofuryl, piperidyl, piperazinyl, 2-oxoppiperazinyl, 2-oxopiperidyl, 2-oxopyrrolidyl, hexahydroazepinyl, 4-piperidonyl, tetrahydropyranyl, morpholinyl, thiomorpholinyl, thiomorpholinylsulfinyl, thiomorpholinylsulfonyl, 1,3-dioxanyl and tetrahydro-1,1-dioxythienyl, etc.. A polycyclic heterocyclyl includes spiro, fused, and bridged heterocyclyls. The spiro, fused, and bridged heterocyclyls involved are optionally attached to other groups by single bond, or are further fused with other cycloalkyl, heterocyclyl, aryl and heteroaryl by any two or more atoms of the ring. The heterocyclyl may be substituted or unsubstituted. When it is substituted, the substituent is preferably one or more following groups, which are independently selected from alkyl, deuterated alkyl, haloalkyl, alkoxy, halogenated alkoxy, alkenyl, alkynyl, alkylthio, alkylamino, halogen, amino, nitro, hydroxyl, thiol, cyano, cycloalkyl, heterocyclyl, aryl, heteroaryl, cycloalkylthio, oxo, carboxy and carboxylic acid ester group.

The term "aryl" refers to an aromatic cyclic hydrocarbon group, for example, with 1, 2, 3, 4 or 5 rings, especially refers to a monocyclic and a bicyclic group, such as phenyl, biphenyl or naphthyl. Any aromatic ring having two or more aromatic rings (bicyclic, etc.), the aromatic rings of aryl may be connected by single bond (such as biphenyl) or fused (such as naphthalene, anthracene, etc.). When an aryl group is preceded by a carbon number limitation, it means the number of aryl groups, for example, a C6-C20 aryl is an aryl group having 6-20 ring carbon atoms. "Substituted aryl" refers to one or more (preferably 1-3) positions in the aryl group are substituted, and can be substituted at any position. Typically substitutions include, but are not limited to, one or more of the following groups: such as hydrogen, deuterium, halogen (e.g., monohalogen substituted or polyhalogen substituted, the latter such as trifluoromethyl or alkyl containing Cl₃), cyano, nitro, oxo (eg. =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ herein may independently represent hydrogen, deuterium, alkyl, cycloalkyl, alkenyl, cycloalkenyl, alkynyl, heterocyclic ring or aromatic ring; R_{b}, R_{c}, and R_{d} may independently represent hydrogen, deuterium, alkyl, cycloalkyl, heterocyclic ring or aromatic ring, or R_{b} and R_{c} together with N atom form a heterocyclic ring; Rₑ may independently represent hydrogen, deuterium, alkyl, cycloalkyl, alkenyl, cycloalkenyl, alkynyl, heterocyclic ring or aromatic ring. The above typical substituents may be optionally substituted. Typically substitutions also include fused ring substituent, in particular fused cycloalkyl, fused cycloalkenyl, fused heterocyclyl or fused aromatic ring group, wherein the above cycloalkyl, cycloalkenyl, heterocyclyl and heterocycloaryl may be optionally substituted.

The term "heteroaryl" refers to an aromatic heterocyclic ring system having one to more (preferably 1, 2, 3 or 4) heteroatoms, which may be monocyclic ring (monocyclic) or fused or covalent connected polycyclic ring (bicyclic, tricyclic or polycyclic). Each heteroatom-containing heterocyclic ring can contain one or more (such as 1, 2, 3, or 4) heteroatoms each independently selected from the group consisting of nitrogen, oxygen, and sulfur. When a heteroaryl group is preceded by a member limitation, it means the ring atom number of heteroaryl group, for example, 4-20-membered heteroaryl is a heteroaryl group having 4 to 20 ring atoms.

The heteroaryl is preferably 5 to 10 membered ring, more preferably 5 or 6 membered, such as pyrrol, pyrazolyl, imidazolyl, oxazolyl, isoxazolyl, thiazolyl, thiosazole, isothiazolyl, furanyl, pyridyl, pyrazinyl, pyrimidinyl, pyridazinyl, triazinyl, triazolyl, tetrazolyl, and the like. The "heteroaryl" may be substituted or unsubstituted. When it is substituted, the substituent is preferably one or more of the following groups, which are independently selected from the group consisting of alkyl, deuterated alkyl, haloalkyl, alkoxy, halogenated alkoxy, alkenyl, alkynyl, alkylthio, alkylamino, halogen, amino, nitro, hydroxyl, thiol, cyano, cycloalkyl, heterocyclyl, aryl, heteroaryl, cycloalkylthio, oxo, carboxy and carboxylic acid ester group.

In the present invention, the term "halogen" refers to F, Cl, Br or I.

In the present invention, the term "halo (halogenated)" means being substituted by halogen.

The term "amino" refers to a group with a structure -NRR', wherein R and R' may independently represent hydrogen, alkyl or substituted alkyl, cycloalkyl or substituted cycloalkyl, cycloalkenyl or substituted cycloalkenyl, aryl or substituted aryl, heterocyclyl or substituted heterocyclyl, which are defined as above. R and R' in the dialkylamine fragments may be the same or different.

The term "ether group" refers to a group with a structure -OR, wherein R may represent hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocycloalkyl or substituted heterocycloalkyl, preferably R may represent hydrogen, substituted or unsubstituted C1-C6 alkyl, substituted or unsubstituted C3-C8 cycloalkyl, substituted or unsubstituted C3-C8 cycloalkenyl, substituted or unsubstituted C6-C12 aryl, substituted or unsubstituted C5-C10 heteroaryl, substituted or unsubstituted C4-C8 heterocycloalkyl, wherein the alkyl, cycloalkyl, cycloalkenyl, aryl, heteroaryl, and heterocycloalkyl are defined as above.

The term "thioether" refers to a group with a structure -SR, wherein R may represent hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocycloalkyl or substituted heterocycloalkyl, preferably R may represent hydrogen, substituted or unsubstituted C1-C6 alkyl, substituted or unsubstituted C3-C8 cycloalkyl, substituted or unsubstituted C3-C8 cycloalkenyl, substituted or unsubstituted C6-C12 aryl, substituted or unsubstituted C5-C10 heteroaryl, substituted or unsubstituted C4-C8 heterocycloalkyl, wherein the alkyl, cycloalkyl, cycloalkenyl, aryl, heteroaryl, and heterocycloalkyl are defined as above.

The term "acyl" refers to a group with a structure -COR, wherein R may represent hydrogen, alkyl or substituted alkyl, cycloalkyl or substituted cycloalkyl, cycloalkenyl or substituted cycloalkenyl, aryl or substituted aryl, heterocyclic ring or substituted heterocyclic ring, which are defined as above.

The term "ester group" refers to a group with a structure -COOR, wherein R may represent hydrogen, alkyl or substituted alkyl, cycloalkyl or substituted cycloalkyl, cycloalkenyl or substituted cycloalkenyl, aryl or substituted aryl, heterocyclic ring or substituted heterocyclic ring, which are defined as above.

The term "amido" refers to a R-CO-NR- group or a -CO-NRR' group, wherein R is hydrogen or alkyl. When an amido group is preceded by carbon number limitation (eg, C2-C8 amido), it means that the amido contains 2-8 carbon atoms. R and R' are each independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocycloalkyl or substituted heterocycloalkyl, preferably, R may represent hydrogen, substituted or unsubstituted C1-C6 alkyl, substituted or unsubstituted C3-C8 cycloalkyl, substituted or unsubstituted C3-C8 cycloalkenyl, substituted or unsubstituted C6-C12 aryl, substituted or unsubstituted C5-C10 heteroaryl, substituted or unsubstituted C4-C8 heterocycloalkyl, wherein the alkyl, cycloalkyl, cycloalkenyl, aryl, heteroaryl and heterocycloalkyl are defined as above.

The term "sulfonamido" refers to a group with the structure -SO₂NRR', wherein R and R' are each independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocycloalkyl or substituted heterocycloalkyl, preferably R may represent hydrogen, substituted or unsubstituted C1-C6 alkyl, substituted or unsubstituted C3-C8 cycloalkyl, substituted or unsubstituted C3-C8 cycloalkenyl, substituted or unsubstituted C6-C12 aryl, substituted or unsubstituted C5-C10 heteroaryl, substituted or unsubstituted C4-C8 heterocycloalkyl, wherein the alkyl, cycloalkyl, cycloalkenyl, aryl, heteroaryl and heterocycloalkyl are defined as above.

The term "alkoxy" refers to R-O- group, wherein R is alkyl, and the alkyl is defined as above. When an alkoxy group is preceded by carbon number limitation, for example, C1-C6 alkoxy means that the alkyl group in the alkoxy group has 1-6 carbon atoms. Representative examples of alkoxy include (but are not limited to): methoxy, ethoxy, n-propoxy, isopropoxy, tert-butoxy, and the like.

In the present invention, the term "deuterated" refers to being substituted by deuterium (D).

As used herein, alone or as part of other substituents, the term "amino" means -NH₂.

As used herein, alone or as part of other substituents, the term "nitro" means -NO₂.

As used herein, alone or as part of other substituents, the term "cyano" means -CN.

In the present invention, the term "substituted" refers to one or more hydrogen atoms on a specified group being substituted with specific substituent. The specific substituents are those described correspondingly in the preceding paragraphs, or the substituents appeared in the various examples. Unless otherwise specified, a substituted group may have a substituent selected from a particular group at any substitutable position of the group, and the substituent may be the same or different at each position. It should be understood by those skilled in the art that combinations of substituents expected by the present invention are those that are stable or chemically achievable. Such substituents are for example (but not limited to): deuterium, halogen (eg, monohalogen substituent or polyhalogen substituent, the latter such as trifluoromethyl or alkyl containing Cl₃), cyano, nitro, oxo (eg=O), trifluoromethyl, trifluoromethoxy, cycloalkyl, alkenyl, cycloalkenyl, alkynyl, heterocycle, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ herein independently represent hydrogen, deuterium, alkyl, cycloalkyl, alkenyl, cycloalkenyl, alkynyl, heterocycle or aromatic ring, R_{b}, Rₑ and R_{d} may independently represent hydrogen, deuterium, alkyl, cycloalkyl, heterocycle or aromatic ring, or R_{b} and Rₑ together with the N atom form heterocycle; Rₑ may independently represent hydrogen, alkyl, cycloalkyl, alkenyl, cycloalkenyl, alkynyl, heterocycle or aromatic ring. The above-mentioned typical substituents such as alkyl, cycloalkyl, alkenyl, cycloalkenyl, alkynyl, heterocycle or aromatic ring can be optionally substituted. Typical substitutions also include spiro, bridged or fused ring substituents, especially spirocycloalkyl, spirocycloalkenyl, spiroheterocycle (excluding heteroaromatic ring), bridged cycloalkyl, bridged cycloalkenyl, bridged heterocycle (excluding heteroaromatic ring), fused cycloalkyl, fused cycloalkenyl, fused heterocyclyl or fused aryl, wherein the above cycloalkyl, cycloalkenyl, heterocyclyl and heteroaryl can be optionally substituted.

### Active ingredient

As used herein, "the compound of the present invention", "the pyridazinone or pyridazine compound of the present invention", or "the compound of formula (I) or (Ia) of the present invention" or "the compound represented by the formula (I) or (Ia) of the present invention" or "the compound of formula (I) or (Ia)" can be used interchangeably and refers to the pyridazinone or pyridazine compound represented by formula (I) or (Ia) or the stereoisomer, tautomer, enantiomer, diastereomer, resonance form, pharmaceutically acceptable salt, hydrate, solvate, or crystal form thereof, 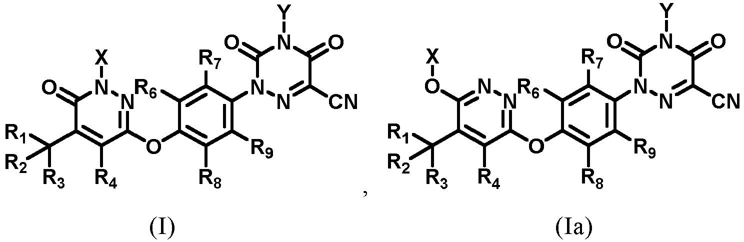 wherein, each group is defined as in the first aspect of the invention. It should be understood that the term also includes mixtures of the above components.

In the present invention, "formula I" and "formula (I)" can be used interchangeably, "formula Ia" and "formula (Ia)" can be used interchangeably, and by analogy, "formula Ib" and "formula (Ib)" can be used interchangeably.

In the present invention, the other elements (such as N, C, O, F, etc.) other than H in the compound of formula (I) or (Ia) are all or substantially (>99wt%) the most abundant naturally occurring elements such as ¹⁴N, ¹²C, ¹⁶O and ¹⁹F.

Unless otherwise stated, it is assumed that any heteroatom that is not in full valence state has enough hydrogen atoms to replenish its valence state.

In another preferred embodiment, in the compound of formula (I) or (Ia) of the present invention, any one of the groups (such as X, Y, Z, W, R₁, R₂, R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀ etc. on the compound of formula (I) or (Ia)) is the corresponding group in the specific compounds described in Table 1, respectively.

In another preferred embodiment, the compound is preferably the compound prepared in the examples.

In another preferred embodiment, the compound is selected from the compounds listed in Table 1.

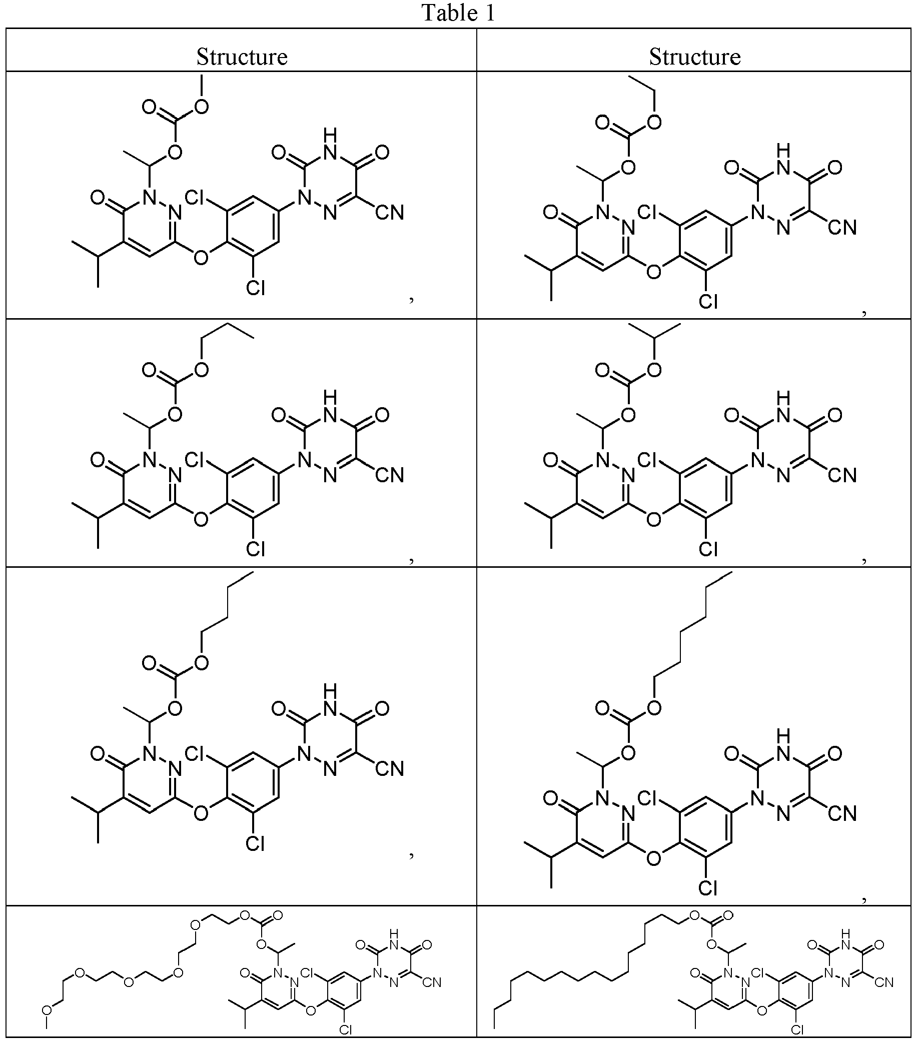

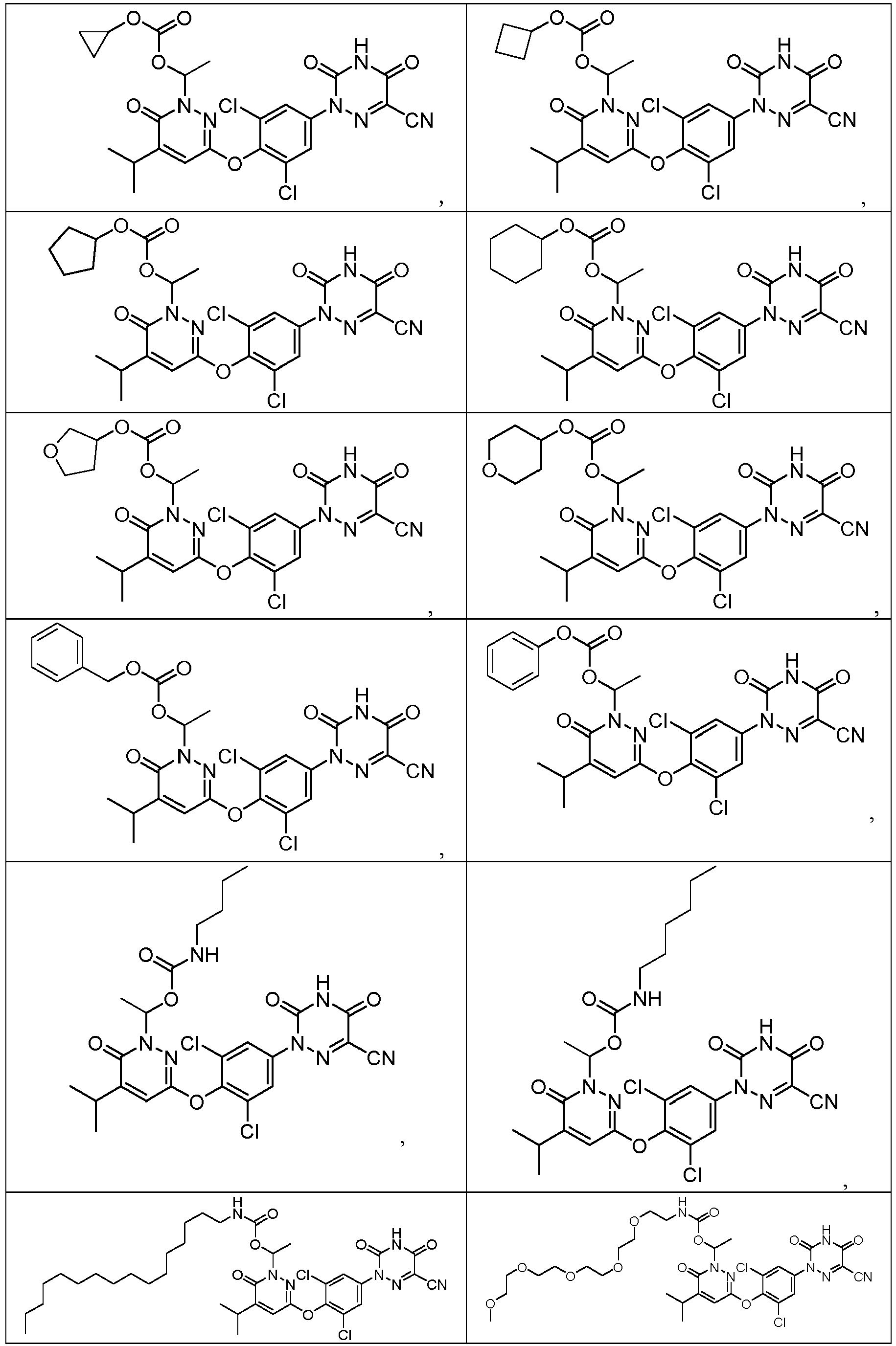

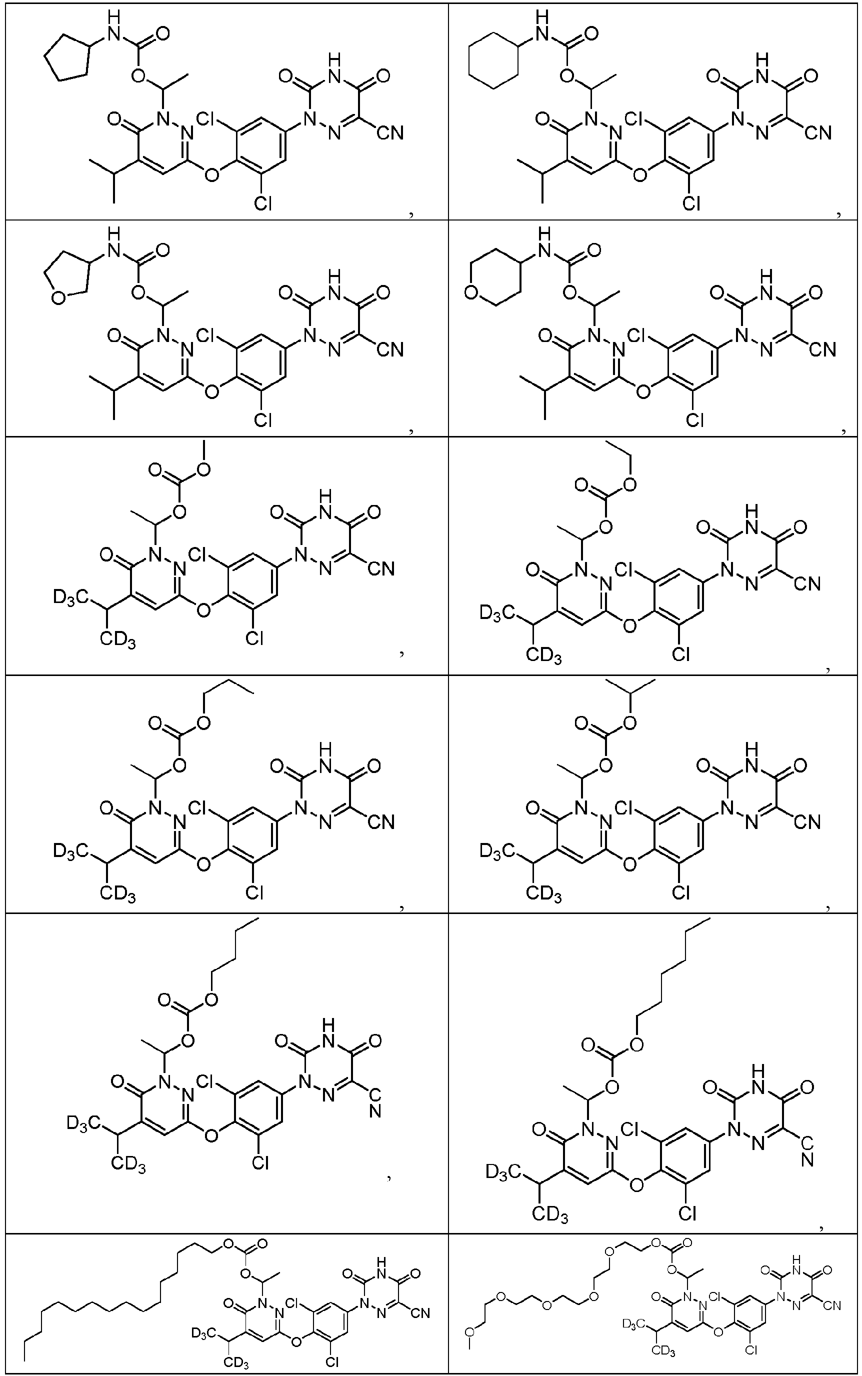

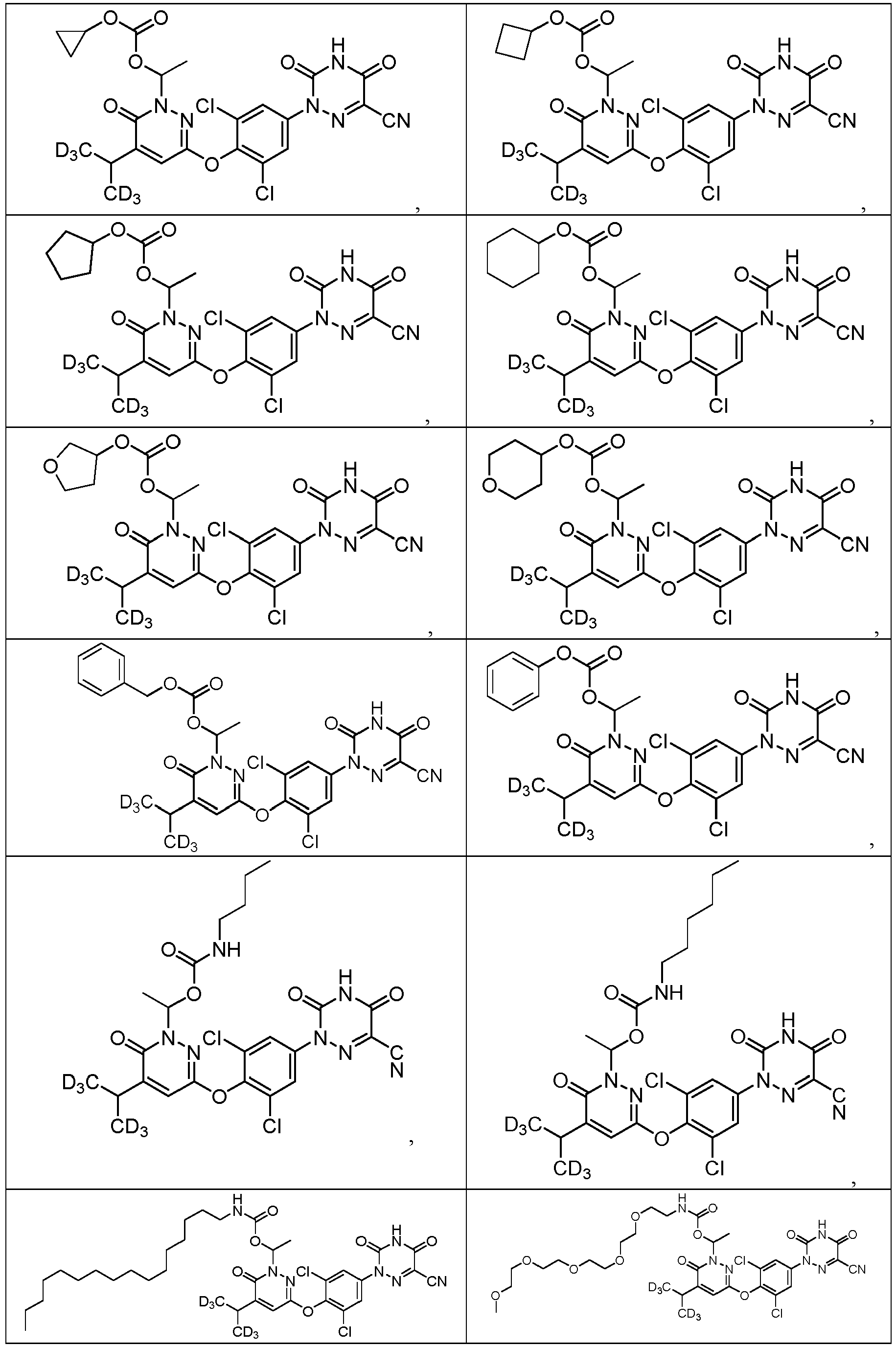

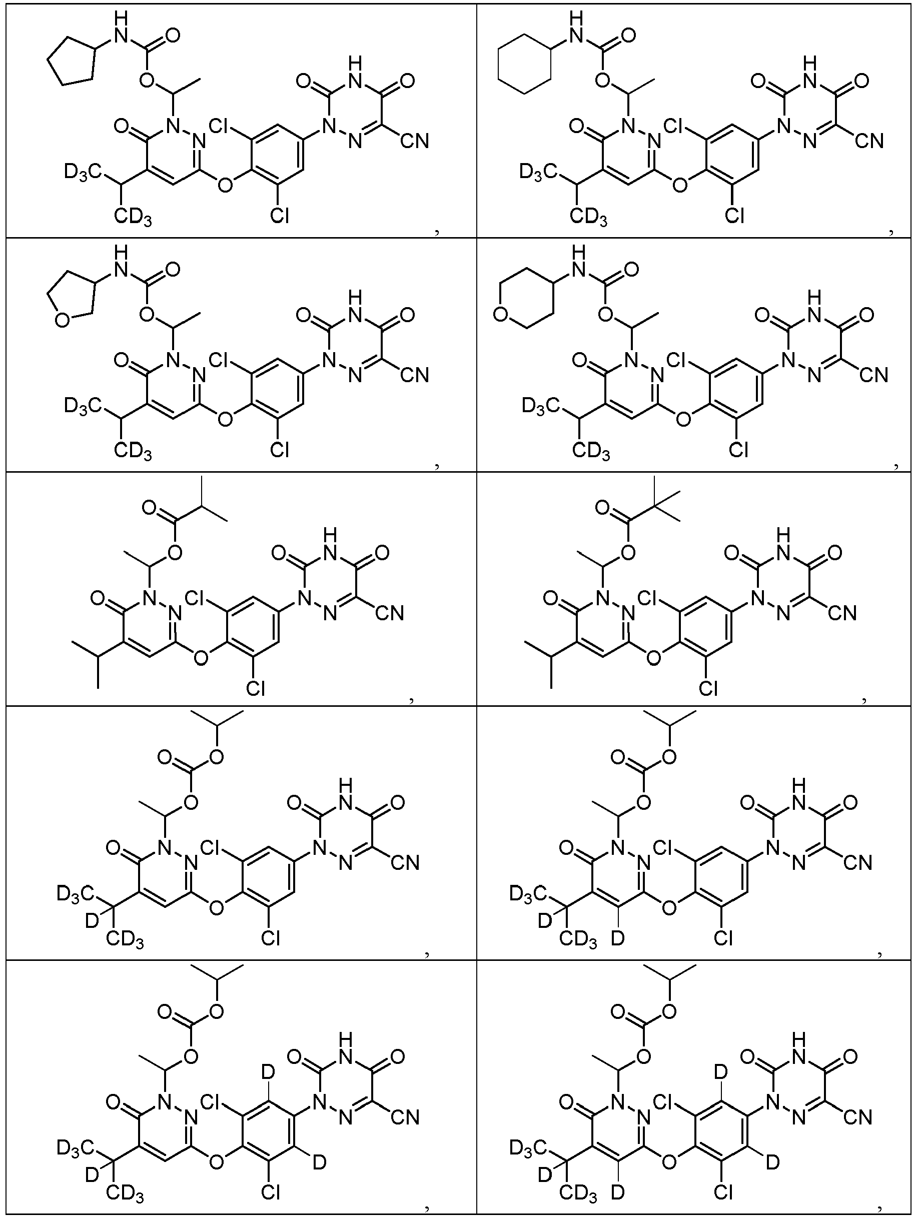

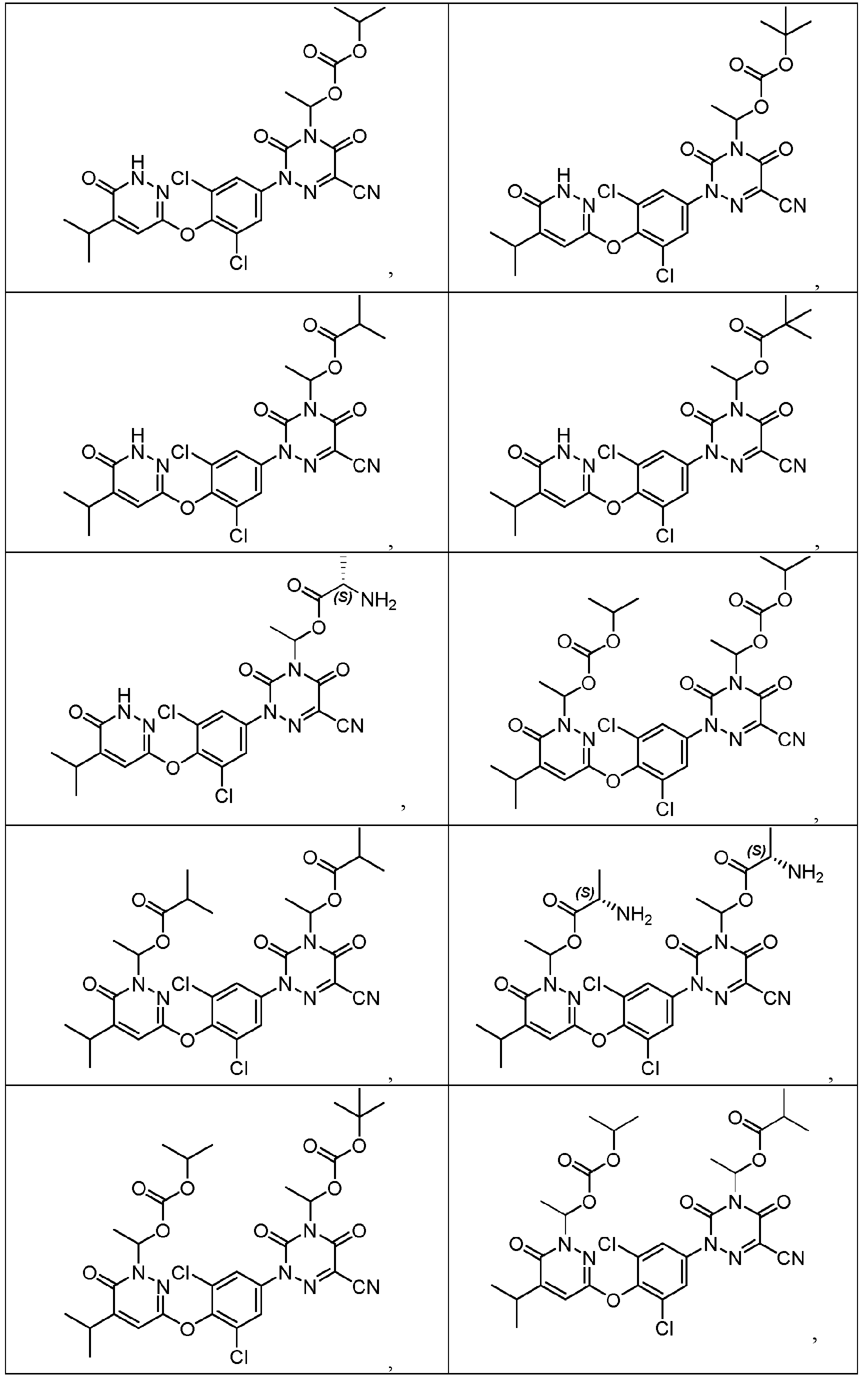

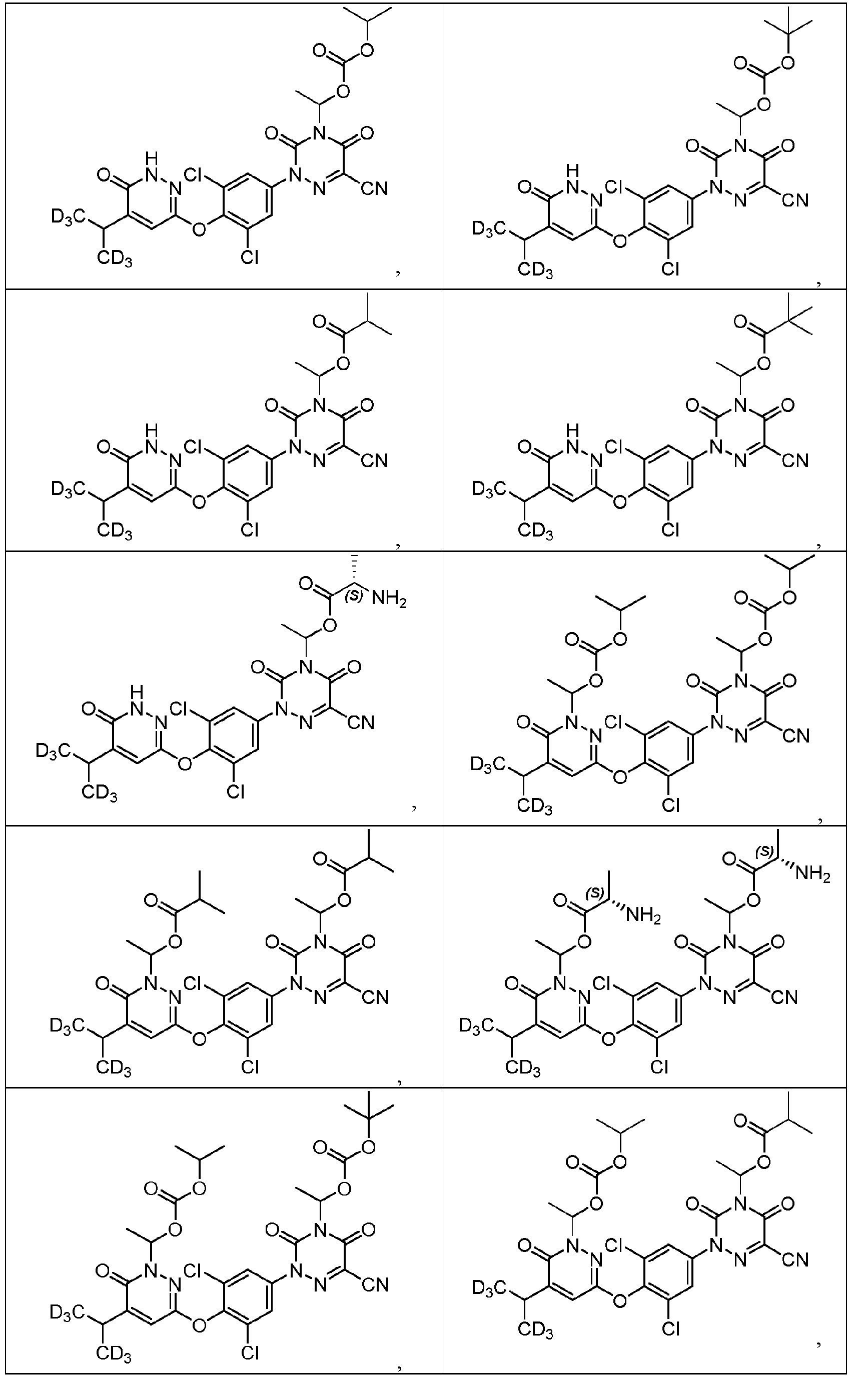

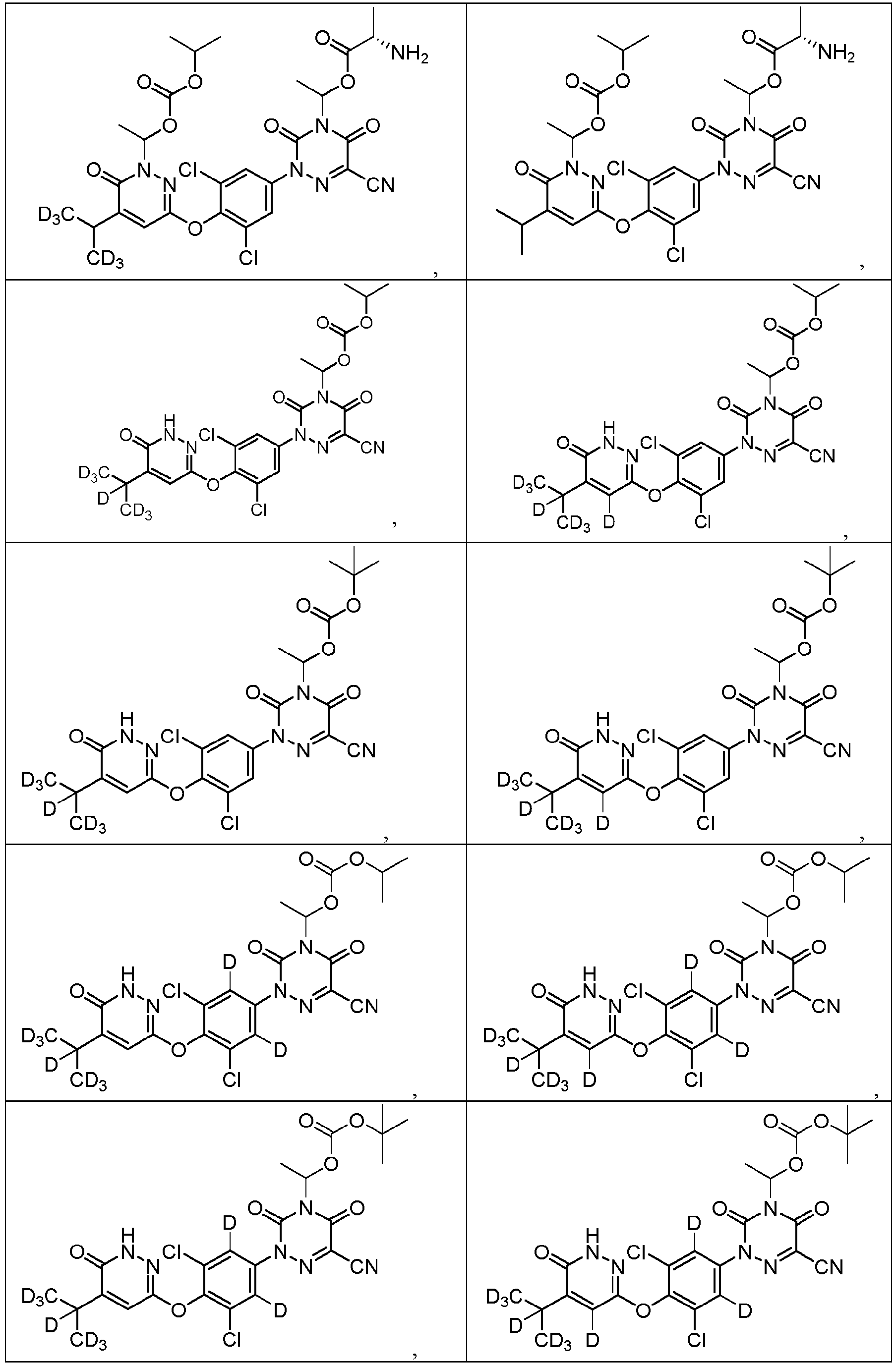

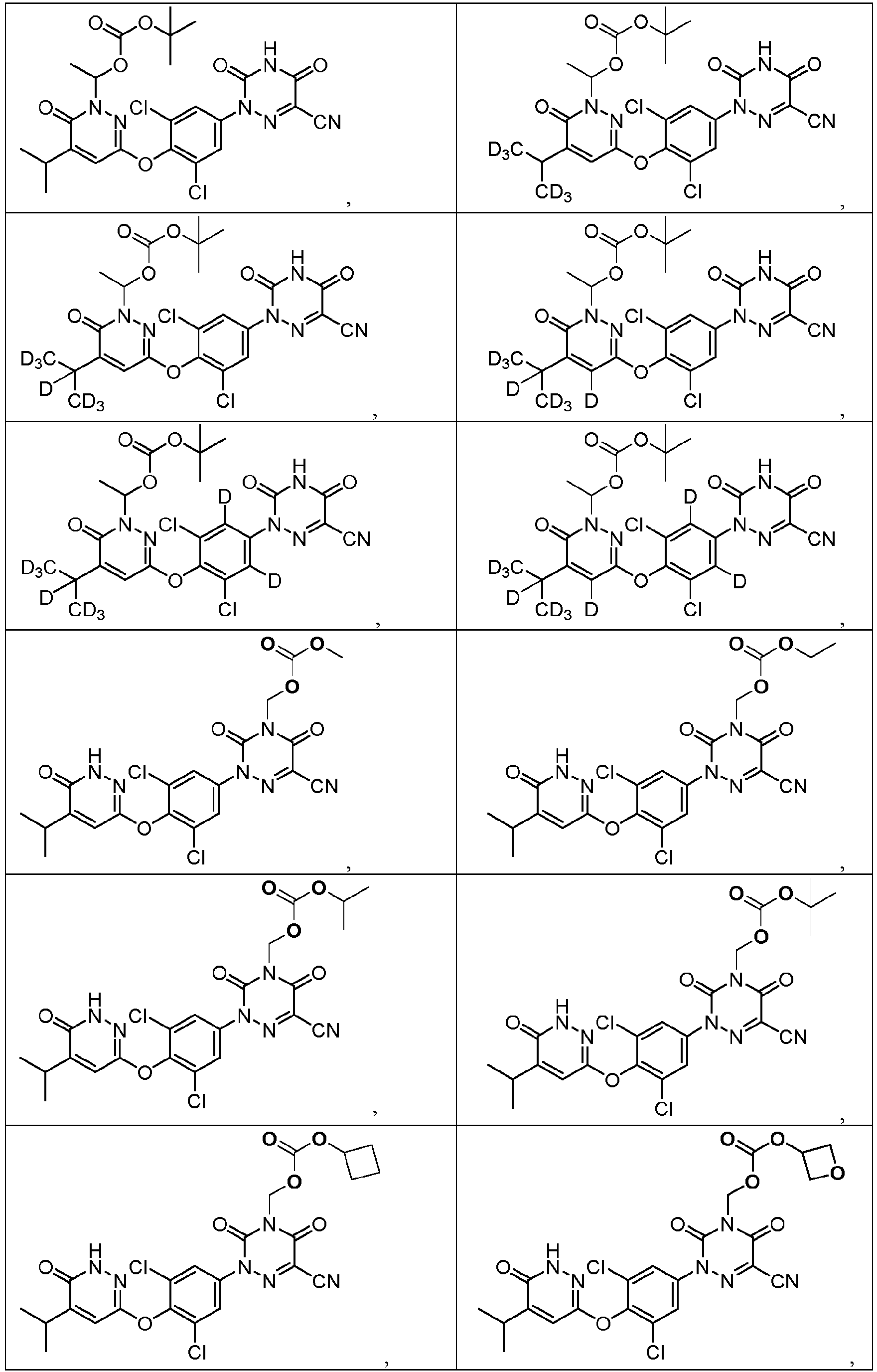

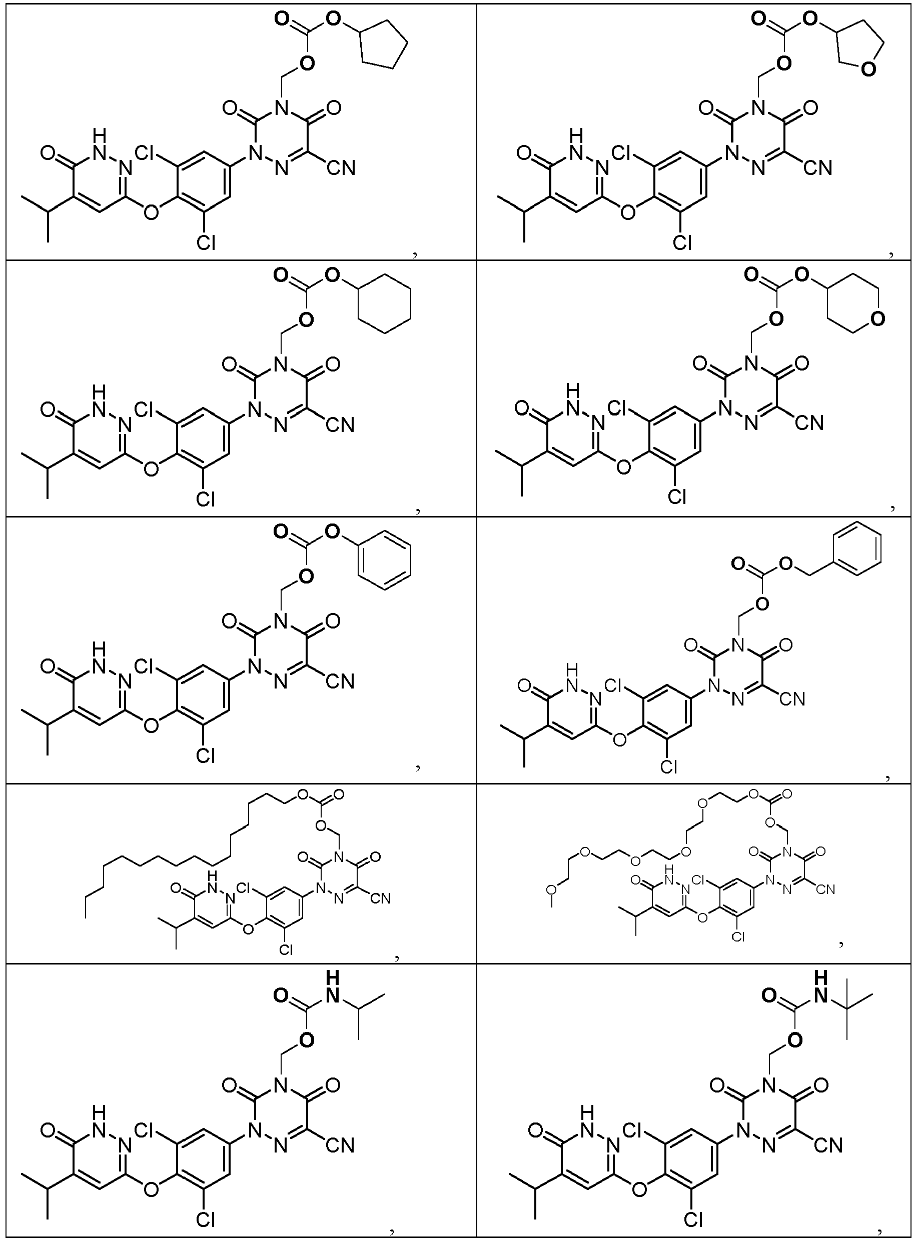

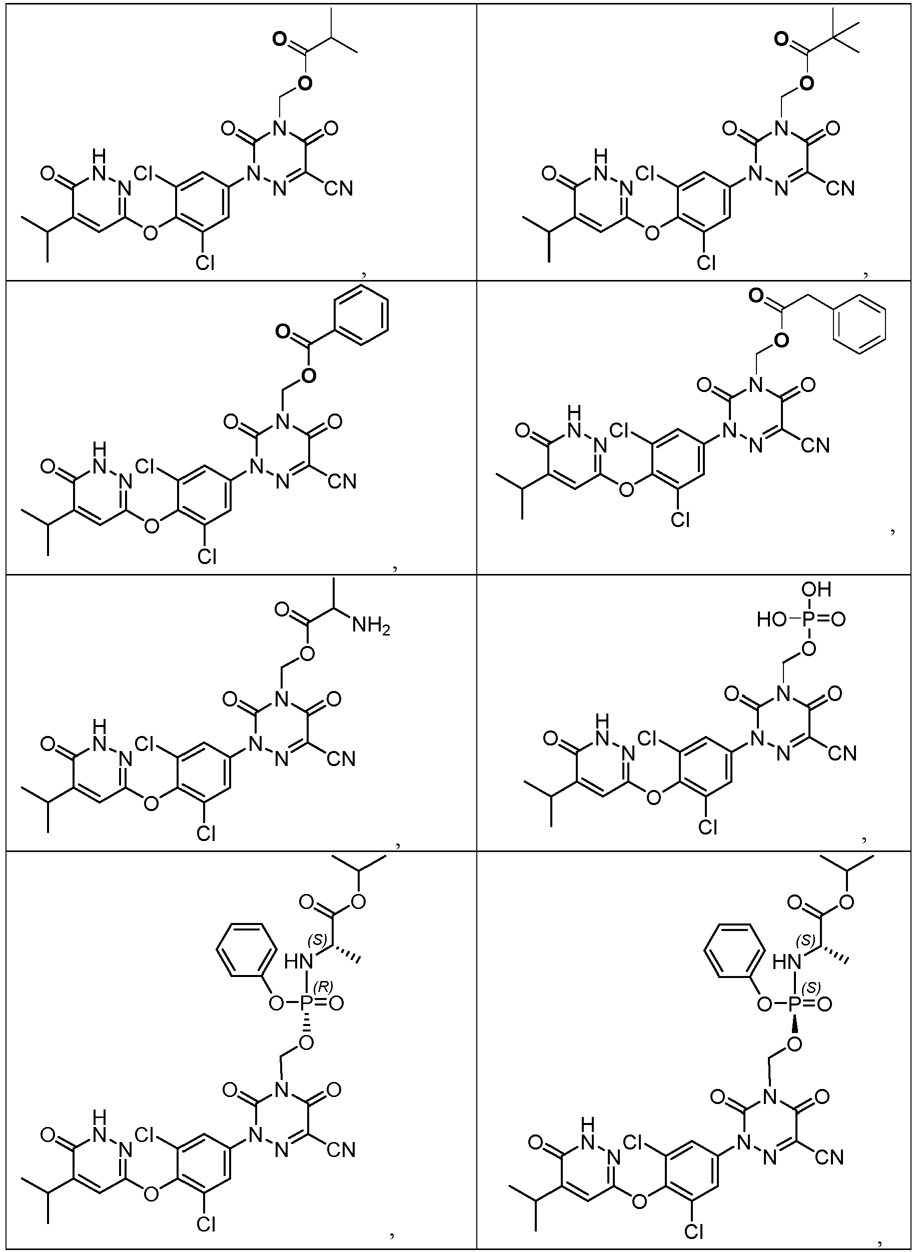

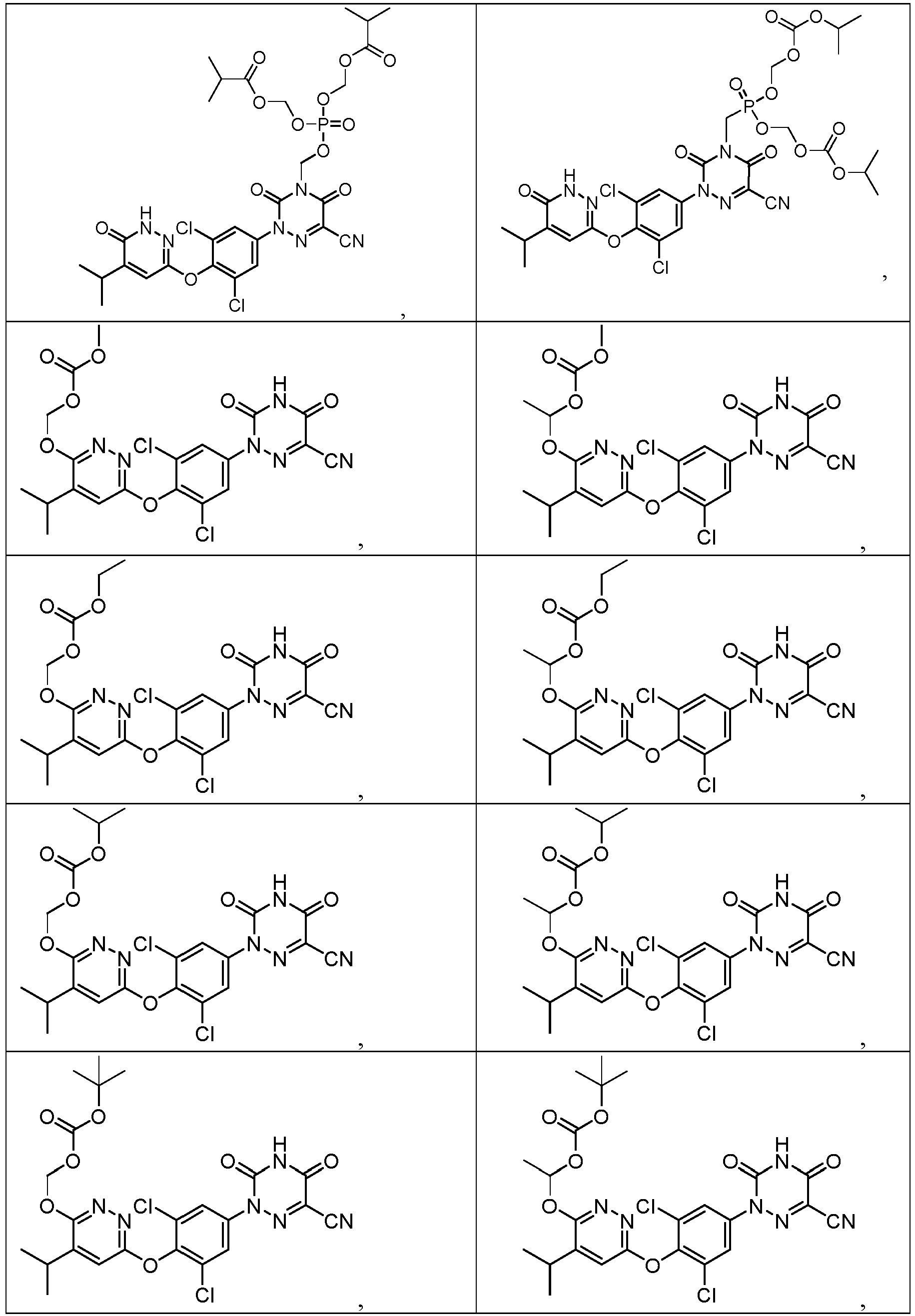

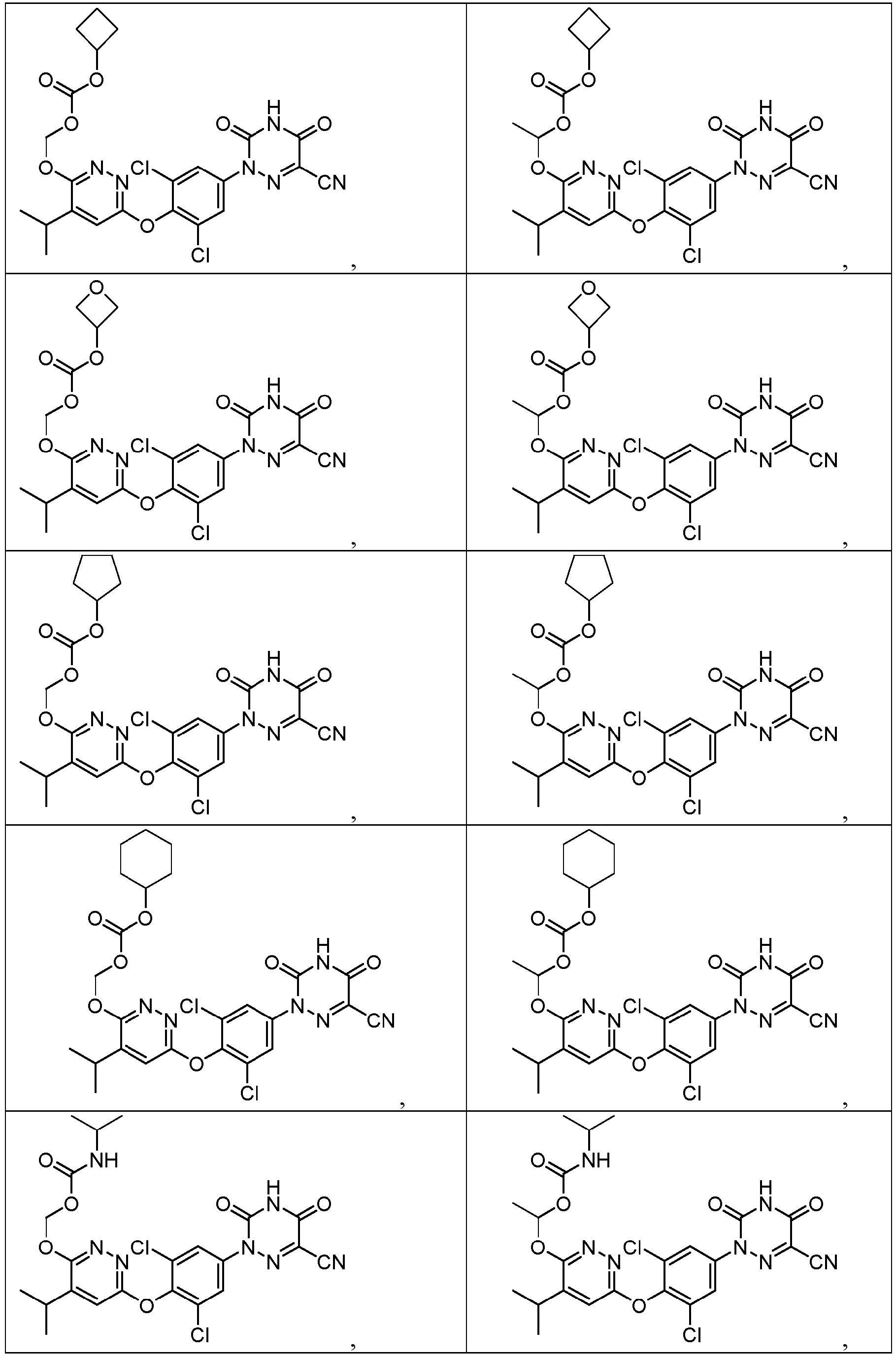

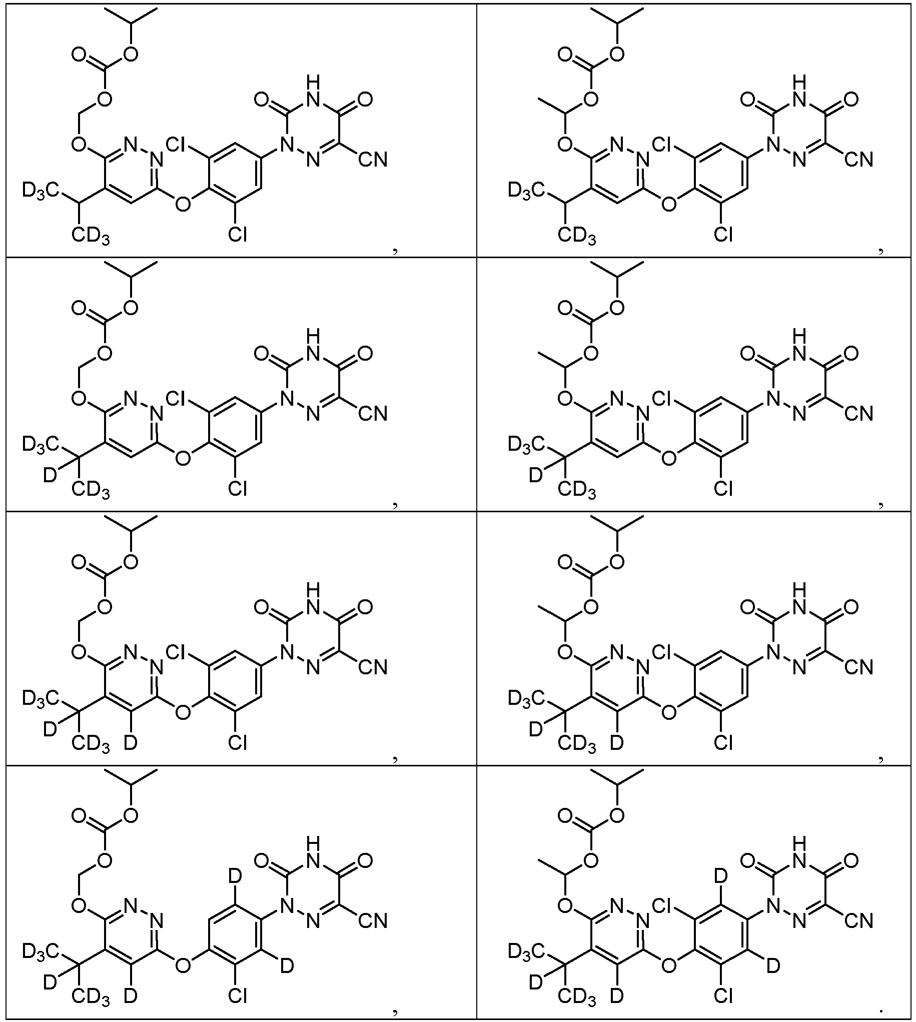

As used herein, the term "pharmaceutically acceptable salt" refers to a salt suitable for use as a medicament formed by the compound of the present invention with an acid or base. Pharmaceutically acceptable salts include inorganic salts and organic salts. Further, when the compound in the present invention contains a base fragment which includes, but is not limited to pyridine or imidazole, when contains an acid fragment which includes, but is not limited to carboxylic acid, the possible zwitter-ion ("inner salt") is included within the scope of the term "salt". Pharmaceutically acceptable (i.e., non-toxic, physiologically acceptable) salt is preferred, although other salts are also useful, for example, may be used in the separation or purification steps of the preparation process. The compound of the present invention may form a salt, for example, compound is reacted with a certain amount (such as an equivalent amount) of an acid or base, and the salt is precipitated from a medium or obtained by freeze-drying from aqueous solution.

Base fragment contained in the compound in the present invention includes but is not limited to amine or pyridine or imidazole ring, which may form salts with organic or inorganic acids. Typical acid salts that can be salted include acetate (such as with acetic acid or trihaloacetic acid, such as trifluoroacetic acid), adipate, alginate, ascorbate, aspartate, benzoate, besylate, bisulfate, borate, butyrate, citrate, camphorate, camphorsulfonate, cyclopentane propionate, diglycolate, dodecyl sulfate, ethanesulfonate, fumarate, glucoheptonate, glycerophosphate, hemisulphate, heptanoate, hexanoate, hydrochloride, hydrobromide, hydroiodrate, hydroxyethyl sulfonate (e.g., 2-hydroxyethyl sulfonate), lactate, maleate, methanesulfonate, naphthalenesulfonate (e.g., 2-naphthalenesulfonate), nicotinate, nitrate, oxalate, pectinate, persulfate, phenylpropionate (such as 3-phenylpropionate), phosphate, picrate, pivalate, propionate, salicylate, succinate, sulfate (such as formed from sulfuric acid), sulfonate, tartrate, thiocyanate, toluenesulfonate (such as *p*-toluenesulfonate), dodecanoate, and the like.

Acidic fragment contained in some compound of the present invention includes but is not limited to carboxylic acid, which may form salts with various organic or inorganic bases. Salts formed by typical base include ammonium salt; alkali metal salt such as sodium, lithium and potassium salt; alkaline earth metal salt such as calcium and magnesium salt; and salt formed by organic bases (such as organic amines), such as benzathine, dicyclohexylamine, hydrabamine (salt formed from *N*,*N*-bis(dehydroabietyl)ethylenediamine), *N*-methyl-D-glucanamine, *N*-methyl-D-glucoamide, tert-butyllamine; and salt formed with amino acids such as arginine, lysine, etc.. Basic nitrogen-containing groups can form quaternary ammonium salts with halides, such as small molecular alkyl halides (such as chlorides, bromides and iodides of methyl, ethyl, propyl and butyl); dialkyl sulfate (such as dimethyl, diethyl, dibutyl, and dipentyl sulfates); long chain halides (such as chlorides, bromides and iodides of decyl, dodecyl, tetradecyl, and tetradecyl), aralkyl halides (such as bromides of benzyl and phenyl), and the like.

The term "solvate" refers to a complex with particular proportion formed by the coordination of the compound of the present invention with solvent molecule. "Hydrate" refers to a complex formed by the coordination of the compound of the present invention with water.

Compound, salt or solvate in the present invention, may be present in tautomeric forms such as amide and imino ether. All of these tautomers are part of the present invention.

Stereisomers (e.g., those asymmetric carbon atoms that may be present due to various substitutions) of all compounds, include their enantiomeric forms and non-enantiomed forms, which all belong to the protection scope of the present invention. The independent stereoisomer in the present invention may not coexist with other isomers (e.g., as a pure or substantially pure optical isomer with special activity), or may be a mixture, e.g., racemate, or mixture formed with all other stereoisomers or a part thereof. The chiral center of the present invention has two configurations of S or R, which is recommended defined by International Union of Pure and Applied Chemistry (IUPAC) in 1974. The racemization form can be solved by physical methods, such as fractional crystallization, or separation crystallization by derivation into diastereomers, or separation by chiral column chromatography. Individual optical isomer can be obtained from racemate by appropriate methods, including but not limited to conventional methods, such as recrystallization after salting with optically active acid.

Weight content of compound in the present invention obtained by preparation, separation and purification in turn is equal to or greater than 90%, such as equal to or greater than 95%, equal to or greater than 99% ("very pure" compound), and listed in the description of the text. The "very pure" compound of the present invention herein is also part of the present invention.

All configuration isomers of the compound of the present invention are within the scope, whether in mixture, pure or very pure form. The compound of the present invention comprises cis (Z) and trans (E) olefin isomers, and cis and trans isomers of carbocyclic ring and heterocyclic ring.

In the entire specification, the groups and substituents can be selected to provide stable fragments and compounds.

Specific functional groups and chemical term definitions are described as follows in detail. For the present invention, the chemical elements are consistent with those defined in Periodic Table of the Elements, CAS version, Handbook of Chemistry and Physics, 75th Ed. The definitions of particular functional groups are also described therein. In addition, the basic principles of organic chemistry as well as specific functional groups and reactivity are described in "Organic Chemistry", Thomas Sorrell, University Science Books, Sausalito: 1999, the entire content of which is incorporated herein by reference.

Some compounds of the present invention may exist in specific geometric or stereoisomer forms. The present invention covers all compounds, including their cis and trans isomers, R and S enantiomers, diastereomers, (D) type isomers, (L) type isomers, racemic mixtures and other mixtures. In addition, asymmetric carbon atom can represent substituent, such as alkyl. All isomers and mixtures thereof are included in the present invention.

According to the invention, mixtures of isomers may contain a variety ratio of isomers. For example, mixtures with only two isomers may have the following combinations: 50:50, 60:40, 70:30, 80:20, 90:10, 95:5, 96:4, 97:3, 98:2, 99:1, or 100:0, all ratios of the isomers are within the scope of the present invention. Similar ratios readily understood by those of ordinary skill in the art and more complex ratios of mixture of isomers are also within the scope of the present invention.

The present invention also includes isotope labeled compounds, which are disclosed herein equivalent to the original compounds. However, in practice, it usually occurs when one or more atoms are substituted by atoms with a different atomic weight or mass number. Examples that may be listed in the isotopes of the compound of the present invention include hydrogen, carbon, nitrogen, oxygen, phosphorus, sulfur, fluorine and chlorine isotopes, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl. The compound, or enantiomer, diastereomer, isomer, or pharmaceutically acceptable salt or solvate in the present invention, containing above compound isotopes or other isotope atoms are all within the scope of the invention. Some isotope-labeled compounds in the present invention, such as the radioactive isotopes of ³H and ¹⁴C, are also included and are useful in experiments on the tissue distribution of drugs and substrates. Tritium (³H) and Carbon-14 (¹⁴C), which are relatively easy to be prepared and detected, are the first choice among the isotopes. In addition, heavier isotope substitutions such as deuterium, i.e. ²H, have advantages in certain therapies due to their good metabolic stability, such as increased half-life *in vivo* or reduced dosage, and thus may be preferred in certain situations. Isotope-labeled compounds can be prepared by conventional methods through substituting readily available isotope-labeled reagents for non-isotopic reagents, and can be prepared using the scheme disclosed in the figures and (or) examples.

If the synthesis of a specific enantiomer of the compound of the invention is to be designed, it can be prepared by asymmetric synthesis, or derivatized with chiral adjuvant, then the resulting diastereomeric mixture is separated and the chiral adjuvant is removed to obtain pure enantiomer. In addition, if a molecule contains basic functional group such as amino acid, or acidic functional group such as carboxyl group, a diastereomer salt can be formed with suitable optically active acid or base, which can be separated by conventional means, such as crystallization or chromatography, to obtain a pure enantiomer.

As described herein, the compound in the present invention may be substituted with any number of substituents or functional groups to extend its scope. In general, whether the term "substituted" appears before or after the term "optional", the general formula that includes substituents in the compound formula of the present invention means the substitution of a specified structural substituent for a hydrogen radical. When multiple positions in a particular structure are substituted by multiple specific substituents, each position of the substituent can be the same or different. The term "substituted" as used herein includes all substitution that allows organic compounds to be substituted. Broadly speaking, the allowable substituents include non-cyclic, cyclic, branched, non-branched, carbocyclic and heterocyclic, aromatic ring and non-aromatic organic compounds. In the present invention, for example, heteroatom nitrogen, its valence state may be supplemented by a hydrogen substituent or by any permitted organic compound described above. Furthermore, the present invention is not intended to limit the allowable substituted organic compounds in any way. The present invention believes that the combination of substituents and variable groups is good for the treatment of diseases, such as infectious or proliferative diseases, in the form of stable compounds. The term "stable" herein refers to a compound that is stable enough to be tested for a sufficient period of time to maintain the structural integrity of the compound, preferably effective for a sufficient period of time, which is hereby used for the above purposes.

The metabolites of the compounds of the present invention and pharmaceutically acceptable salts thereof, and compounds that can be converted into the compounds of the present invention *in vivo,* are also within the scope of the present invention.

### Preparation method

The preparation method of the compound of formula (I) or (Ia) of the present invention is described in more detail below, but these specific methods do not constitute any limitation to the present invention. The compounds of the present invention can also be conveniently prepared by optionally combining various synthetic methods described in this specification or known in the art, and such combinations can be easily performed by those skilled in the art to which the present invention belongs.

Typically, the preparation process of the compounds of the present invention is as follows, wherein unless otherwise specified, the raw materials and reagents used can be commercially available.

Exemplarily, the compound represented by formula (I) is prepared as follows:

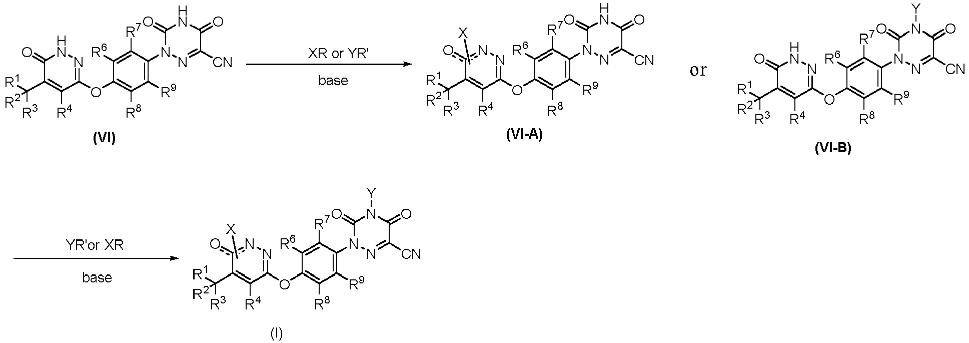

A compound of formula (VI) is reacted with XR or YR' (R, R' are leaving groups, such as Cl, Br or I) under alkaline condition (such as NaH, cesium carbonate, triethylamine or DIPEA, etc.) to obtain an intermediate of formula (VI-A) or formula (VI-B); and the intermediate is further reacted with YR' or XR (R', R are leaving groups, such as Cl, Br or I) under alkaline condition (such as NaH, cesium carbonate, triethylamine or DIPEA, etc.) to obtain the target compound (I).

Or the compound represented by formula (I) is synthesized as the following steps:

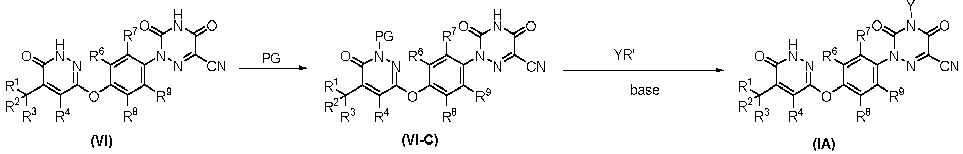

A compound of formula (VI) is protected with protecting group (PG is a protecting group, such as SEM or THP) to obtain an intermediate (VI-C), and then the intermediate (VI-C) is reacted with YR' under alkaline condition to obtain the compound of formula (I) (X is hydrogen).

### Pharmaceutical composition and administration method

The present invention also provides a pharmaceutical composition, the composition comprises:
1) a therapeutically effective amount of the pyridazinone or pyridazine compound represented by formula (I) or (Ia) of the present invention or the stereoisomer, tautomer, enantiomer, diastereomer, resonator, pharmaceutically acceptable salt, hydrate, solvate, or crystal form thereof; and
2) a pharmaceutically acceptable carrier.

In another preferred embodiment, the content of the pyridazinone or pyridazine compound represented by formula (I) or (Ia), or a stereoisomer, tautomer, enantiomer, diastereomer, resonate, pharmaceutically acceptable salt, hydrate, solvate, or crystal form thereof is 0.01-99.99 wt.%, preferably 0.1-99.9 wt.%, more preferably 1-99wt.%, more preferably 5-95 wt.%, more preferably 10-90 wt.%, more preferably 20-80 wt.%, most preferably 30-70 wt.%, based on the weight of the composition.

The present invention provides a method for preparing a pharmaceutical composition, comprising the steps of: mixing pharmaceutically acceptable carrier with pyridazinone or pyridazine compound represented by formula (I) or (Ia) of the present invention, or the stereoisomer, tautomer, enantiomer, diastereomer, resonate, pharmaceutically acceptable salt, hydrate, solvate, crystal form or prodrug thereof, thereby forming the pharmaceutical composition.

It should be understood that the compound of formula (I) or (Ia) may be used in combination with other drugs known to treat or ameliorate similar conditions. When administered in combination, the administration pattern and dosage of the original drug can remain unchanged, and the compound of formula (I) or (Ia) may be administered simultaneously or subsequently. When the compound of formula (I) or (Ia) is administered simultaneously with one or more other drugs, a pharmaceutical composition containing one or more known drugs and the compound of formula (I) or (Ia) can be preferably used. The drug combination also includes administering the compound of formula (I) or (Ia) and other one or more known drugs at overlapping time. When the compound of formula (I) or (Ia) is administered in a pharmaceutical combination with one or more other drugs, the dose of the compound of formula (I) or (Ia) or known drug may be lower than that of their individual use.

Other active ingredients described in the present invention include but are not limited to bile acid receptor (FXR) agonists (such as Obeticholic acid, Tropifexor, GS-9674, ZG5266), peroxisome proliferative activation receptor (PPAR) agonists (such as Elafibranor, Saroglitazar, Remogliflozin Etabonate), thyroid hormone receptor β (THRβ) agonists (such as MGL-3196), diacylglycerol-O-acyltransferase (DGAT) inhibitors (such as Pradigast, PF-06865571), acetyl-CoA carboxylase (ACC) inhibitors (such as GS-0976, PF-05221304), caspase inhibitors (such as Emricasan), smoothened receptor (SMO) inhibitors (such as Vismodegib), galectin inhibitors (such as GR-MD-02), C-C chemokine receptor 2 and 5 (such as CCR2 and CCR5) double antagonists (such as Cenicriviroc), ketohexokinase (KHK) inhibitors (PF-06835919), glucagon-like peptide-1 (GLP-1) receptor agonists (such as Liraglutide, semaglutide), lysyl oxidase like protein 2 (LOXL2) monoclonal antibody (such as simtuzumab), composite of bile acid and arachidonic acid (Aramchol), and the like.

The pharmaceutical composition of the invention comprises the compound of the present invention or pharmaceutically acceptable salt thereof in a safe and effective amount and pharmacologically acceptable excipient or carrier. Wherein the "safe and effective amount" means that the amount of compound is sufficient to significantly ameliorate the condition without causing significant side effects. Generally, the pharmaceutical composition contains 0.1-2000 mg of the compound of the invention per dose, preferably, 1-1000 mg of the compound of the invention per dose. Preferably, the "one dose" is a capsule or tablet.

"Pharmaceutically acceptable carrier" means one or more compatible solids or liquid fillers, or gelatinous materials which are suitable for human use and should be of sufficient purity and sufficiently low toxicity. "Compatibility" as used herein means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

The administration mode of the compound or pharmaceutical compositions of the present invention is not particularly limited, and the representative administration modes include (but are not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain adjuvants such as wetting agents, emulsifiers and suspending agents, sweeteners, flavoring agents and perfuming agents.

In addition to the active compounds, the suspension may contain suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms of the compounds of the invention for topical administration include ointments, powders, patches, sprays and inhalants. The active ingredient is mixed with physiologically acceptable carriers and any preservatives, buffers, or propellants that may be required if necessary under sterile conditions.

The compound of formula (I) or (Ia) in the present invention can be administrated alone, or in combination with other pharmaceutically acceptable compounds (such as antitumor drugs).

The therapeutic methods of the present invention involve administration alone or combination therapy in combination with other therapeutic means or therapeutic agents.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000 mg, preferably 50-1000 mg. Of course, the particular dose should also depend on various factors, such as the route of administration, patient healthy status, which are well within the skills of an experienced physician.

Inflammation, cardiovascular diseases, infection, immune diseases, metabolic diseases or cancer involved in the present invention include (but are not limited to) primary biliary cirrhosis (PBC), primary sclerosing cholangitis (PSC), cholestasis, autoimmune hepatitis, viral hepatitis (such as hepatitis B), alcoholic liver disease, non-alcoholic fatty liver disease (NAFLD), non-alcoholic fatty steatohepatitis (NASH) or liver fibrosis; arteriosclerosis, dyslipidemia, hypercholesterolemia or hypertriglyceridemia; type I diabetes, type II diabetes or obesity; lung cancer, breast cancer, prostate cancer, esophageal cancer, colorectal cancer, blood cancer, bone cancer, kidney cancer, gastric cancer, liver cancer, bowel cancer.

### Use

The present invention also provides a use of a pyridazinone or pyridazine compound represented by formula (I) or (Ia) or a stereoisomer, tautomer, enantiomer, diastereomer, resonator, pharmaceutically acceptable salt, hydrate, solvate, or crystal form thereof, or a pharmaceutical composition of the present invention, for (i) preparing thyroid hormone receptor agonist pharmaceutical preparation or drug; (ii) preparing pharmaceutical preparation or drug for a disease related to decreased thyroid hormone receptor activity; and/or (ii) preparing pharmaceutical preparation or drug for preventing and/or treating a disease including, but not limited to, inflammation, cancer, cardiovascular disease, infection, immunological disease, metabolic disease, and a combination thereof.

In the present invention, the term "prevent" refers to a method of preventing the onset of a disease and/or its attendant symptoms or protecting a subject from acquiring a disease. "Prevent" as used herein also includes delaying the onset of the disease and/or its attendant symptoms and reducing the risk of a disease in a subject.

In the present invention, the term "treat" refers to any treatment of a disease in a mammal, including but not limited to: (a) inhibiting the disease, ie, slowing or preventing the development of clinical symptoms; and/or (b) alleviating the disease, ie, causing regression of clinical symptoms, and/or (c) alleviating or eliminating the disease and/or its attendant symptoms.

In a preferred embodiment, the thyroid hormone receptor is thyroid hormone α receptor and/or thyroid hormone β receptor. Preferably, the thyroid hormone receptor is thyroid hormone β receptor.

In the invention, the diseases related to decreased thyroid hormone receptor activity include (but are not limited to) inflammation, cancer, cardiovascular disease, infection, immunological disease, metabolic disease, and a combination thereof.

In another preferred embodiment, the diseases include (but are not limited to) non-alcoholic steatohepatitis, non-alcoholic fatty liver disease, liver fibrosis, live cirrhosis (such as primary biliary cirrhosis), gallstone, atherosclerosis, obesity, hyperlipidemia, diabetes.

In another preferred embodiment, the hyperlipidemia is high triglyceride and/or high cholesterol.

In another preferred embodiment, the obesity is obesity caused by high fat diet.

In another preferred embodiment, the diseases include (but are not limited to) primary biliary cirrhosis (PBC), primary sclerosing cholangitis (PSC), cholestasis, autoimmune hepatitis, viral hepatitis (such as hepatitis B), alcoholic liver disease, non-alcoholic fatty liver disease (NAFLD), non-alcoholic steatohepatitis (NASH), liver fibrosis, arteriosclerosis, dyslipidemia, hypercholesterolemia, hypertriglyceridemia, type I diabetes, type II diabetes, obesity.

In another preferred embodiment, the cancers include (but are not limited to) lung cancer, breast cancer, prostate cancer, esophageal cancer, colorectal cancer, blood cancer, bone cancer, kidney cancer, gastric cancer, liver cancer, bowel cancer, and a combination thereof.

Typically, the compound of formula (I) or (Ia) described in the present invention is used for preparing drug for preventing and/or treating obesity.

The present invention further provides an *in vitro* non-therapeutic and non-diagnostic method for enhancing or improving thyroid hormone receptor activity, the method comprises the step of: in an *in vitro* culture system, thyroid hormone receptors or cells expressing thyroid hormone receptors are contacted with the pyridazinone or pyridazine compound represented by formula (I) or (Ia) according to the present invention, or a stereoisomer, tautomer, enantiomer, diastereomer, resonator, pharmaceutically acceptable salt, hydrate, solvate, or crystal form thereof, thereby enhancing or improving thyroid hormone receptor activity.

The present invention further provides a method of preventing and/or treating a disease including (but not limited to) inflammation, cancer, cardiovascular disease, infection, immunological disease, metabolic disease, diabetes, the method comprises the step of: an effective amount of the pyridazinone or pyridazine compound represented by formula (I) or (Ia) according to the present invention, or a stereoisomer, tautomer, enantiomer, diastereomer, resonator, pharmaceutically acceptable salt, hydrate, solvate, or crystal form thereof is administered to a patient in need thereof.

It should be understood in the present invention that the term "condition" includes diseases and their symptoms.

### Thyroid hormone receptor agonist

The present invention further provides a thyroid hormone receptor agonist, the agonist comprises agonized effective amount of a pyridazinone or pyridazine compound represented by formula (I) or (Ia) according to the present invention, or a stereoisomer, tautomer, enantiomer, diastereomer, resonator, pharmaceutically acceptable salt, hydrate, solvate, or crystal form thereof.

### The main advantages of the present invention include:

(1) The compound represented by formula (I) or (Ia) of the present invention has better metabolic properties and pharmacodynamic properties *in vivo* (such as in rats);
(2) The compound represented by the formula (I) or (Ia) of the present invention has a higher drug concentration in the liver tissue *in vivo* (eg, in rats), thereby having better pharmacodynamic properties.
(3) The compound represented by the formula (I) or (Ia) of the present invention or its metabolite has an excellent selective agonistic effect on the thyroid hormone β receptor.

The present invention will be further illustrated below with reference to the specific examples. It should be understood that these examples are only to illustrate the invention but not to limit the scope of the invention. The experimental methods with no specific conditions described in the following examples are generally performed under the conventional conditions such as conditions described in Sambrook et al., Molecular Cloning: A Laboratory Manual (New York: Cold Spring Harbor Laboratory Press, 1989), or according to the manufacturer's instructions. Unless indicated otherwise, percentages and parts are calculated by weight.

Unless otherwise defined, all professional and scientific terms used herein have the same meanings as those familiar to those skilled in the art. In addition, any methods and materials similar or equivalent to those described can be used in the methods of the present invention. Preferred methods and materials described herein are provided for illustrative purposes only.

The structures of the compounds of the present invention were determined by nuclear magnetic resonance (NMR) and liquid chromatography-mass spectrometry (LC-MS).

NMR was detected by Bruker AVANCE-400 nuclear magnetic instrument, and the determination solvent included deuterated dimethyl sulfoxide (DMSO-d₆), deuterated acetone (CD₃COCD₃), deuterated chloroform (CDCl₃) and deuterated methanol (CD₃OD), etc. The internal standard is tetramethylsilane (TMS) and chemical shifts are measured in parts per million (ppm).

Liquid chromatography-mass spectrometry (LC-MS) was performed using an Agilent 1200 Infinity Series mass spectrometer. The HPLC assay was performed using an Agilent 100 high pressure chromatograph (Microsorb 5 micron C18 100 x 3.0 mm column).

Qingdao GF254 silica gel plate was used for thin layer chromatography, 0.15-0.20 mm for TLC and 0.4 mm-0.5 mm for preparative thin layer chromatography. 200-300 mesh silica gel from Yantai Huanghai silica gel was used as a carrier in column chromatography.

The starting materials in the examples of the present invention are all known and commercially available, or can be synthesized by adopting or according to the literature data reported in the field.

Unless otherwise specified, all the reactions of the present invention are carried out under the protection of dry inert gas (such as nitrogen or argon) by continuous magnetic stirring, and the unit of reaction temperature is Celsius.

### Example 1.

### Preparation of 2-(3,5-dichloro-4-((5-isopropyl-6-oxo-1,6-dihydropyridazin-3-yl)oxy) phenyl)-3,5-dioxo-2,3,4,5-tetrahydro-1,2,4-triazin-6-carbonitrile

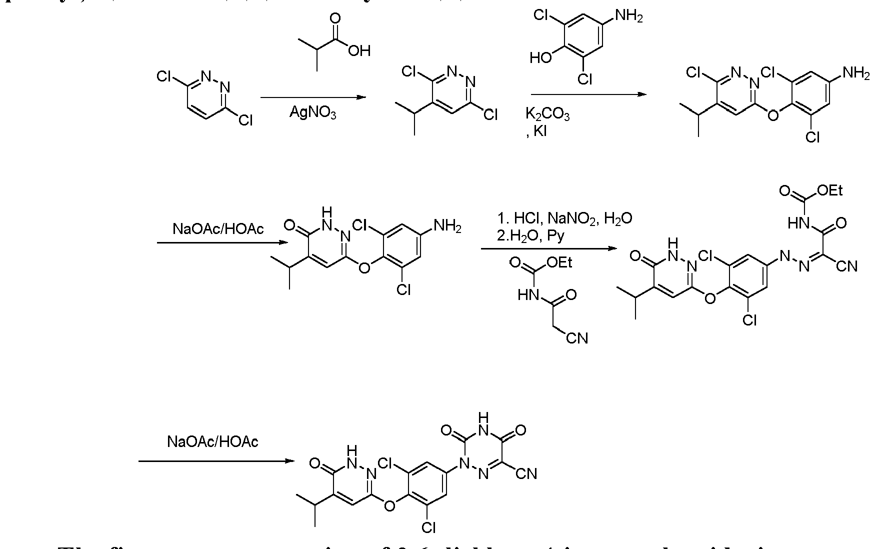

### The first step: preparation of 3,6-dichloro-4-isopropylpyridazine

Isobutyric acid (1.4 g, 0.0151 mol) was added into the mixed solution of 3,6-dichloropyridazine (2.25 g, 0.015 mol) in acetonitrile (3.5 mL), sulfolane (10.7 mL) and water (24.5 mL) at room temperature. Silver nitrate (1.3 g, 0. mol) was then added. The reaction mixture was heated to 55 °C and the solution of concentrated sulfuric acid (2.4 mL) in water (7.5 mL) was added in one portion, followed by dropwise addition of a solution of ammonium persulfate (5.2 g, 0.022 mol) in water (7.5 mL) within 35 min. The reaction mixture was reacted at 70 °C for 20 minutes and then cooled to room temperature, and stirred at room temperature for 24 hours. The obtained reaction mixture was cooled to 0 °C and the pH was slowly adjusted to 8 with aqueous ammonia (10 mL). The obtained mixture was diluted with water (50 mL) and filtered, and the filter cake was washed with ethyl acetate (50 mL). The organic phase was collected from the filtrate and the aqueous phase was extracted with ethyl acetate (2 x 50 mL). The combined organic phases were washed successively with water (40 mL) and saturated brine (40 mL), and then dried and filtered. The filtrate was concentrated in vacuum to obtain the crude product, which was then subjected to silica gel column chromatography to obtain the target compound (1.9 g, yield 67%).

LC-MS: m/z 191 (M+H)⁺.

### The second step: preparation of 3,5-dichloro-4-((6-chloro-5-isopropylpyridazin -3-yl)oxy)aniline

Under the protection of argon, 4-amino-2,6-dichlorophenol (0.5 g, 2.8 mmol), anhydrous potassium carbonate (1.6 g, 11.2 mmol) and cuprous iodide (0.32 g, 1.7 mmol) were added into a solution of 3,6-dichloro-4-isopropylpyridazine (0.54 g, 2.8 mmol) in anhydrous DMSO (2 mL) at room temperature. The reaction mixture was reacted at 90 °C for 24 hours, then cooled down to room temperature and poured into water (100 mL). The resulting mixture was adjusted to pH 8 with dilute hydrochloric acid (1 N), then ethyl acetate (50 mL) was added. The mixture was filtered through celite, and the filter cake was washed with ethyl acetate. The organic phase was separated and collected, and the aqueous phase was extracted with ethyl acetate. The combined organic phases were washed with saturated brine, dried and then filtered. The filtrate was concentrated under reduced pressure to obtain the target product (0.5 g, yield 53%) by silica gel column chromatography.

LC-MS: m/z 338 (M+H)⁺. ¹H NMR (400 MHz, DMSO-*d₆*) δ7.629 (s, 1H), 6.674 (s, 2H), 5.649 (brs, 2H),3.114 (m,1H), 1.237 (d, *J=* 6.8 Hz, 6H).

### The third step: preparation of 6-(4-amino-2,6-dichlorophenoxy)-4-isopropylpyridazin-3(2H)-one

A mixture of 3,5-dichloro-4-((6-chloro-5-isopropylpyridazin-3-yl)oxy)aniline (1.0 g, 3.0 mmol), glacial acetic acid (30 mL) and sodium acetate (860 mg , 10.5 mmol) was reacted at 100 °C for 24 hours, then cooled down to room temperature and stirred at room temperature for 2 days. The resulting mixture was diluted with water and then adjusted to pH 9 with aqueous sodium hydroxide (1 N). The obtained suspension was extracted with ethyl acetate. After the aqueous phase was separated, the pH was adjusted to 5 with concentrated hydrochloric acid, followed by extraction with ethyl acetate. The organic phases were combined, dried and then filtered. The filtrate was concentrated by vacuum drying. The obtained residue was diluted with methanol (20 mL), then aqueous sodium hydroxide (1 N, 20 mL, 20 mmol) was added. The obtained reaction mixture was reacted at 120 °C for 24 hours, then cooled down to room temperature and the solvent was removed under reduced pressure. The residue was diluted with water (100 mL), and then extracted with ethyl acetate. The organic phases were combined, washed with dilute aqueous hydrochloric acid (pH 5) and saturated brine, and then dried and filtered. The filtrate was concentrated under reduced pressure in vacuo, and the obtained residue was subjected to preparative chromatography to obtain the target compound (0.48 g, yield 50%).

LC-MS: m/z 314 (M+H)⁺. ¹H NMR (400 MHz, DMSO-*d₆*) δ 12.125 (s, 1H), 7.270 (s, 1H), 6.671 (s, 2H), 5.624 (brs, 2H), 3.013 (m, 1H), 1.177 (d, *J=* 6.8 Hz, 6H).

### The fourth step: preparation of ethyl (2-cyano-2-(2-(3,5-dichloro-4-((5-isopropyl -6-oxo-1,6-dihydropyridazin-3-yl))oxy)phenyl)hydrazone)acetyl)carbamate

Concentrated hydrochloric acid (2.8 mL) was added to a suspension of 6-(4-amino-2,6-dichlorophenoxy)-4-isopropylpyridazin-3(2H)-one (134 mg, 0.42 mmol) in water (5.6 mL). The reaction mixture was cooled to 0 °C and then a solution of sodium nitrite (36.5 mg, 0.529 mmol) in water (0.2 mL) was added. The resulting mixture was stirred at 0 °C for 30 minutes, then quickly filtered and quickly added to a mixed solution of N-cyanoaceturethane (72 mg, 0.46 mmol), water (9.4 mL) and pyridine (2.8 mL) pre-cooled to 0 °C. The obtained suspension was stirred at 0 °C for 30 minutes and then filtered. The solid was washed successively with water and petroleum ether, and then dried under vacuum at 80 °C overnight to obtain the target product (114 mg, yield 56%).

LC-MS: m/z 481 (M+H)⁺. ¹H NMR (400 MHz, DMSO-*d₆*) δ 12.25 (s, 1H), 12.22 (s, 1H), 10.89 (s, 1H), 7.99 (s, 2H), 7.36 (s, 1H), 4.21 (q, *J* = 7.1 Hz, 2H), 3.06 (m, 1H), 1.27 (t, *J* = 7.1 Hz, 3H), 1.20 (m, 6H).

### The fifth step: preparation of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazin-6-carbonit rile

Sodium acetate (0.3 g, 3.6 mmol) was added to a solution of ethyl (2-cyano-2-(2-(3,5-dichloro-4-((5-isopropyl-6-oxo-1,6-dihydropyridazin-3-yl)oxy)phenyl)hydrazone)acetyl)carb amate (0.35 g, 0.72 mmol) in glacial acetic acid (7.2 mL) at room temperature. The reactants were reacted for 1.5 hours at 120 °C, then cooled to 0 °C, diluted with water (20 mL) and stirred for 30 minutes. After filtration, the filter cake was washed successively with water and petroleum ether, and then dried for 30 minutes in air, and then slurried with acetonitrile and water to obtain the target product (0.16 g, yield 50%).

LC-MS: m/z 435 (M+H)⁺. ¹H NMR (400 MHz, DMSO-*d₆*) δ 13.200 (brs, 1H), 12.257 (s, 1H), 7.795 (s, 2H), 7.458 (s, 1H), 3.059 (m, 1H), 1.177 (d, *J* = 6.9 Hz, 6H).

### Example 2.

### Preparation of 1-(3-(2,6-dichloro-4-(6-cyano-3,5-dioxo-4,5-dihydro-1,2,4-triazin -2(3H)-yl)phenoxy)-5-isopropyl-6-oxopyridazin-1(6H)-yl)ethyl isopropyl carbonate and 1-((6-(2,6-dichloro-4-(6-cyano-3,5-dioxo-4,5-dihydro-1,2,4-triazin-2(3H)-yl)phenoxy)-4-i sopropylpyridazin-3-yl)oxy)ethyl isopropyl carbonate

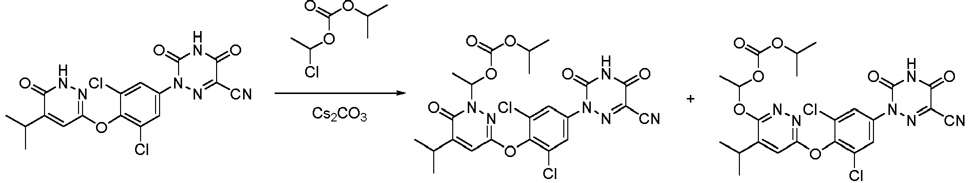

### The first step: preparation of 1-(3-(2,6-dichloro-4-(6-cyano-3,5-dioxo-4,5-dihydro-1,2,4-triazine-2(3H)-yl)phenoxy)-5-i sopropyl-6-oxopyridazin-1(6H)-yl)ethyl isopropyl carbonate and 1-((6-(2,6-dichloro -4-(6-cyano-3,5-dioxo-4,5-dihydro-1,2,4-triazin-2(3H)-yl)phenoxy)-4-isopropylpyridazin -3-yl)oxy)ethyl isopropyl carbonate

The compound 2-(3,5-dichloro-4-(5-(propyl-2-yl)-6-oxo-1,6-dihydropyridazin-3-yloxy) phenyl)-3,5-dioxo-2,3,4,5-tetrahydro-1,2,4-triazin-6-nitrile (500 mg, 1.15 mmol), cesium carbonate (747 mg, 2.30 mmol), 1-chloroethyl isopropyl carbonate (287 mg, 1.72 mmol) and N,N-dimethylformamide (20 mL) were added sequentially to the flask. The reaction solution was reacted at room temperature for 20 hours. The crude product was subjected to preparative liquid phase purification to obtain two compounds: compound **2A** (34 mg) and compound **2B** (77 mg).

### Compound 2A: 1-(3-(2,6-dichloro-4-(6-cyano-3,5-dioxo-4,5-dihydro-1,2,4-triazin -2(3H)-yl)phenoxy)-5-isopropyl-6-oxopyridazin-1(6H)-yl)ethyl isopropyl carbonate

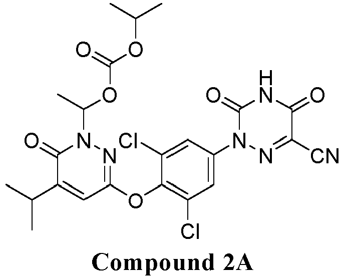

LC-MS: m/z 587 (M+Na)⁺. ¹H NMR (400 MHz, DMSO-*d₆*) δ7.82 (s, 2H), 7.53 (s, 1H), 6.89 (q, *J=* 6.0 Hz, 1H), 4.67-4.73 (m, 1H), 3.06-3.13 (m, 1H), 1.29 (d, *J=* 6.0 Hz, 3H), 1.21 (d, *J* = 7.2Hz, 6H), 1.18 (d, *J* = 6.4 Hz, 6H).

### Compound 2B: 1-((6-(2,6-dichloro-4-(6-cyano-3,5-dioxo-4,5-dihydro-1,2,4-triazin -2(3H)-yl)phenoxy)-4-isopropylpyridazin-3-yl)oxy)ethyl isopropyl carbonate

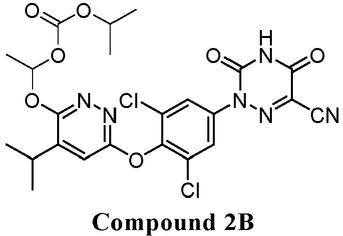

LC-MS: m/z 565 (M+H)⁺. ¹H NMR (400 MHz, DMSO-*d₆*) δ7.80 (s, 2H), 7.66 (s, 1H), 6.96 (q, *J=* 5.2Hz, 1H), 4.73-4.79 (m, 1H), 3.01-3.08 (m, 1H), 1.61 (d, *J=* 5.2 Hz, 3H), 1.24 (dd, *J* = 6.8 Hz, 12.4 Hz, 6H), 1.18 (dd, *J* = 6.4 Hz, 9.6 Hz, 6H).

### Example 3.

### Preparation of 1-(3-(2,6-dichloro-4-(6-cyano-3,5-dioxo-4,5-dihydro-1,2,4-triazin -2(3H)-yl)phenoxy)-6-oxo-5-(propan-2-yl-1,1,1,3,3,3-d6)pyridazin-1(6H)-yl)ethyl isopropyl carbonate and 1-((6-(2,6-dichloro-4-(6-cyano-3,5-dioxo-4,5-dihydro -1,2,4-triazin-2(3H)-yl)phenoxy)-4-(propan-2-yl-1,1,1,3,3,3-d6)pyridazin-3-yl)oxy)ethyl isopropyl carbonate

According to the preparation method of Example 1 and Example 2, compounds 3A and 3B were synthesized using 2-(trideuteromethyl)-3,3,3-trideuteropropionic acid instead of isobutyric acid as the starting material.

### Compound 3A: 1-(3-(2,6-dichloro-4-(6-cyano-3,5-dioxo-4,5-dihydro-1,2,4-triazin -2(3H)-yl)phenoxy)-6-oxo-5-(propan-2-yl-1,1,1,3,3,3-d6)pyridazin-1(6H)-yl)ethyl isopropyl carbonate

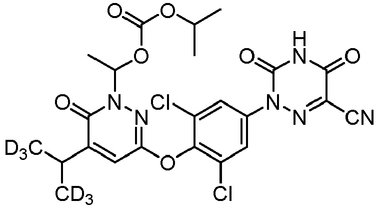

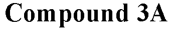

LC-MS: m/z 571(M+H)⁺.

### Compound 3B: 1-((6-(2,6-dichloro-4-(6-cyano-3,5-dioxo-4,5-dihydro-1,2,4-triazin -2(3H)-yl)phenoxy)-4-(propan-2-yl-1,1,1,3,3,3-d6)pyridazin-3-yl)oxy)ethyl isopropyl carbonate

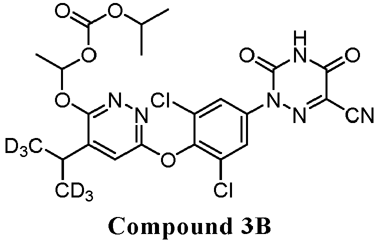

LC-MS: m/z 571(M+H).

### Example 4.

### Preparation of 1-(3-(2,6-dichloro-4-(6-cyano-3,5-dioxo-4,5-dihydro-1,2,4-triazin -2(3H)-yl)phenoxy)-6-oxo-5-(propan-2-yl-d7)pyridazin-1(6H)-yl)ethyl isopropyl carbonate and 1-((6-(2,6-dichloro-4-(6-cyano-3,5-dioxo-4,5-dihydro-1,2,4-triazin -2(3H)-yl)phenoxy)-4-(propane-2-yl-d7)pyridazin-3-yl)oxy)ethyl isopropyl carbonate

According to the preparation method of Example 1 and Example 2, compounds 4A and 4B were synthesized using 2-(methyl-d3)propyl-2,3,3,3-d4 acid instead of isobutyric acid as the starting material.

### Compound 4A: 1-(3-(2,6-dichloro-4-(6-cyano-3,5-dioxo-4,5-dihydro-1,2,4-triazin -2(3H)-yl)phenoxy)-6-oxo-5-(propan-2-yl-d7)pyridazin-1(6H)-yl)ethyl isopropyl carbonate

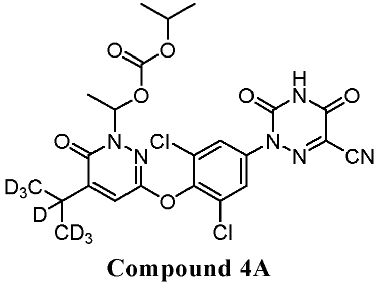

LC-MS: m/z 572 (M+H)⁺.

### Compound 4B: 1-((6-(2,6-dichloro-4-(6-cyano-3,5-dioxo-4,5-dihydro-1,2,4-triazin -2(3H)-yl)phenoxy)-4-(propan-2-yl-d7)pyridazin-3-yl)oxy)ethyl isopropyl carbonate

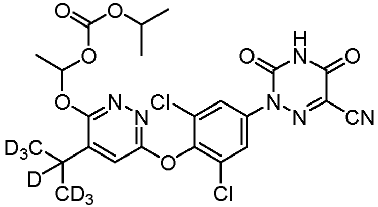

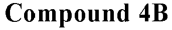

LC-MS: m/z 572 (M+H)⁺.

### Example 5

### Preparation of 1-(3-(2,6-dichloro-4-(6-cyano-3,5-dioxo-4,5-dihydro-1,2,4-triazin -2(3H)-yl)phenoxy)-6-oxo-5-(propan-2-yl-d7)pyridazin-1(6H)-yl-4-d)ethyl isopropyl carbonate and 1-((6-(2,6-dichloro-4-(6-cyano-3,5-dioxo-4,5-dihydro-1,2,4-triazin -2(3H)-yl)phenoxy)-4-(propan-2-yl-d7)pyridazin-3-yl-5-d)oxy)ethyl isopropyl carbonate

According to the preparation method of Example 1 and Example 2, compounds 5A and 5B were synthesized using 2-(methyl-d3)propyl-2,3,3,3-d4 acid instead of isobutyric acid and using 3,6-dichloropyridazine-4,5-d2 instead of 3,6-dichloropyridazine as the starting material.

### Compound 5A: 1-(3-(2,6-dichloro-4-(6-cyano-3,5-dioxo-4,5-dihydro-1,2,4-triazin -2(3H)-yl)phenoxy)-6-oxo-5-(propan-2-yl-d7)pyridazin-1(6H)-yl-4-d)ethyl isopropyl carbonate

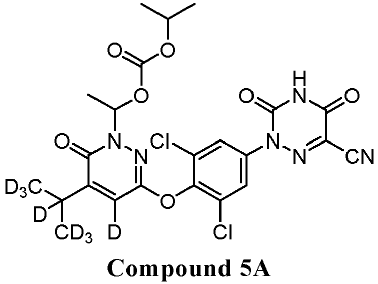

LC-MS: m/z 573 (M+H)⁺.

### Compound 5B: 1-((6-(2,6-dichloro-4-(6-cyano-3,5-dioxo-4,5-dihydro-1,2,4-triazin -2(3H)-yl)phenoxy)-4-(propan-2-yl-d7)pyridazin-3-yl-5-d)oxy)ethyl isopropyl carbonate

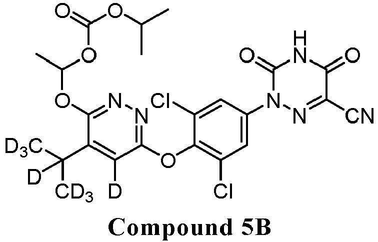

LC-MS: m/z 573 (M+H)⁺.

### Example 6

### Preparation of compound 6: (3-(2,6-dichloro-4-(6-cyano-3,5-dioxo-4,5-dihydro -1,2,4-triazin-2(3H)-yl)phenoxy)-5-isopropyl-6-oxopyridazin-1(6H)-yl)methyl isopropyl carbonate and (6-cyano-2-(3,5-dichloro-4-((5-isopropyl-6-oxo-1,6-dihydropyridazin -3-yl)oxy)phenyl)-3,5-dioxo-2,5-dihydro-1,2,4-triazin-4(3H)-yl)methyl isopropyl carbonate

According to the preparation method of Example 2, compound 6 was synthesized using chloromethyl isopropyl carbonate instead of 1-chloroethyl isopropyl carbonate as the starting material.

### Compound 6A: (3-(2,6-dichloro-4-(6-cyano-3,5-dioxo-4,5-dihydro-1,2,4-triazin -2(3H)-yl)phenoxy)-5-isopropyl-6-oxopyridazin-1(6H)-yl)methyl isopropyl carbonate

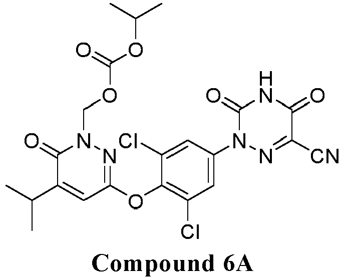

LC-MS: m/z 551 (M+H)⁺. ¹H NMR (400 MHz, DMSO-*d₆*) δ7.79 (s, 2H), 7.54 (s, 1H), 5.72 (s, 2H), 4.69-4.75 (m, 1H), 3.05-3.12 (m, 1H), 1.19-1.22 (m, 12H).

### Compound 6B: ((6-(2,6-dichloro-4-(6-cyano-3,5-dioxo-4,5-dihydro-1,2,4-triazin-2(3H)-yl)phenoxy)-4-iso propylpyridazin-3-yl)oxy)methyl isopropyl carbonate

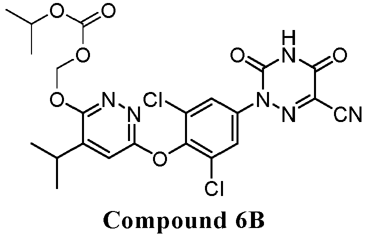

LC-MS: m/z 551 (M+H)⁺. ¹H NMR (400 MHz, DMSO-*d₆*) δ7.80 (s, 2H), 7.68 (s, 1H), 6.08 (s, 2H), 4.77-4.86 (m, 1H), 3.01-3.11 (m, 1H), 1.21-1.25 (m, 12H).

### Example 7

### Preparation of 1-(3-(2,6-dichloro-4-(6-cyano-3,5-dioxo-4,5-dihydro-1,2,4-triazin -2(3H)-yl)phenoxy)-5-isopropyl-6-oxopyridazin-1(6H)-yl)ethyl ethyl carbonate and 1-((6-(2,6-dichloro-4-(6-cyano-3,5-dioxo-4,5-dihydro-1,2,4-triazin-2(3H)-yl)phenoxy)-4-i sopropylpyridazin-3-yl)oxy )ethyl ethyl carbonate

According to the preparation method of Example 2, compound 7 was synthesized using 1-chloroethyl ethyl carbonate instead of 1-chloroethyl isopropyl carbonate as the starting material.

### Compound 7A: 1-(3-(2,6-dichloro-4-(6-cyano-3,5-dioxo-4,5-dihydro-1,2,4-triazin -2(3H)-yl)phenoxy)-5-isopropyl-6-oxopyridazin-1(6H)-yl)ethyl ethyl carbonate

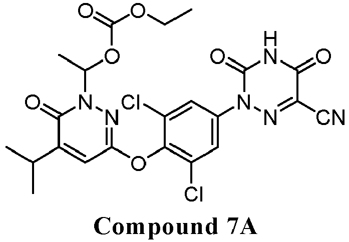

LC-MS: m/z 551 (M+H)⁺.

### Compound 7B: 1-((6-(2,6-dichloro-4-(6-cyano-3,5-dioxo-4,5-dihydro-1,2,4-triazin -2(3H)-yl)phenoxy)-4-isopropylpyridazin-3-yl)oxy)ethyl ethyl carbonate

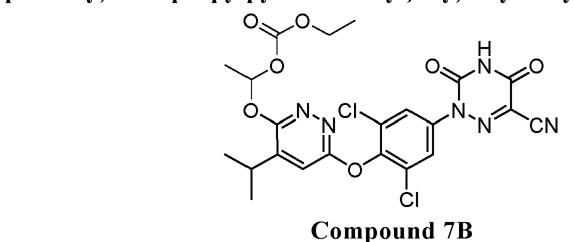

LC-MS: m/z 551 (M+H)⁺.

### Example 8

### Preparation of (6-cyano-2-(3,5-dichloro-4-((5-isopropyl-6-oxo-1,6-dihydropyridazin -3-yl)oxy)phenyl)-3,5-dioxo-2,5-dihyd ro- 1,2,4-triazin-4(3H)-yl)m ethyl isobutyrate

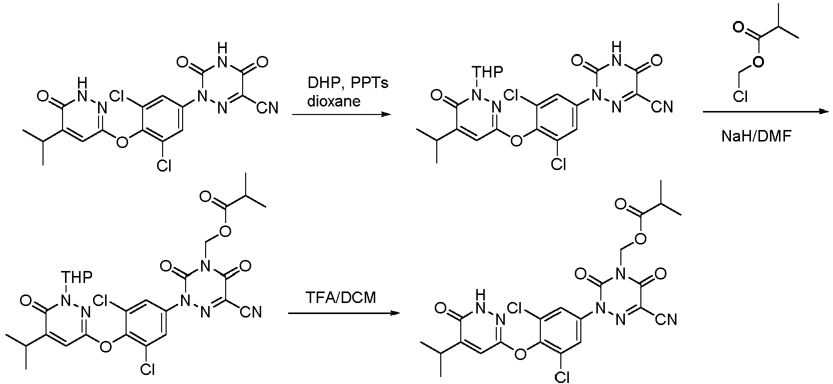

### The first step: preparation of 2-(3,5-dichloro-4-((5-isopropyl-6-oxo-1 -(tetrahydro-2H-pyran-2-yl)-1,6-dihydropyridazin-3-yl)oxy)phenyl)-3,5-dioxo-2,3,4,5-tet rahydro-1,2,4-triazin-6-carbonitrile

Compound 2-(3,5-dichloro-4-(5-(propyl-2-yl)-6-oxo-1,6-dihydropyridazin-3-yloxy) phenyl)-3,5-dioxo-2,3,4,5-tetrahydro-1,2,4-triazin-6-nitrile (500 mg, 1.15 mmol), pyridinium p-toluenesulfonate (29 mg, 0.16 mmol), 3,4-dihydropyran (386 mg, 4.6 mmol) and 1,4-dioxane (5 mL) were added sequentially to a Schlenk tube. After addition, the reaction solution was heated to 65 °C and reacted at this temperature for 16 hours. The obtained mixture was concentrated under reduced pressure, and the residue was subjected to preparative liquid phase purification to obtain the target compound (350 mg, yield 59%).

LC-MS: m/z 519 (M+H)⁺.

### The second step: preparation of (6-cyano-2-(3,5-dichloro-4-(5-(propyl -2-yl)-6-oxo-1-(tetrahydro-2H-pyran-2-yl)-1,6-dihydropyridazin-3-yloxy)phenyl)-3,5-dio xo-2,3-dihydro-1,2,4-triazin-4(5H)-yl)methyl isobutyrate

Compound 2-(3,5-dichloro-4-(5-(propyl-2-yl)-6-oxo-1-(tetrahydro-2H-pyran-2-yl)-1,6-dihydropyridazin-3-yloxy)phenyl)-3,5-dioxo-2,3,4,5-tetrahydro-1,2,4-triazin-6-carbonitrile (214 mg, 0.41 mmol) and N,N-dimethylformamide (10 mL) were added to a round bottom flask, followed by addition of sodium hydride (41 mg, 1.03 mmol). The resulting mixture was stirred for 10 min and then chloromethyl isobutyrate (113 mg, 0.82 mmol) was added dropwise. After the addition, the reaction solution was raised to 55 °C, and the reaction was carried out at this temperature for 17 hours. The obtained mixture was quenched with water, and then subjected to preparative purification to give the target compound (110 mg, yield 43%).

LC-MS: m/z 663 (M+HCOO⁻)⁻.

### The third step: preparation of (6-cyano-2-(3,5-dichloro-4-((5-isopropyl-6-oxo-1,6-dihydropyridazin-3-yl)oxy)phenyl)-3,5-dioxo-2,5-dihydro-1,2,4-triazin-4(3H)-yl)methyl isobutyrate

Compound (6-cyano-2-(3,5-dichloro-4-(5-(propyl-2-yl)-6-oxo-1-(tetrahydro-2H-pyran) -2-yl)-1,6-dihydropyridazin-3-yloxy)phenyl)-3,5-dioxo-2,3-dihydro-1,2,4-triazin-4(5H)-yl)m ethyl isobutyrate (110 mg, 0.18 mmol) and dichloromethane (10 mL) were added to a round bottom flask. Trifluoroacetic acid (1 mL) was then added dropwise. The reaction solution was reacted at room temperature for 6 hours after the addition, then quenched and concentrated under reduced pressure. The obtained crude product was subjected to preparative liquid phase purification to give the target compound (31 mg, yield 33%).

LC-MS: m/z 535 (M+H)⁺_{∘} ¹H NMR(400 MHz, DMSO-*d₆*)δ12.22 (s, 1H), 7.80 (s, 2H), 7.45 (s,

1H), 5.85 (s, 2H), 3.11-3.00 (m, 1H), 2.62-2.54 (m, 1H), 1.20 (d, J=7.2Hz, 6H), 1.10 (d, J=6.8Hz, 6H).

**The compounds of Examples 9-10 were synthesized using the same method with different starting materials according to Example 8:**

### Example 9 preparation of (6-cyano-2-(3,5-dichloro-4-((5-isopropyl-6-oxo-1,6-dihydropyridazin-3-yl))oxy)phenyl)-3,5-dioxo-2,5-dihydro-1,2,4-triazin-4(3H)-yl)methyl pivalate

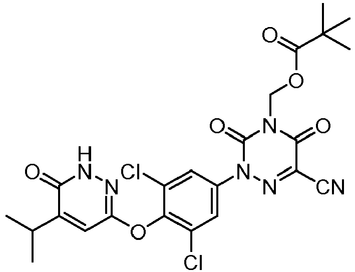

LC-MS: m/z 549 (M+H)⁺. ¹H NMR (400 MHz, DMSO-*d₆*) δ12.24 (s, 1H), 7.80 (s, 2H), 7.45 (s, 1H), 5.83 (s, 2H), 3.09-3.02 (m, 1H), 2.62-2.54 (m, 1H), 1.24-1.16 (m, 15H).

### Example 10 preparation of (6-cyano-2-(3,5-dichloro-4-((5-isopropyl-6-oxo-1,6-dihydropyridazin-3-yl)oxy)phenyl)-3,5-dioxo-2,5-dihydro-1,2,4-triazin-4(3H)-yl)methyl L-alaninate trifluoroacetate

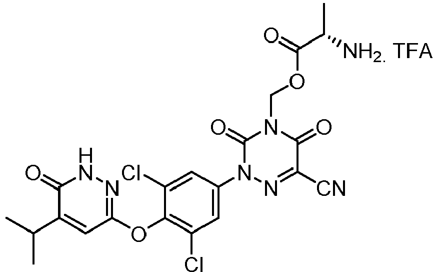

LC-MS: m/z 536 (M+H)⁺. ¹H NMR (400 MHz, DMSO-*d₆*) δ12.19 (s, 1H), 8.38 (s, 3H), 7.75 (s, 2H), 7.36 (s, 1H), 5.91 (dd, *J* = 10.4 Hz, 19.2 Hz, 2H), 4.07-4.08 (m, 1H), 2.94-3.01 (m, 1H), 1.32 (d, *J* = 7.2 Hz, 3H), 1.12 (d, *J* = 6.8 Hz, 6H).

### Example 11 preparation of ((6-cyano-2-(3,5-dichloro-4-((5-isopropyl-6-oxo-1-(tetrahydro-2H-pyran-2-yl)-1,6-dihydropyridazin-3-yl)oxy)phenyl)-3,5-dioxo-2,5-dihydr o-1,2,4-triazin-4(3H)-yl)methyl)dihydrophosphate

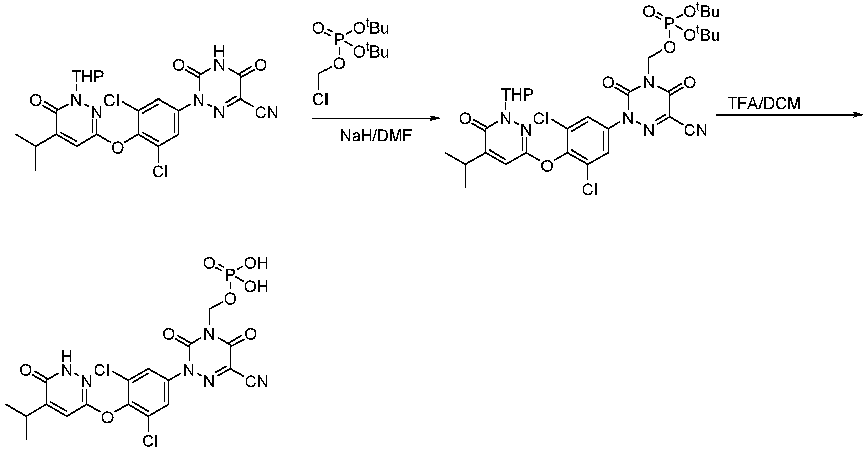

### The first step: preparation of di-tert-butyl ((6-cyano-2-(3,5-dichloro-4-((5-isopropyl -6-oxo-1-(tetrahydro-2H-pyran-2-yl))-1,6-dihydropyridazin-3-yl)oxy)phenyl)-3,5-dioxo-2,5-dihydro-1,2,4-triazin-4(3H)-yl)methyl)phosphate

Compound 2-(3,5-dichloro-4-(5-(propyl-2-yl)-6-oxo-1-(tetrahydro-2H-pyran-2-yl)-1,6-dihydropyridazin-3-yloxy)phenyl)-3,5-dioxo-2,3,4,5-tetrahydro-1,2,4-triazin-6-nitrile (300 mg, 0.58 mmol) and N,N-dimethylformamide (10 mL) were added to a round bottom flask, followed by the addition of sodium hydride (58 mg, 1.44 mmol). The reaction solution was stirred at room temperature for 10 min, and then di-tert-butyl chloromethyl phosphate (299 mg, 1.16 mmol) was added dropwise. After the dropwise addition, the reaction solution was raised to 65 °C and reacted for 46 hours. The obtained mixture was quenched with water, and then subjected to preparative purification to give the target compound (15 mg, yield 4%).

LC-MS: m/z 785 (M+HCOO⁻)⁻.

### The second step: preparation of ((6-cyano-2-(3,5-dichloro-4-((5-isopropyl-6-oxo-1-(tetrahydro-2H-pyran-2-yl)-1,6-dihyd ropyridazin-3-yl)oxy)phenyl)-3,5-dioxo-2,5-dihydro-1,2,4-triazin-4(3H)-yl)methyl)dihyd rophosphate

Compound di-tert-butyl(6-cyano-2-(3,5-dichloro-4-(5-(propyl-2-yl)-6-oxo-1-(tetrahydro -2H-pyran-2-yl)-1,6-dihydropyridazin-3-yloxy)phenyl)-3,5-dioxo-2,3-dihydro-1,2,4-triazin-4 (5H)-yl)methyl phosphate (15 mg, 0.02 mmol) and dichloromethane (3 mL) were added to a round bottom flask. Trifluoroacetic acid (0.2 mL) was then added dropwise, and the reaction was carried out at room temperature for 5 hours after the addition. The obtained mixture was subjected to preparation purification to obtain the target compound (6 mg, yield 54%).

LC-MS: m/z 545 (M+H)⁺. ¹H NMR (400 MHz, DMSO-*d₆*): δ 12.25 (s, 1H), 7.81 (s, 2H), 7.45 (s, 1H), 5.51 (d, *J=* 7.2 Hz, 2H), 3.05 (m, 1H), 1.20 (d, *J=* 6.8 Hz, 6H)_{∘} ³¹P NMR (162 MHz, DMSO-*d₆*): δ-3.2 (s).

### Example 12 preparation of (6-cyano-2-(3,5-dichloro-4-((1-((isobutyryloxy)methyl) -5-isopropyl-6-oxo-1,6-dihydropyridazin-3-yl)oxy)phenyl)-3,5-dioxo-2,5-dihydro-1,2,4-tr iazin-4(3H)-yl)methyl isobutyrate

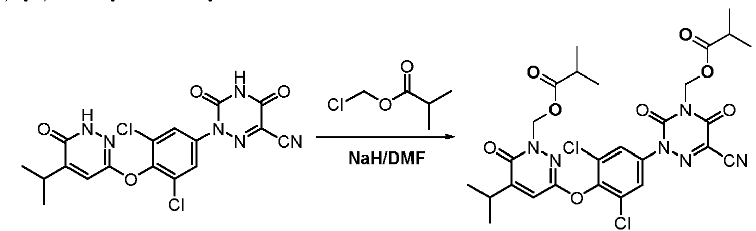

Compound 2-(3,5-dichloro-4-(5-(propyl-2-yl)-6-oxo-1,6-dihydropyridazin-3-yloxy) phenyl)-3,5-dioxo-2,3,4,5-tetrahydro-1,2,4-triazin-6-nitrile (50 mg, 0.12 mmol) and N,N-dimethylformamide (3 mL) were added to a Schlenk tube, stirred to dissolve, and then sodium hydride (14 mg, 0.35 mmol) was added. The obtained mixture was stirred at room temperature for 10 minutes, and then chloromethyl isobutyrate (63 mg, 0.46 mmol) was added dropwise. After the addition, the reactants were reacted at 60 °C for 23 hours. The obtained mixture was subjected to preparative liquid phase purification to obtain the target compound (15 mg, yield 21%).

LC-MS: m/z 657 (M+Na)⁺. ¹H NMR (400 MHz, CDCl₃): δ 7.65 (s, 2H), 7.09 (s, 1H), 6.02 (s, 2H), 5.79 (s, 2H), 3.29-3.20 (m, 1H), 2.64-2.57 (m, 1H), 2.55-2.48 (m, 1H), 1.28 (d, *J* = 6.8 Hz, 6H), 1.20 (d, *J* = 6.8 Hz, 6H), 1.11 (d, *J* = 6.8 Hz, 6H)

### Test Example 1

### Pharmacokinetic testing evaluation

Male SD rats, weighing about 220 g, were divided into 2 groups after fasting overnight, with 4 rats in each group. The first group was intragastrically administered 10 mg/kg solution of compound MGL-3196 prepared in Example 1, and the second group was intragastrically administered 10 mg/kg solution of compound of Example 5 of WO2009037172A1 (The dosage is calculated by the relative content of compound MGL-3196 prepared in Example 1), wherein the vehicle of the solution of compound MGL-3196 and compound of Example 5 of WO2009037172A1 is DMSO/PEG400. Blood was collected from each group at 0.5, 1.0, 2.0, 4.0, 6.0, 8.0, 12 and 24 hours after administration, and the concentration of active ingredient MGL-3196 in plasma was determined by LC/MS/MS.

The structure of compound MGL-3196 is as follows:

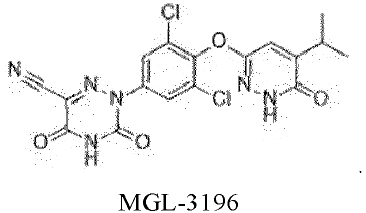

The structure of compound of Example 5 of WO2009037172A1 is as follows:

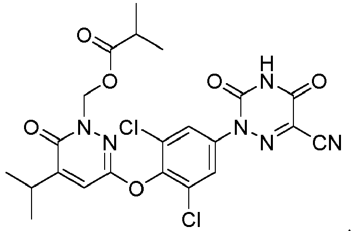

### Compound of Example 5 of WO2009037172A1

### Pharmacokinetic test results

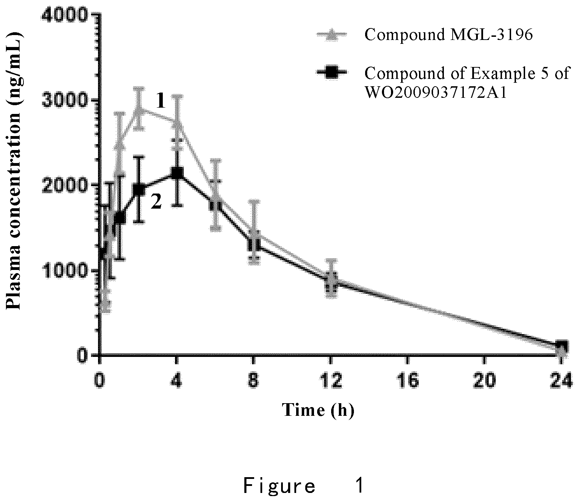

The plasma concentration-time curves of MGL-3196 prepared in Example 1 and the compound of Example 5 of WO2009037172A1 are shown in Figure 1, and the pharmacokinetic parameters are shown in Table 2:

**Table 2 Summary of pharmacokinetic parameters (n=4, mean)**

| Parameter | Unit | MGL-3196 | WO2009037172A1 Example 5 |
|---|---|---|---|
| Dosage (Calculated based on MGL-3196) | | 10 mg/kg | 10 mg/kg |
| AUC | ng.h/mL | 28200 | 24400 |
| Cmax | ng/mL | 3210 | 2570 |
| Tmax | Hours | 2.75 | 3.63 |
| T_{1/2} | Hours | 3.35 | 4.51 |

Remarks: The plasma concentration is the concentration of MGL-3196.

### Test Example 2

### Pharmacokinetic testing evaluation

Male SD rats, weighing about 220 g, were divided into 2 groups after fasting overnight, with 4 rats in each group. The first group was intragastrically administered 10 mg/kg solution of compound MGL-3196 prepared in Example 1, and the second group was intragastrically administered 10 mg/kg solution of compound 6A prepared in Example 6 (The dosage is calculated by the relative content of compound MGL-3196 prepared in Example 1), wherein the vehicle of the solution of MGL-3196 compound and compound 6A prepared in Example 6 is DMSO/PEG400. Blood was collected from each group at 0.5, 1.0, 2.0, 4.0, 6.0, 8.0, 12 and 24 hours after administration, and the concentration of active ingredient MGL-3196 in plasma was determined by LC/MS/MS.

### Pharmacokinetic test results

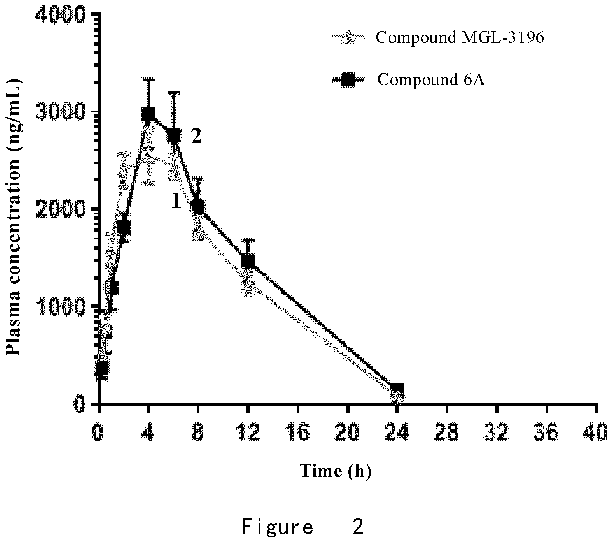

The plasma concentration-time curves of MGL-3196 prepared in Example 1 and compound 6A prepared in Example 6 are shown in Figure 2, and the pharmacokinetic parameters are shown in Table 3:

**Table 3 Summary of pharmacokinetic parameters (n=4, mean)**

| Parameter | Unit | MGL-3196 | Compound 6A |
|---|---|---|---|
| Dosage (Calculated based on MGL-3196) | | 10 mg/kg | 10 mg/kg |
| AUC | ng.h/mL | 31100 | 34100 |
| Cmax | ng/mL | 2710 | 3070 |
| Tmax | Hours | 5.00 | 4.50 |
| T_{1/2} | Hours | 3.54 | 4.13 |

### Test Example 3

### Pharmacokinetic testing evaluation

Male SD rats, weighing about 220 g, were divided into 3 groups after fasting overnight, with 4 rats in each group. The first group was intragastrically administered 10 mg/kg solution of compound MGL-3196 prepared in Example 1, and the second group was intragastrically administered 10 mg/kg solution of compound 6B prepared in Example 6 (The dosage is calculated by the relative content of compound MGL-3196 prepared in Example 1), wherein the vehicle of the solution of compound MGL-3196 and compound 6B prepared in Example 6 is DMSO/PEG400. Blood was collected from each group at 0.5, 1.0, 2.0, 4.0, 6.0, 8.0, 12 and 24 hours after administration, and the concentration of active ingredient MGL-3196 in plasma was determined by LC/MS/MS.

### Pharmacokinetic test results

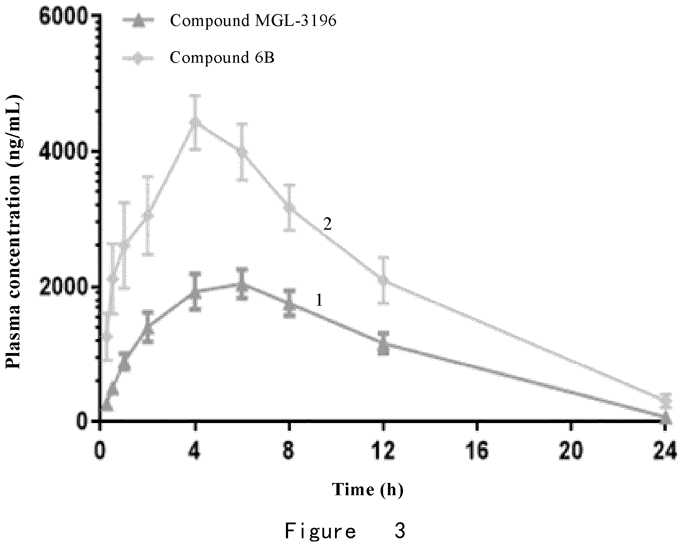

The plasma concentration-time curves of MGL-3196 prepared in Example 1 and compound 6B prepared in Example 6 are shown in Figure 3, and the pharmacokinetic parameters are shown in Table 4:

**Table 4 Summary of pharmacokinetic parameters (n=4, mean)**

| Parameter | Unit | MGL-3196 | Compound 6B |
|---|---|---|---|
| Dosage (Calculated based on MGL-3196) | | 10 mg/kg | 10 mg/kg |
| AUC | ng.h/mL | 25985 | 52653 |
| Cmax | ng/mL | 2122.5 | 4652.5 |
| Tmax | Hours | 6.0 | 4.0 |
| T_{1/2} | Hours | 3.55 | 4.73 |

### Test Example 4

### Pharmacokinetic testing evaluation

Male SD rats, weighing about 220 g, were divided into 3 groups after fasting overnight, with 4 rats in each group. The first group was intragastrically administered 10 mg/kg solution of compound MGL-3196 prepared in Example 1, the second group was intragastrically administered 10 mg/kg solution of compound 2A prepared in Example 2 (The dosage is calculated by the relative content of compound MGL-3196 prepared in Example 1), and the third group was intragastrically administered 10 mg/kg solution of compound 2B prepared in Example 2 (The dosage is calculated by the relative content of compound MGL-3196 prepared in Example 1), wherein the vehicle of the solution of compound MGL-3196, compound 2A, and compound 2B is DMSO/PEG400. Blood was collected from each group at 0.5, 1.0, 2.0, 4.0, 6.0, 8.0, 12 and 24 hours after administration, and the concentration of active ingredient MGL-3196 in plasma was determined by LC/MS/MS.

### Pharmacokinetic test results

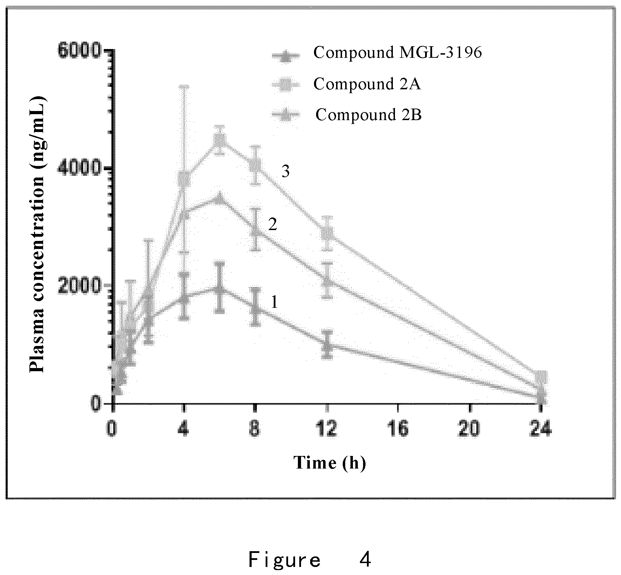

The plasma concentration-time curves of MGL-3196 prepared in Example 1, compound 2A and compound 2B prepared in Example 2 are shown in Figure 4, and the pharmacokinetic parameters are shown in Table 5:

**Table 5 Summary of pharmacokinetic parameters (n=4, mean)**

| Parameter | Unit | MGL-3196 | Compound 2A | Compound 2B |
|---|---|---|---|---|
| Dosage (Calculated based on MGL-3196) | | 10 mg/kg | 10 mg/kg | 10 mg/kg |
| AUC | ng.h/mL | 24169 | 58340 | 45222 |
| Cmax | ng/mL | 2142.5 | 5400 | 3972 |
| Tmax | Hours | 4.5 | 6 | 5.5 |
| T_{1/2} | Hours | 3.73 | 4.91 | 3.97 |

In summary, it can be seen from Tables 2-5 and Figures 1-4 that compared to comparative compound MGL-3196 prepared in Example 1 and compound of Example 5 in WO2009037172A1, compound 2A, compound 2B, and compound 6B of the present invention showed better metabolic properties, longer half-life, higher maximum plasma concentration (Cmax) and plasma exposure AUC in rats (Compound 2A is 2.4 times more than compound MGL-3196, compound 2B is 1.87 times more than compound MGL-3196, and compound 6B is 2.0 times more than compound MGL-3196). The results show that compound 2A, compound 2B and compound 6B have excellent bioavailability and efficacy.

### Test Example 5

### Pharmacokinetic testing evaluation

ICR mice, weighing 20-30 g, were divided into 4 groups after fasting overnight, with 8 mice in each group. The first group was intragastrically administered 10 mg/kg solution of compound MGL-3196 prepared in Example 1, the second group was intragastrically administered 10 mg/kg solution of compound 2A prepared in Example 2 (The dosage is calculated by the relative content of compound MGL-3196 prepared in Example 1), the third group was intragastrically administered 10 mg/kg solution of compound 2B prepared in Example 2 (The dosage is calculated by the relative content of compound MGL-3196 prepared in Example 1) and the fourth group was intragastrically administered 10 mg/kg solution of compound 6B prepared in Example 6 (The dosage is calculated by the relative content of compound MGL-3196 prepared in Example 1), wherein the vehicle of the solution of compound MGL-3196, compound 2A, compound 2B and compound 6B is DMSO/PEG400. The livers of mice in each group were collected at 1hr and 6hr after administration, respectively, and the concentration of the active ingredient MGL-3196 in the liver was determined by LC/MS/MS.

**Table 6 Summary of drug concentrations in liver tissue (n=4, mean)**

| Parameter | Unit | MGL-3196 | Compound 2A | Compound 2B | Compound 6B |
|---|---|---|---|---|---|
| Dosage (Calculated based on MGL-3196) | | 10 mg/kg | 10 mg/kg | 10 mg/kg | 10 mg/kg |
| Average concentration (ng/g) (1 hr) | | 36975 | 60313 | 66500 | 52975 |
| Average concentration (ng/g) (6 hr) | | 33438 | 38913 | 49838 | 53125 |

It can be seen from Table 6 that compound 2A, compound 2B and compound 6B in the present invention have higher drug concentrations in mouse liver at 1 hr and 6 hr compared to comparative compound MGL-3196 prepared in Example 1.

### Test Example 6

### Diet-induced obesity (DIO) C57B1/6J mouse model studies

6-week-old C57Bl/6J mice were given a high-fat diet for 34 weeks. Mice were intragastrically administered vehicle (2% Klucel LF, 0.1% Tween 80 in water); 1, 3 or 10 mg/kg of compound 2A, compound 2B prepared in Example 2 or compound MGL-3196 prepared in Example 1 starting from day 0 (1st day of 35 weeks) respectively, 9 mice in each group, for 23 consecutive days. In a parallel study, mice were intragastrically administered vehicle (Dulbecco's phosphate buffered saline, pH adjusted to 9.0 with IN NaOH) or 10, 30 or 100 µg/kg triiodothyronine (T3) starting from day 0 (day 1 of week 35) respectively, 9 mice in each group, for 23 consecutive days. Body weight and food intake were monitored during the study. BMD and body composition analyses were performed on day 22. At necropsy on day 23, organ weighing was performed and cholesterol and other blood biochemical parameters of blood samples were evaluated.

Compound 2A and compound 2B prepared in Example 2 and compound 6B prepared in Example 6 in the present invention have excellent efficacy in lowering cholesterol and liver fat.

All documents mentioned herein are incorporated by reference in this application as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A pyridazinone or pyridazine compound represented by formula (I) or (Ia) or stereoisomer, tautomer, enantiomer, diastereomer, resonator, pharmaceutically acceptable salt, hydrate, solvate, or crystal form thereof, 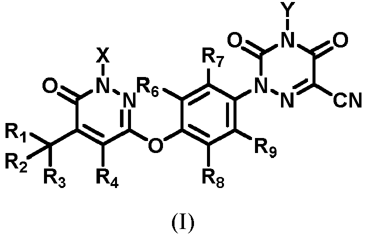 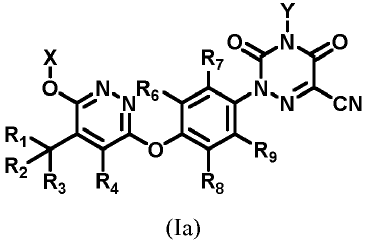 wherein:
X is selected from H or -C(R₁₇R₁₈)OZ,
Y is selected from H or -C(R₁₉R₂₀)OW, provided that both X and Y are not hydrogen at a time;
Z and W are each independently selected from -C(O)OR₅, -C(O)NHR₅, -C(O)NR₅R₁₀, -C(O)R₅, -P(=O)(X₁R₁₁)(X₂R₁₂), -P(=O)(X₁R₁₁)(X₃R₁₄R₁₅), -P(=O)(X₃R₁₄R₁₅)(X₃R₁₄R₁₅), -CH₂P(=O)(X₁R₁₁)(X₂R₁₂), -CH₂P(=O)(X₁R₁₁)(X₃R₁₄R₁₅), -CH₂P(=O)(X₃R₁₄R₁₅)(X₃R₁₄R₁₅), -P(=S)(X₁R₁₁)(X₂R₁₂), -P(=S)(X₁R₁₁)(X₃R₁₄R₁₅), -P(=S)(X₃R₁₄R₁₅)(X₃R₁₄R₁₅), -CH₂P(=S)(X₁R₁₁)(X₂R₁₂), -CH₂P(=S)(X₁R₁₁)(X₃R₁₄R₁₅), -CH₂P(=S)(X₃R₁₄R₁₅)(X₃R₁₄R₁₅), -P(=NR₁₃)(X₁R₁₁)(X₂R₁₂), -P(=NR₁₃)(X₁R₁₁)(X₃R₁₄R₁₅), -P(=NR₁₃)(X₃R₁₄R₁₅)(X₃R₁₄R₁₅), -CH₂P(=NR₁₃)(X₁R₁₁)(X₂R₁₂), -CH₂P(=NR₁₃)(X₁R₁₁)(X₃R₁₄R₁₅), -CH₂P(=NR₁₃)(X₃R₁₄R₁₅)(X₃R₁₄R₁₅), -OP(=O)(X₁R₁₁)(X₂R₁₂), -OP(=O)(X₁R₁₁)(X₃R₁₄R₁₅), -OP(=O)(X₃R₁₄R₁₅)(X₃R₁₄R₁₅), -OCH₂P(=O)(X₁R₁₁)(X₂R₁₂), -OCH₂P(=O)(X₁R₁₁)(X₃R₁₄R₁₅), -OCH₂P(=O)(X₃R₁₄R₁₅)(X₃R₁₄R₁₅), -OP(=S)(X₁R₁₁)(X₂R₁₂), -OP(=S)(X₁R₁₁)(X₃R₁₄R₁₅), -OP(=S)(X₃R₁₄R₁₅)(X₃R₁₄R₁₅), -OCH₂P(=S)(X₁R₁₁)(X₂R₁₂), -OCH₂P(=S)(X₁R₁₁)(X₃R₁₄R₁₅), -OCH₂P(=S)(X₃R₁₄R₁₅)(X₃R₁₄R₁₅), -OP(=NR₁₃)(X₁R₁₁)(X₂R₁₂), -OP(=NR₁₃)(X₁R₁₁)(X₃R₁₄R₁₅), -OP(=NR₁₃)(X₃R₁₄R₁₅)(X₃R₁₄R₁₅), -OCH₂P(=NR₁₃)(X₁R₁₁)(X₂R₁₂), -OCH₂P(=NR₁₃)(X₁R₁₁)(X₃R₁₄R₁₅), and -OCH₂P(=NR₁₃)(X₃R₁₄R₁₅)(X₃R₁₄R₁₅);
R₁, R₂ and R₃ are each independently selected from the group consisting of hydrogen, deuterium, undeuterated or one or more (preferably 1-4) deuterated or perdeuterated C1-C4 alkyl, hydroxyl, or two of R₁, R₂ and R₃ together with adjacent C form substituted or unsubstituted C3-C8 cycloalkyl; the "substituted" refers to being substituted by one or more substituents selected from the group consisting of C1-C4 alkyl, C3-C8 cycloalkyl, hydroxyl, amino, carbonyl, C2-C8 ester group, cyano, ether group, thioether group, C2-C8 amido, and sulfonamido;
R₄, R₆, R₇, R₈ and R₉ are each independently selected from the group consisting of hydrogen, deuterium, and halogen;
R₅ is substituted or unsubstituted C1-C20 alkyl, substituted or unsubstituted C3-C20 cycloalkyl, substituted or unsubstituted 4-20 membered heterocycloalkyl, substituted or unsubstituted C6-C20 aryl, substituted or unsubstituted C6-C20 aryl-C1-C8 alkyl-, substituted or unsubstituted 5-12 membered heteroaryl, substituted or unsubstituted 5-12 membered heteroaryl-C1-C8 alkyl-, or substituted or unsubstituted C1-C4 alkyl-(substituted or unsubstituted C₁-C₄ alkyl-O)ₘ-substituted or unsubstituted C1-C4 alkyl-, wherein, m is positive integer from 1 to 8; the "substituted" refers to being substituted by one or more substituents selected from the group consisting of C1-C4 alkyl, C1-C4 alkoxy, C3-C8 cycloalkyl, halogen, hydroxyl, amino, amino, C2-C8 carbonyl, C2-C8 ester group, cyano, ether group, thioether group, C2-C8 amido, and sulfonamido;
R₁₀ is selected from substituted or unsubstituted C1-C6 alkyl, substituted or unsubstituted C3-C8 cycloalkyl, or substituted or unsubstituted 4-10 membered heterocycloalkyl; the "substituted" refers to being substituted by one or more substituents selected from the group consisting of C1-C4 alkyl, C1-C4 alkoxy, C3-C8 cycloalkyl, halogen, hydroxyl, amino, amino, C2-C8 carbonyl, C2-C8 ester group, cyano, ether group, thioether group, C2-C8 amido, or sulfonamido;
X₁ and X₂ are each independently selected from the group consisting of oxygen, and sulfur;
X₃ is nitrogen;
R₁₁, R₁₂, R₁₃, R₁₄ and R₁₅ are each independently selected from the group consisting of hydrogen, substituted or unsubstituted C1-C20 alkyl, substituted or unsubstituted deuterated C1-C20 alkyl, substituted or unsubstituted C3-C10 cycloalkyl, substituted or unsubstituted C4-C10 heterocycloalkyl, substituted or unsubstituted C6-C10 aryl, substituted or unsubstituted 5-10 membered heteroaryl, or R₁₁ and R₁₂ together with adjacent X₁, X₂ and P form substituted or unsubstituted 5-7 membered heterocycloalkyl; the "substituted" refers to being substituted by one or more substituents selected from the group consisting of deuterium, C1-C20 alkyl, halogenated C1-C20 alkyl, C1-C6 alkoxy, C3-C10 cycloalkyl, 4-10 membered heterocycloalkyl, C6-C10 aryl, halogenated C6-C10 aryl, C5-C10 heteroaryl, halogen, amino, nitro, -COR₁₆, -COOR₁₆, -OCOOR₁₆, cyano, hydroxyl, amido, and sulfonamido;
R₁₆ is selected from the group consisting of hydrogen, substituted or unsubstituted C1-C18 alkyl, substituted or unsubstituted deuterated C1-C20 alkyl, substituted or unsubstituted C3-C10 cycloalkyl, substituted or unsubstituted C3-C10 cycloalkenyl, substituted or unsubstituted C6-C10 aryl, amino, substituted or unsubstituted 4-10 membered heterocycloalkyl, wherein the "substituted" refers to being substituted by one or more C6-C10 aryl;
R₁₇, R₁₈, R₁₉ and R₂₀ are each independently selected from the group consisting of hydrogen, deuterium, substituted or unsubstituted C1-C3 alkyl, provided that when X is attached to N and Y is hydrogen, both R₁₇ and R₁₈ are not hydrogen at a time.

2. The compound according to claim 1, wherein the compound is a pyridazinone or pyridazine compound as shown in formula (IV-A), (IV-B), (IV-C), (IV-D) or a stereoisomer, tautomer, enantiomer, diastereomer, resonator, pharmaceutically acceptable salt, hydrate, solvate, or crystal form thereof, 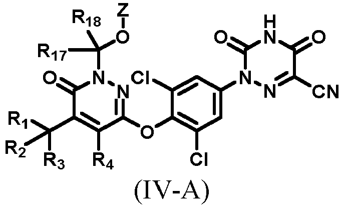 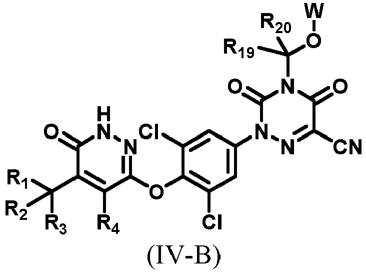 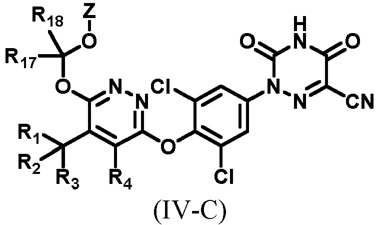 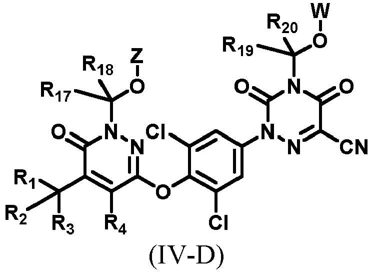 wherein, R₁, R₂, R₃, R₄, R₁₇, R₁₈, R₁₉, R₂₀, Z, and W are defined as in claim 1.

3. The compound according to claim 1, wherein the compound is a pyridazinone or pyridazine compound as shown in formula (V-A), (V-B), (V-C), and (V-D), or a stereoisomer, tautomer, enantiomer, diastereomer, resonator, pharmaceutically acceptable salt, hydrate, solvate, or crystal form thereof, 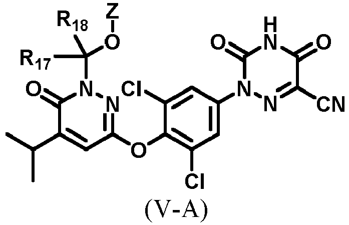 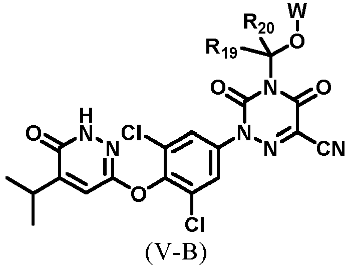 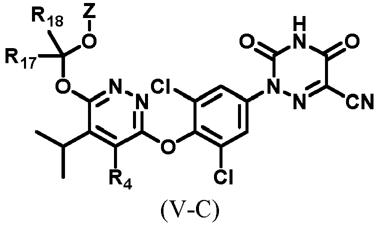 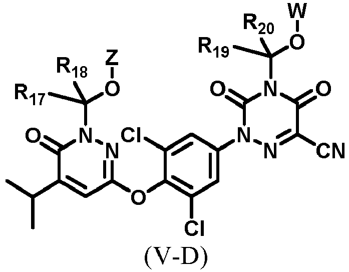 wherein, R₄, R₁₇, R₁₈, R₁₉, R₂₀, Z and W are defined as in claim 1.

4. The pyridazinone or pyridazine compound as shown in formula (I) or (Ia), or the stereoisomer, tautomer, enantiomer, diastereomer, resonator, pharmaceutically acceptable salt, hydrate, solvate, or crystal form thereof according to claim 1, wherein the compound is selected from the group consisting of

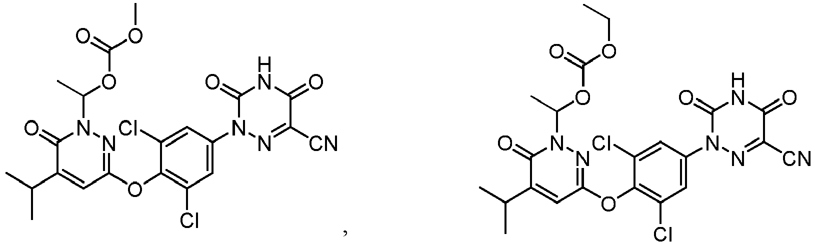

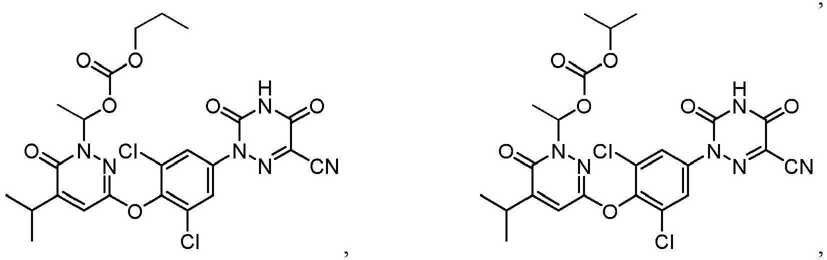

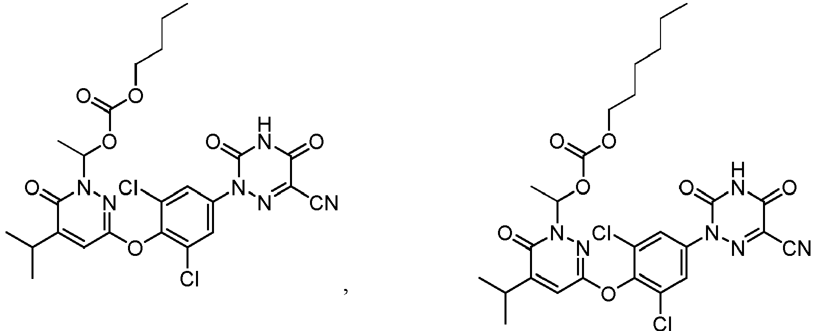

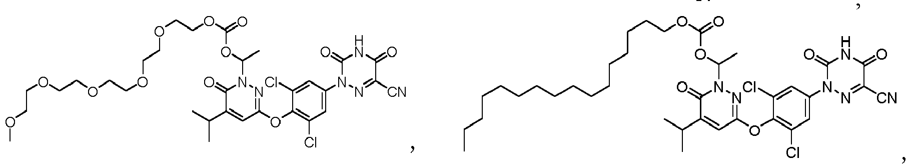

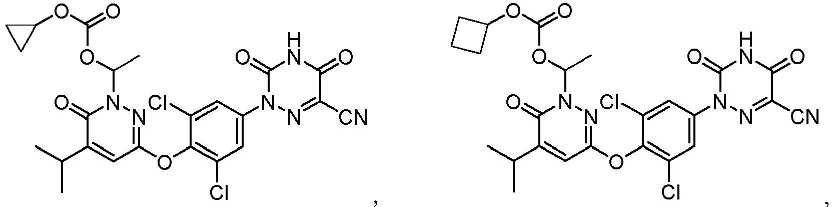

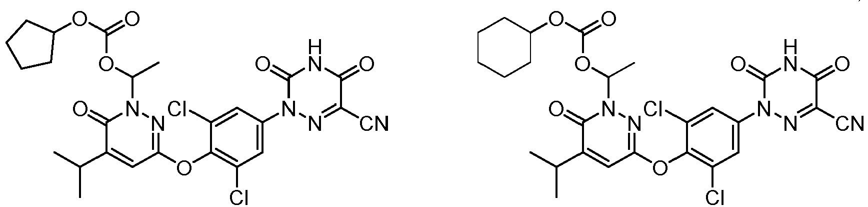

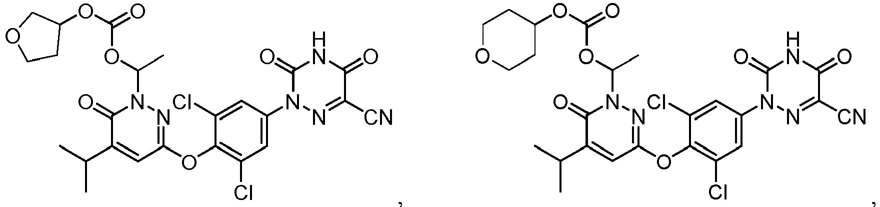

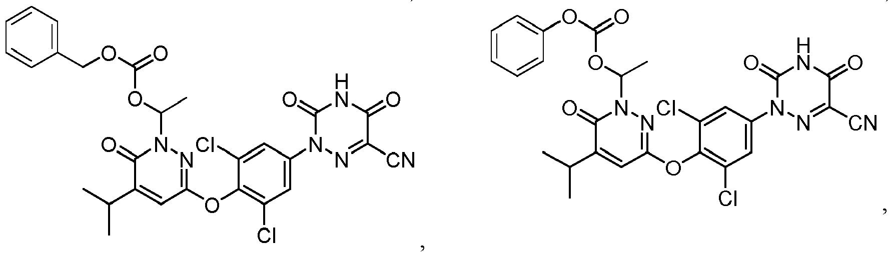

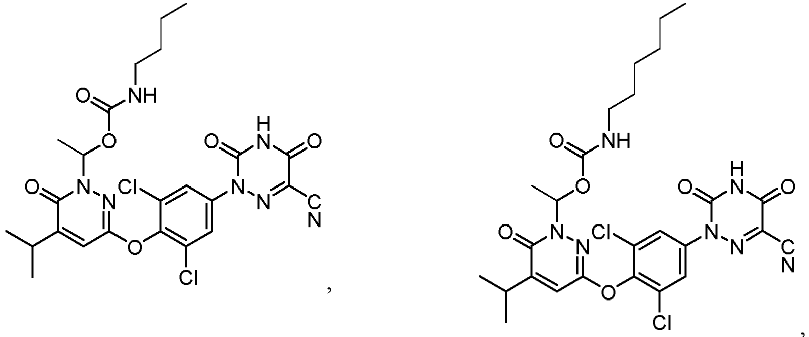

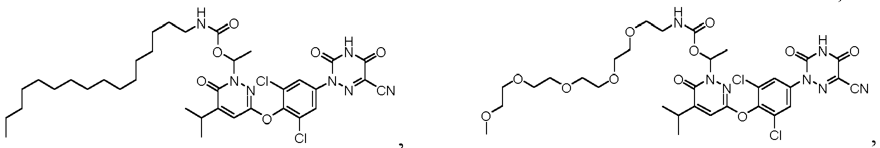

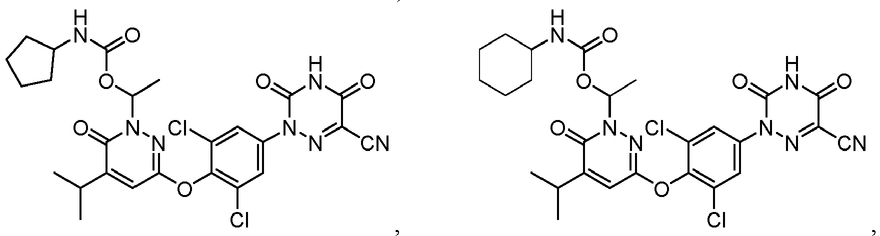

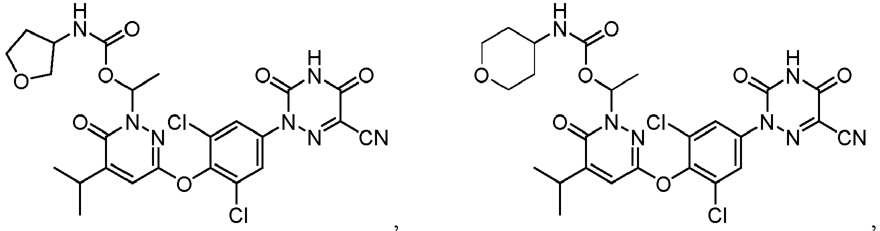

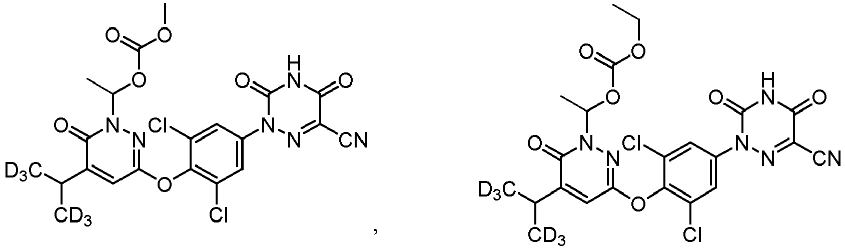

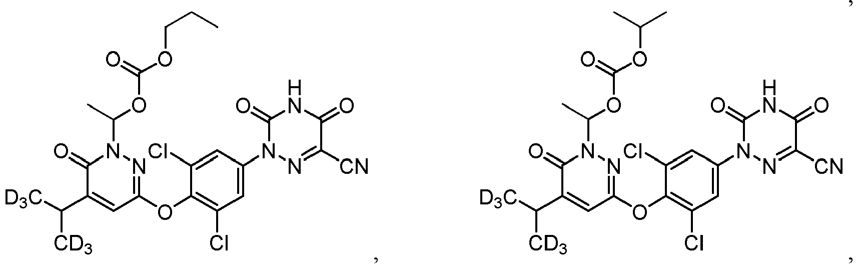

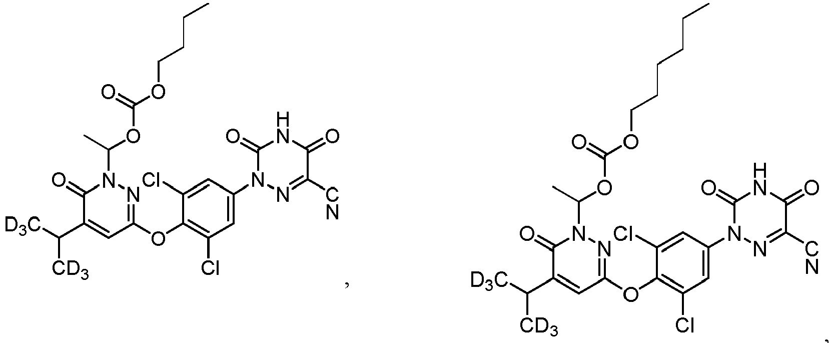

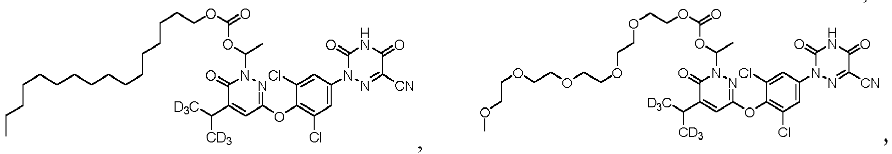

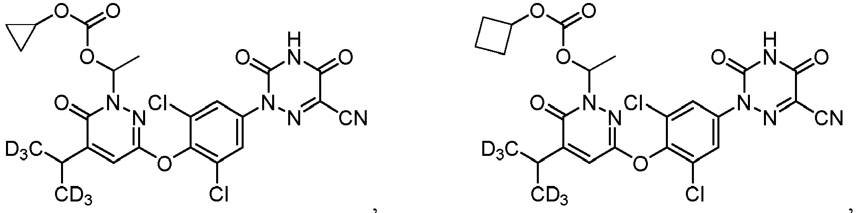

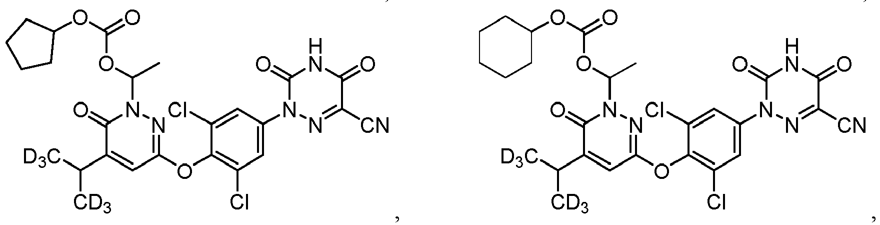

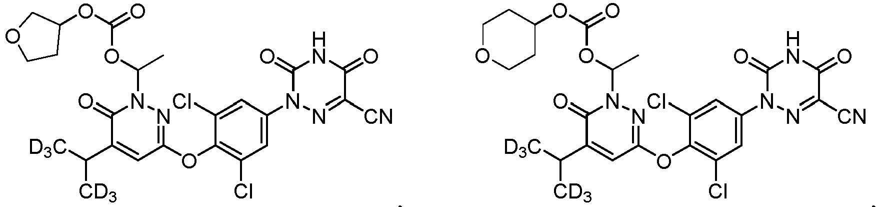

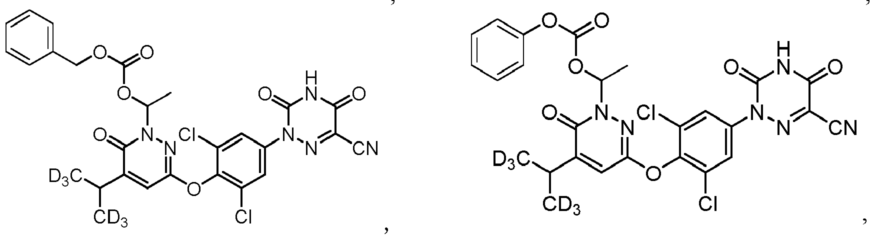

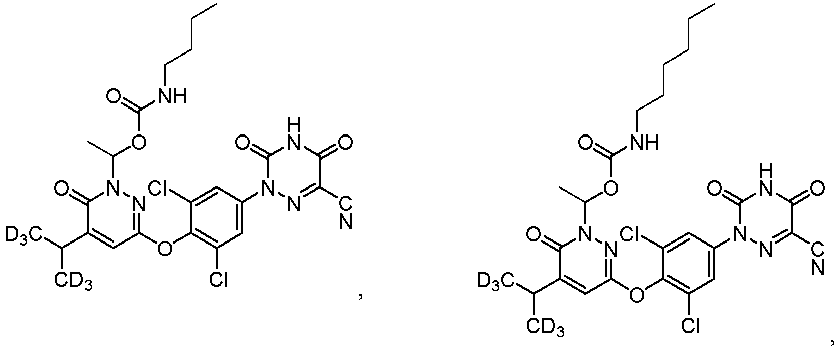

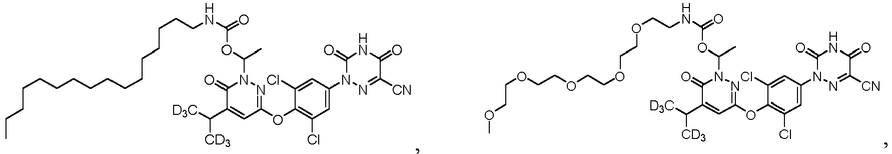

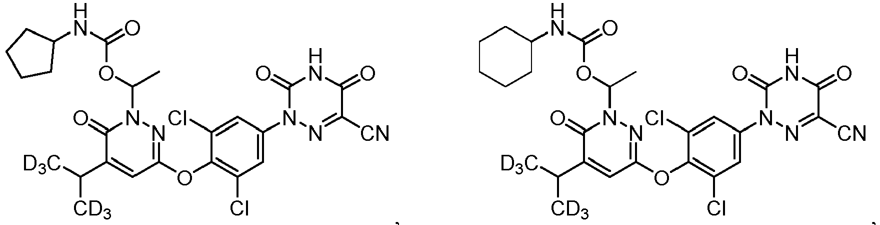

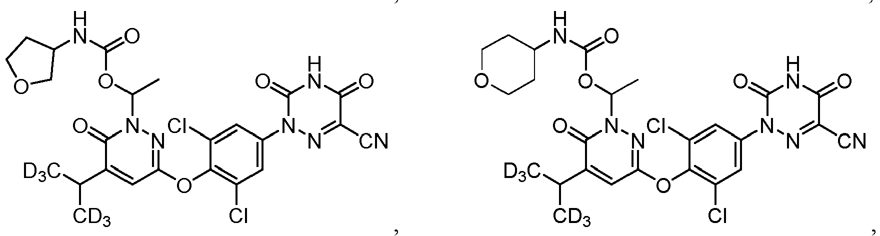

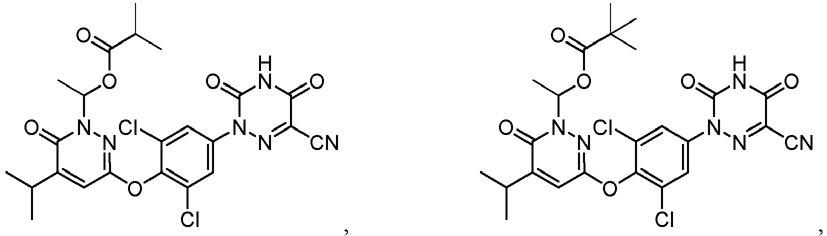

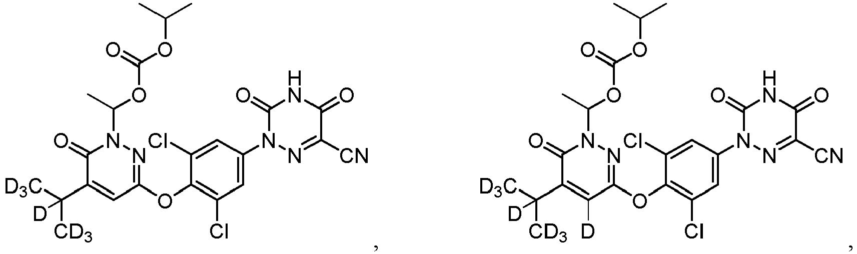

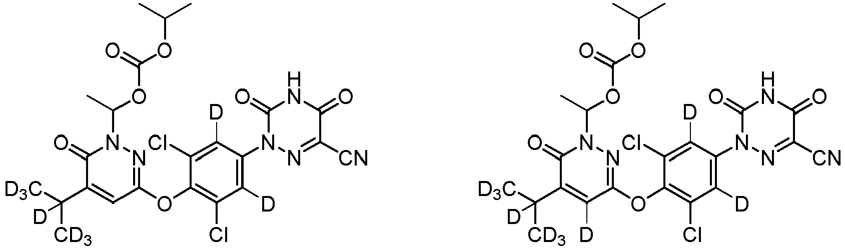

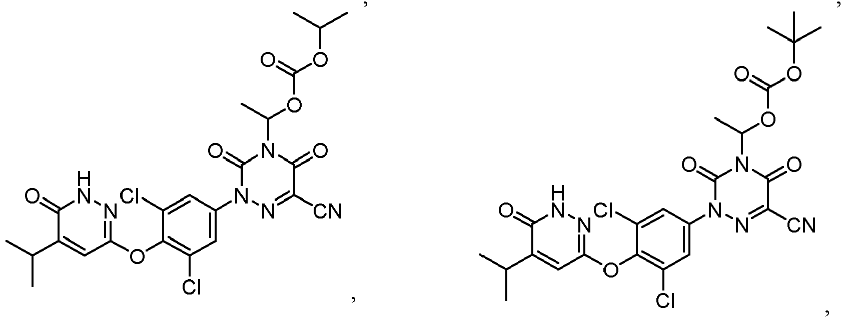

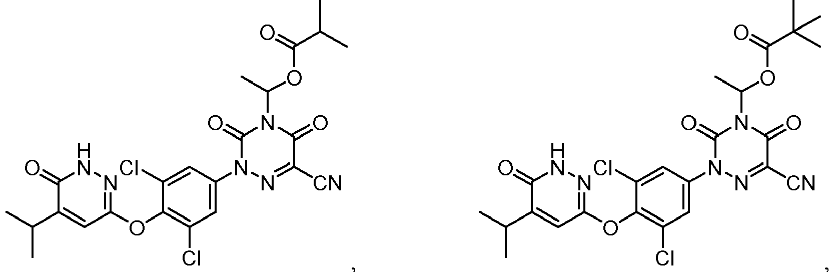

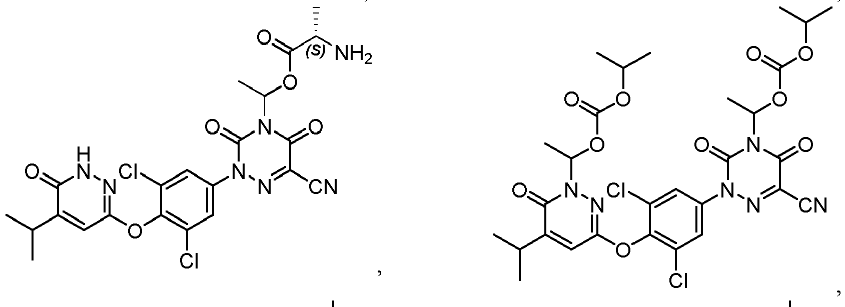

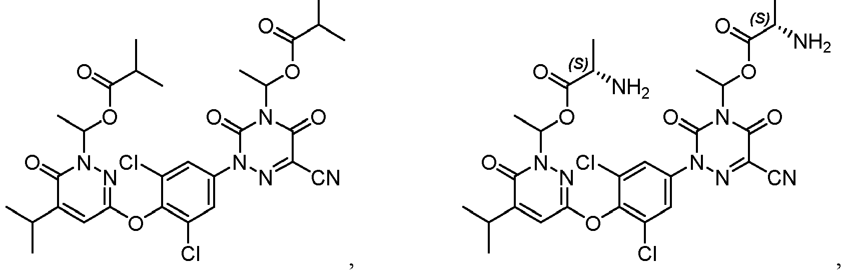

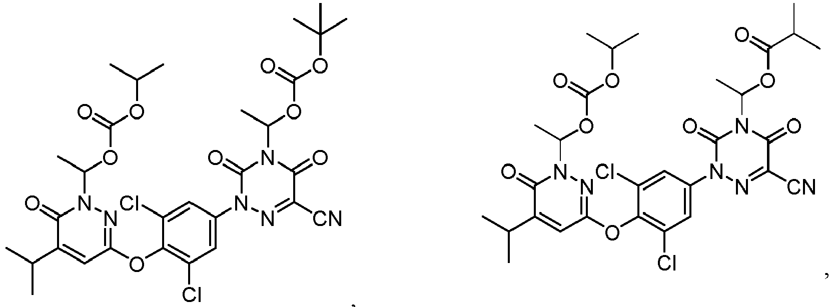

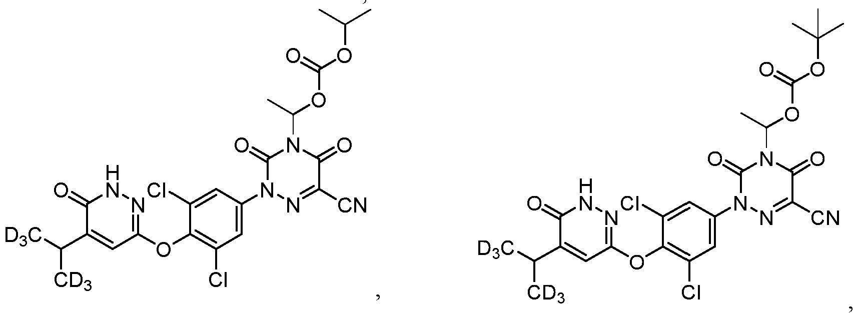

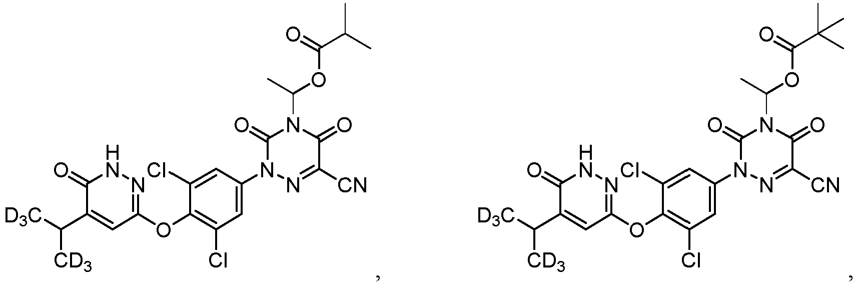

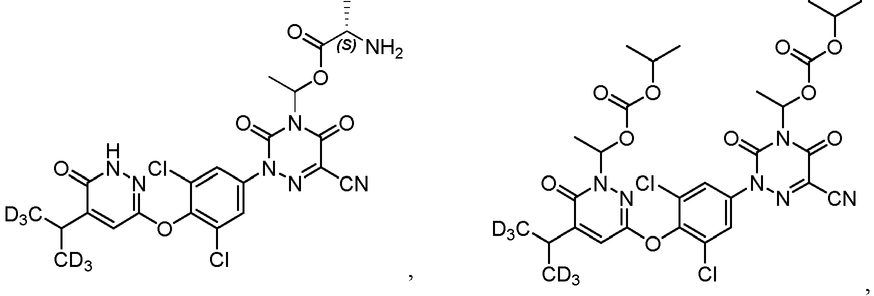

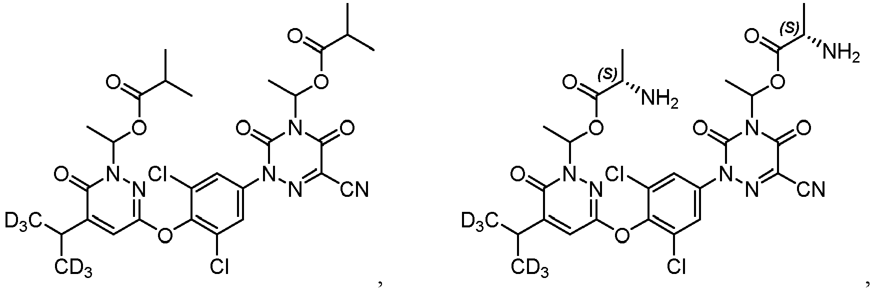

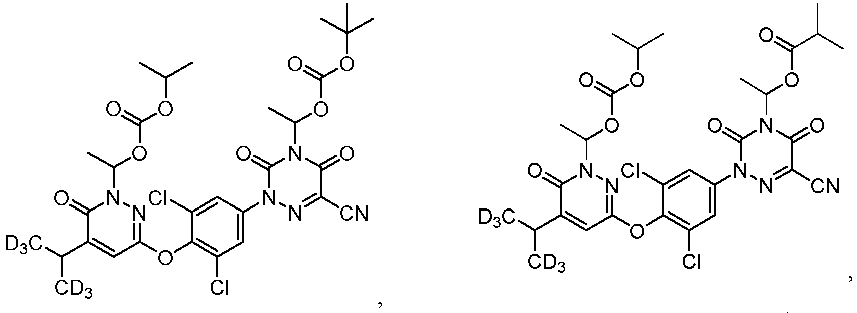

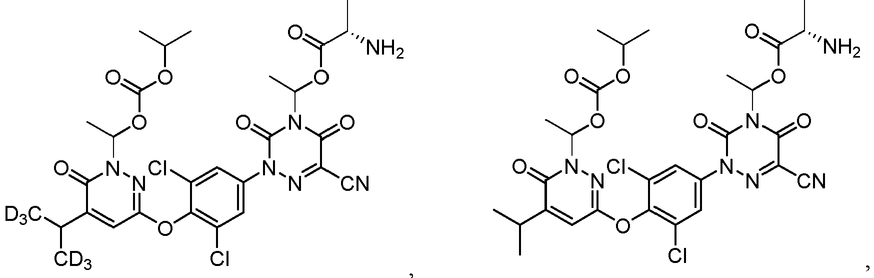

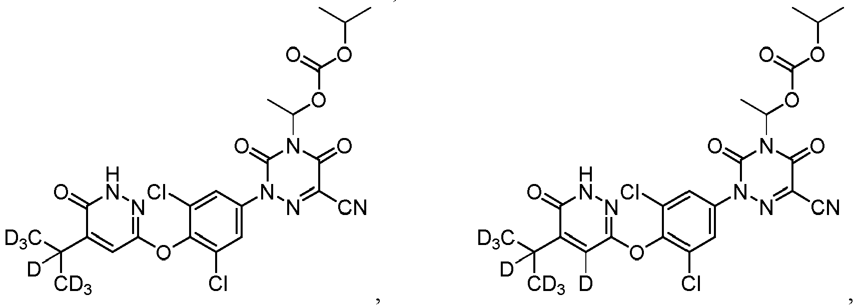

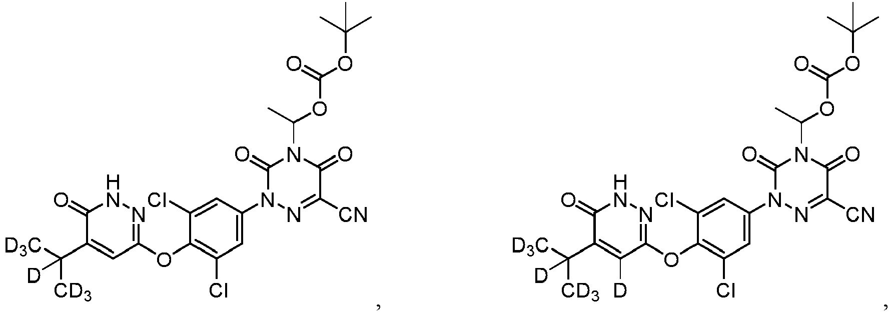

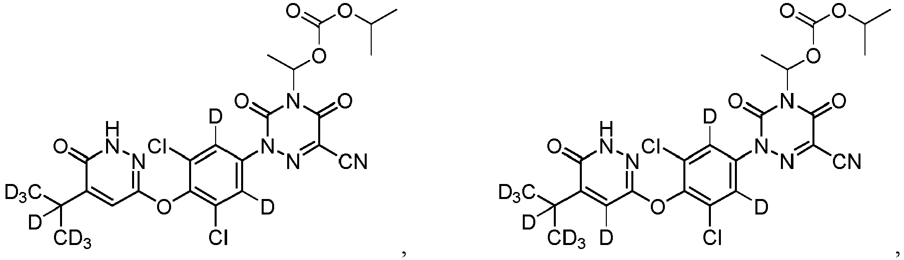

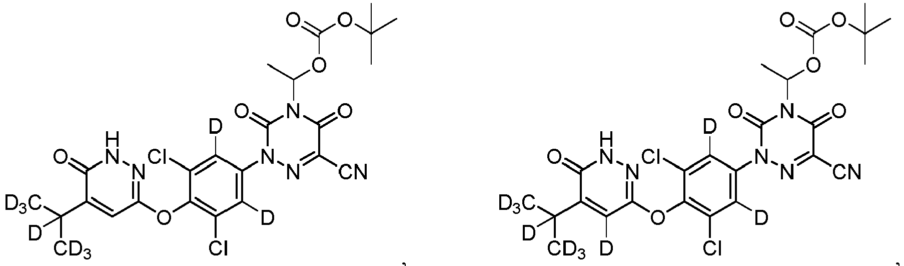

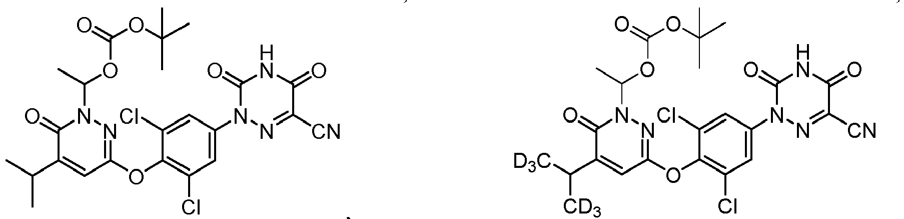

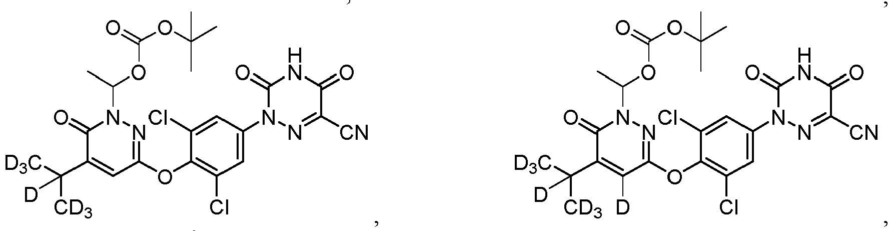

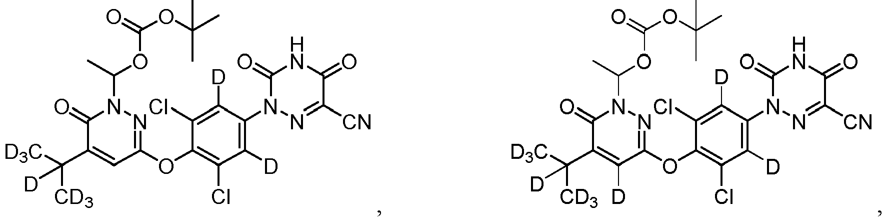

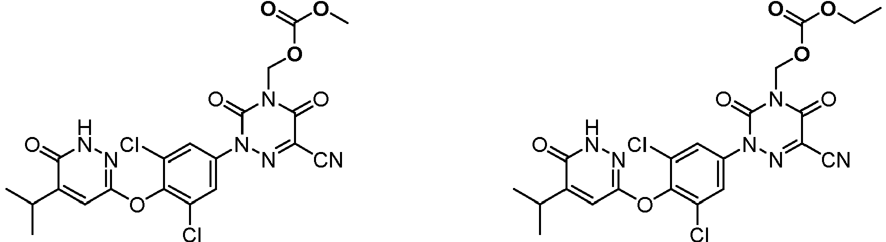

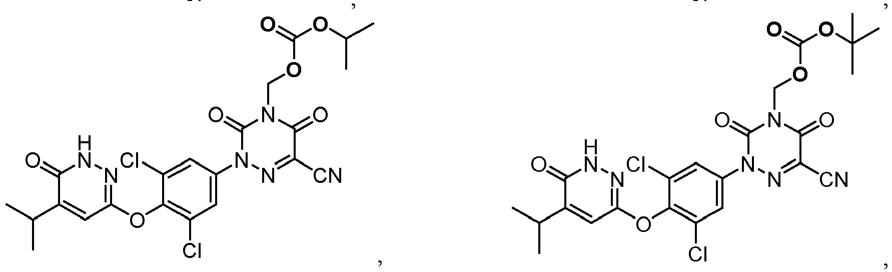

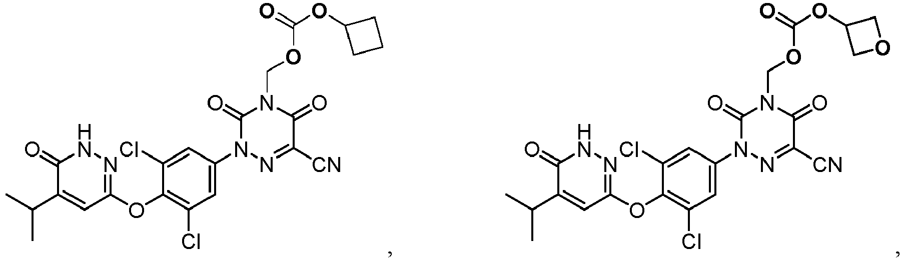

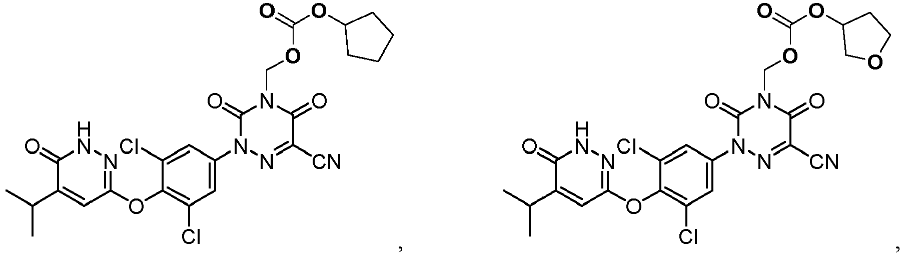

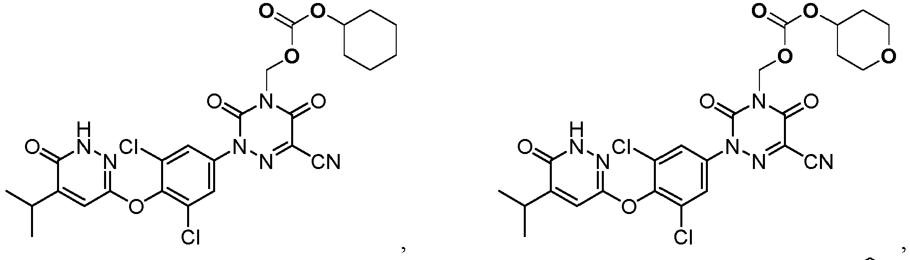

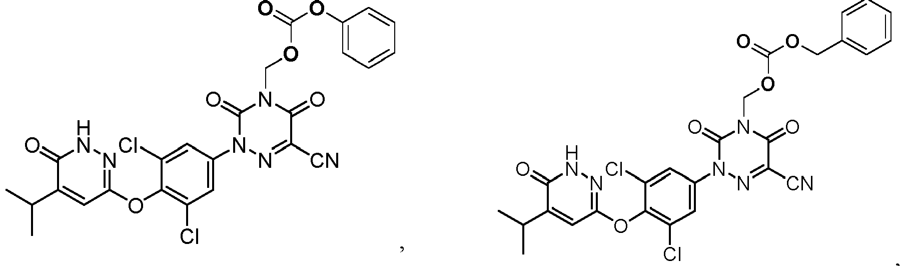

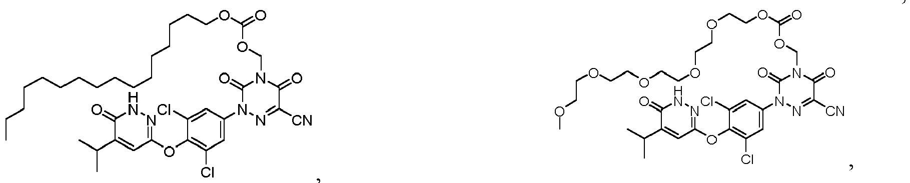

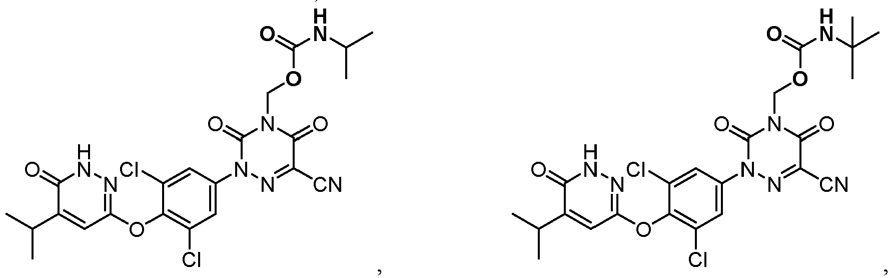

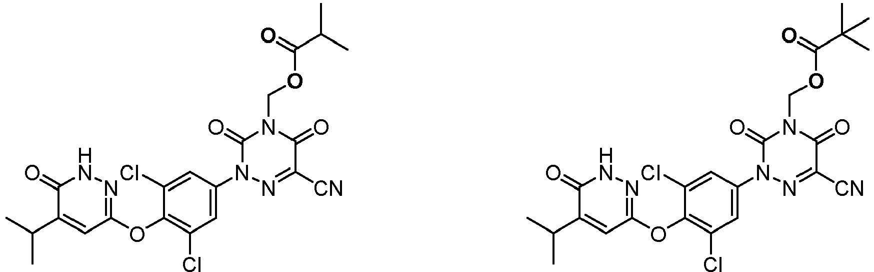

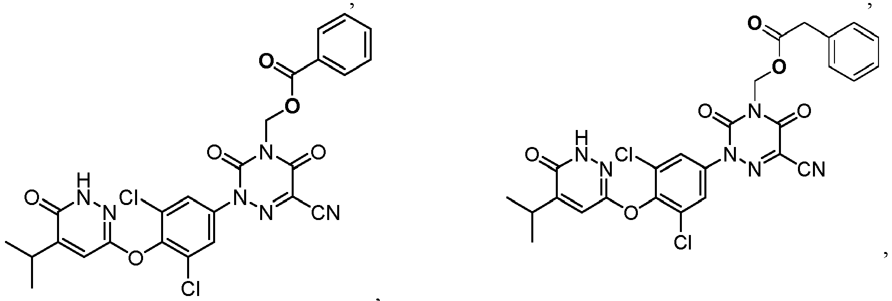

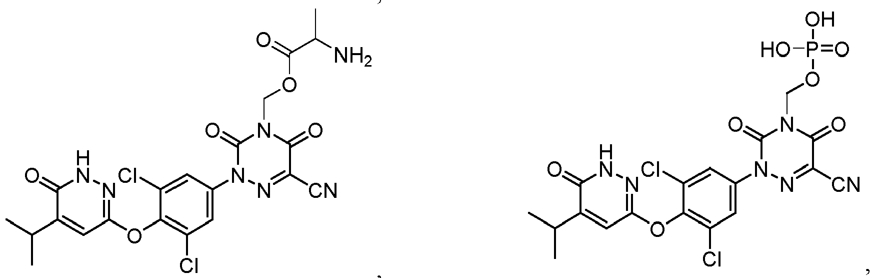

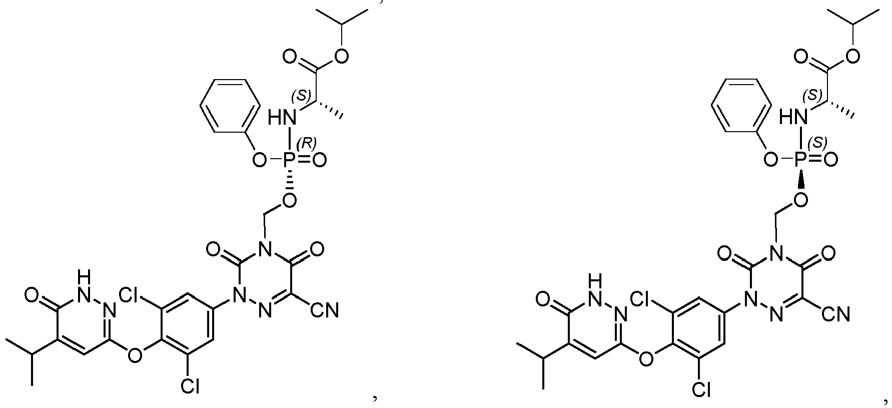

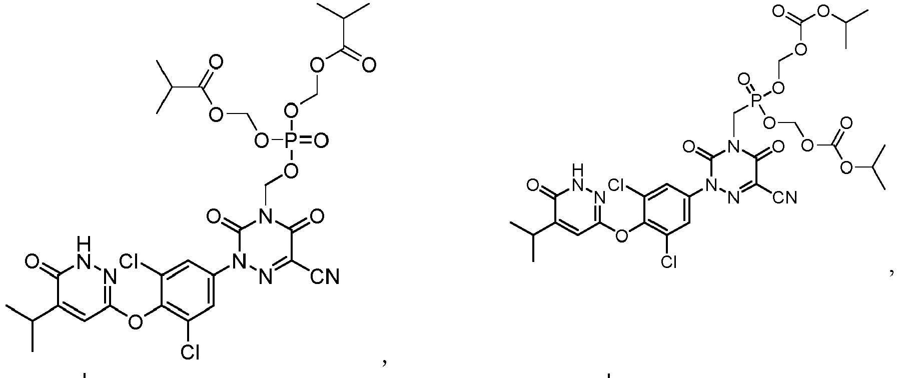

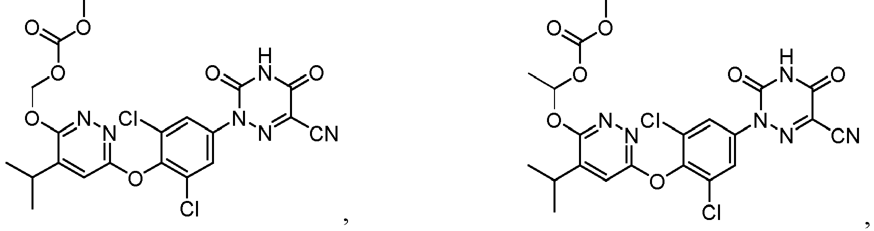

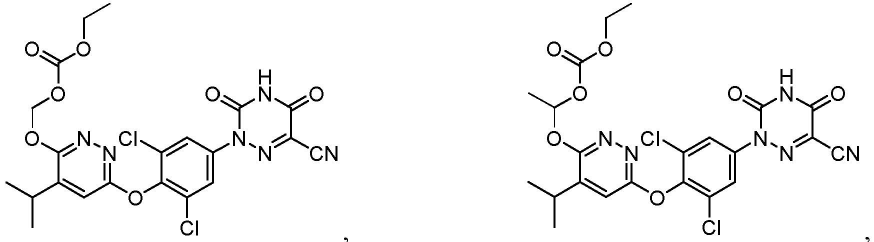

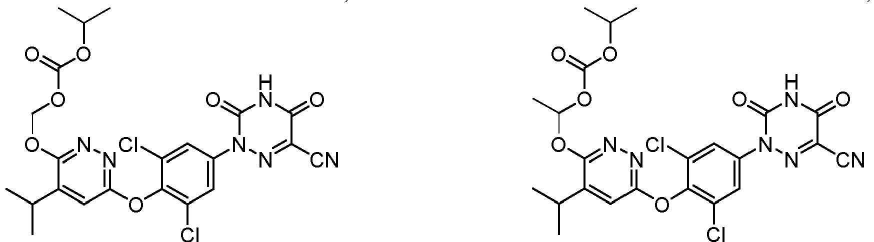

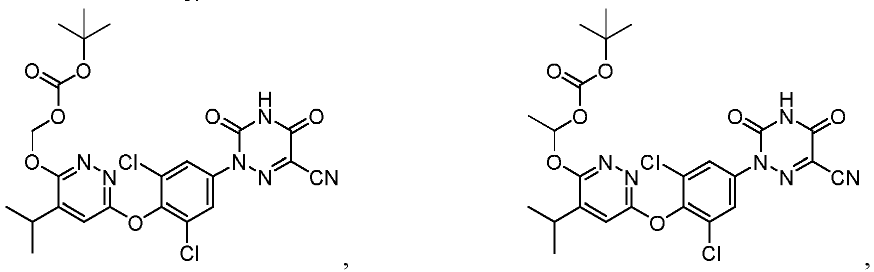

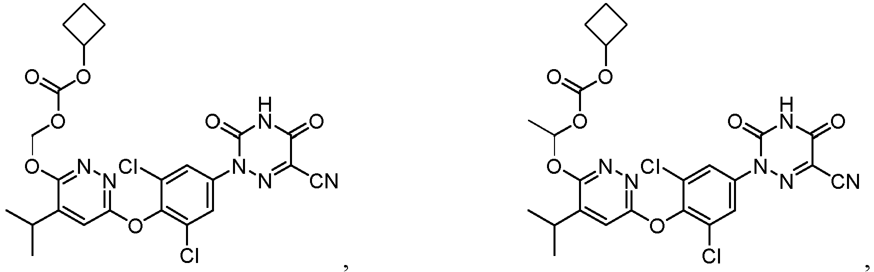

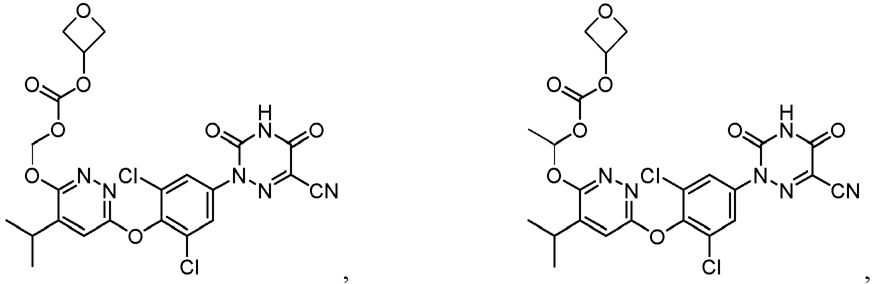

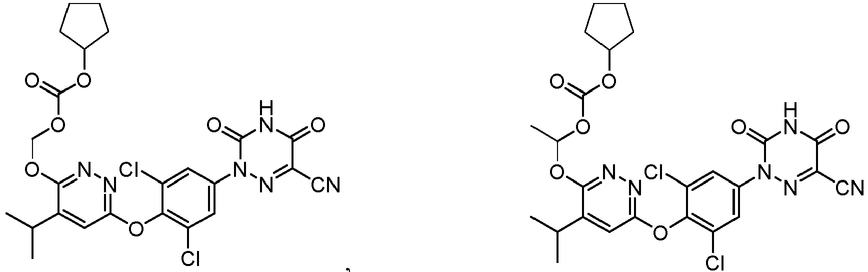

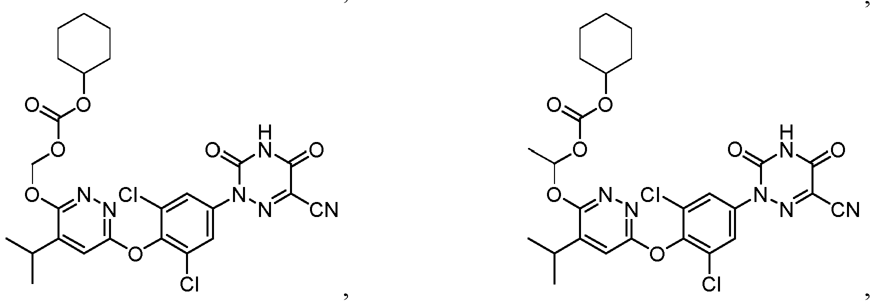

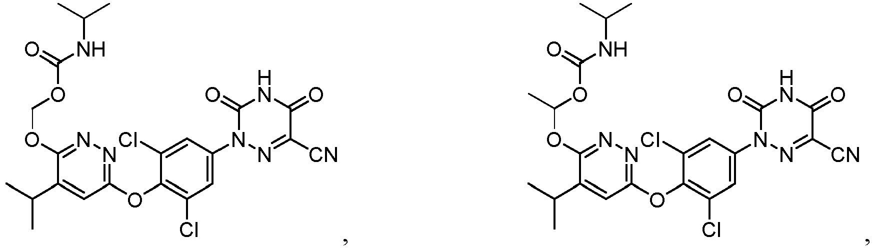

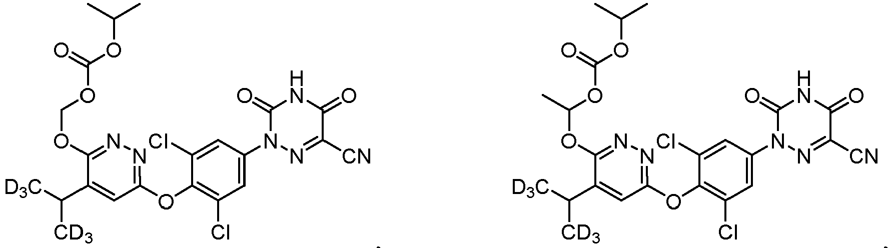

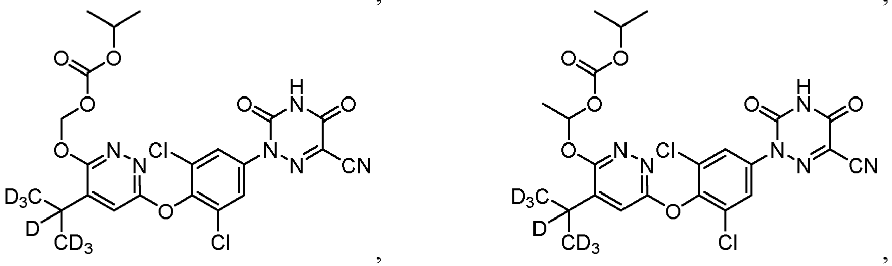

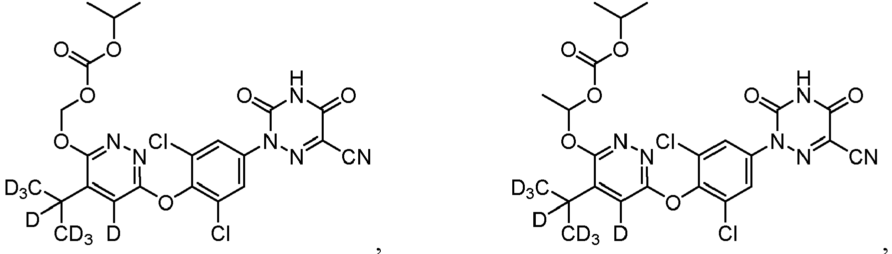

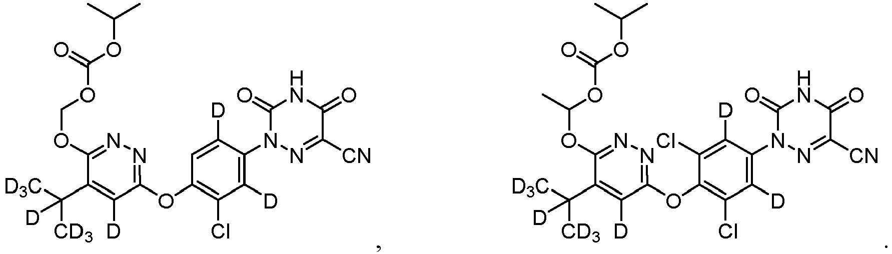

5. A method for preparing the pyridazinone or pyridazine compound as shown in formula (I) or (Ia) according to claim 1, or the stereoisomer, tautomer, enantiomer, diastereomer, resonator, pharmaceutically acceptable salt, hydrate, solvate, or crystal form thereof, wherein the method comprises the steps of: 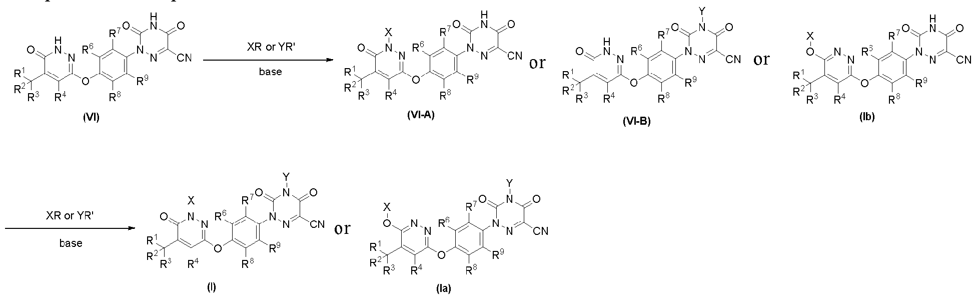
(1) reacting a compound of formula (VI) with XR or YR' to obtain an intermediate of formula (VI-A), (VI-B) or (Ib) under alkaline condition;
(2) reacting the intermediate of formula (VI-A), (VI-B) or (Ib) with YR' or XR to obtain the compound of formula (I) or (Ia) under alkaline condition;
wherein, R, R' are leaving groups.

6. A pharmaceutical composition, comprising:
1) a therapeutically effective amount of the pyridazinone or pyridazine compound represented by formula (I) or (Ia) according to claim 1, or the stereoisomer, tautomer, enantiomer, diastereomer, resonator, pharmaceutically acceptable salt, hydrate, solvate, or crystal form thereof; and 2) a pharmaceutically acceptable carrier.

7. A use of the pyridazinone or pyridazine compound represented by formula (I) or (Ia) according to claim 1, or the stereoisomer, tautomer, enantiomer, diastereomer, resonator, pharmaceutically acceptable salt, hydrate, solvate, or crystal form thereof, or the pharmaceutical composition according to claim 6, for (i) preparing thyroid hormone receptor agonist pharmaceutical preparation or drug; (ii) preparing pharmaceutical preparation or drug for a disease related to decreased thyroid hormone receptor activity; and/or (ii) preparing pharmaceutical preparation or drug for preventing and/or treating a disease selected from the group consisting of inflammation, cancer, cardiovascular disease, infection, immunological disease, metabolic disease, and a combination thereof.

8. The pharmaceutical composition according to claim 6, wherein the pharmaceutical composition further comprises other drug for preventing and/or treating a disease selected from the group consisting of inflammation, cancer, cardiovascular disease, infection, immunological disease, metabolic disease, and a combination thereof.

9. The use according to claim 7, wherein the thyroid hormone receptor is thyroid hormone α receptor and/or thyroid hormone β receptor.

10. The use according to claim 7, wherein the disease is selected from the group consisting of non-alcoholic fatty hepatitis, non-alcoholic fatty liver disease, liver fibrosis, cirrhosis (such as primary biliary cirrhosis), gallstone, atherosclerosis, obesity, hyperlipidemia, diabetes.

11. A thyroid hormone receptor agonist, wherein the agonist comprises an agonized effective amount of the pyridazinone or pyridazine compound as shown in formula (I) or (Ia) according to claim 1, or the stereoisomer, tautomer, enantiomer, diastereomer, resonator, pharmaceutically acceptable salt, hydrate, solvate, or crystal form thereof.

12. An *in vitro* non-therapeutic and non-diagnostic method for enhancing or improving thyroid hormone receptor activity, wherein the method comprises the step of: contacting thyroid hormone receptors or cells expressing thyroid hormone receptors with the pyridazinone or pyridazine compound represented by formula (I) or (Ia) according to claim 1, or the stereoisomer, tautomer, enantiomer, diastereomer, resonator, pharmaceutically acceptable salt, hydrate, solvate, or crystal form thereof in an *in vitro* culture system, thereby enhancing or improving thyroid hormone receptor activity.

13. A method of preventing and/or treating a disease selected from the group consisting of inflammation, cancer, cardiovascular disease, infection, immunological disease, metabolic disease, diabetes, wherein, administering an effective amount of the pyridazinone or pyridazine compound represented by formula (I) or (Ia) according to claim 1, or the stereoisomer, tautomer, enantiomer, diastereomer, resonator, pharmaceutically acceptable salt, hydrate, solvate, or crystal form thereof to a patient in need thereof.
